# EUROPEAN PATENT APPLICATION

(11) **EP 4 822 352 A1**
(43) Date of publication of application: **14.10.2026**
(21) Application number: 24903390.3
(22) Date of filing: 07.11.2024
(51) Int. Cl.: G01N 33/48, G01N 30/72, G01N 33/68

(54) **METHOD FOR PREPARING TRACE PROTEIN-CONTAINING SAMPLE FOR PROTEOME ANALYSIS BY USING LECTIN**

(30) Priority: 15.12.2023 JP 2023211819
(71) Applicant: Kazusa DNA Research Institute, Kisarazu-shi, Chiba 292-0818 (JP)
(72) Inventor: KAWASHIMA, Yusuke, Kisarazu-shi Chiba 292-0818 (JP); OHARA, Osamu, Kisarazu-shi Chiba 292-0818 (JP); NAKAJIMA, Daisuke, Kisarazu-shi Chiba 292-0818 (JP); KONNO, Ryo, Kisarazu-shi Chiba 292-0818 (JP); ISHIKAWA, Masaki, Kisarazu-shi Chiba 292-0818 (JP)
(74) Representative: J A Kemp LLP
(86) International application number: PCT/JP2024/039643
(87) International publication number: WO 2025/126730

(57) **Abstract**

The present invention provides a preparation method of a sample for proteome analysis, which enables efficient recovery of trace proteins in a biological sample while removing, as much as possible, highly abundant proteins that hinder deep analysis in the proteome analysis. In the preparation method for a sample for proteome analysis of the present invention, glycoproteins having binding affinity to a lectin selected from the group consisting of N-acetylglucosamine oligomer-binding lectins and fucose-binding lectins in a biological sample are recovered by contacting the biological sample with a carrier on which said lectin or a binding fragment thereof is immobilized. In one embodiment, the biological sample is contacted with a carrier on which a combination comprising *Solanum tuberosum* lectin or a binding fragment thereof and *Lycopersicon esculentum* lectin or a binding fragment thereof is immobilized.

## Description

### TECHNICAL FIELD

The present invention relates to a method for preparing a trace protein-containing sample for proteome analysis from a biological sample using a lectin, and the like.

### BACKGROUND ART

Serum and plasma can be collected via minimally invasive procedures and are the most primary specimens used in clinical testing. In fact, since most tests using biomarkers employ serum or plasma, discovery of novel biomarkers from serum or plasma is expected. Another advantage of screening for biomarkers in serum or plasma is that, since serum and plasma are collected in most biobanks, the time and effort required for collecting specimens can be saved by using these samples.

Despite extensive efforts in biomarker discovery via proteome analysis of serum and plasma, there has been limited success in identifying biomarkers that can be used in actual clinical practice. This is presumed to be because the dynamic range of protein concentrations in serum and plasma is extremely wide, such that only proteins abundant in serum and plasma can be analyzed, and a sufficient scope for biomarker discovery could not be secured. Of course, the likelihood of discovering promising biomarkers will certainly increase with improvement in the depth of proteomics analysis and broaden the scope of biomarker discovery (i.e., the number of detectable proteins). Therefore, to identify biomarker proteins using mass spectrometry, it is imperative to reduce the dynamic range and to conduct proteome analysis more deeply.

The most common method for reducing the dynamic range of protein concentrations in serum and plasma is to remove highly abundant proteins such as albumin, IgG, and transferrin using antibody columns (Non-Patent Documents 1 to 3). Although depletion of highly abundant proteins is an effective method, no antibody column can deplete all highly abundant proteins, making this method insufficient for detecting trace proteins in serum. One approach for overcoming this problem and studying trace proteins in serum and plasma, avoiding interference from highly abundant proteins, involves collecting and analysing extracellular vesicles via LC-MS (Non-Patent Document 4). In addition, the use of chemical nanoparticle cocktails for capturing trace proteins in serum and plasma has recently gained attention (Non-Patent Documents 5 and 6). On the other hand, target-based analysis using antibodies or aptamers is also advancing, with services offered by Olink and SomaLogic, enabling the simultaneous measurement of thousands of proteins in serum and plasma (Non-Patent Documents 7 and 8). This indicates that various technologies are still under development to broaden the scope of biomarker discovery.

More than 50% of the human proteome is estimated to be glycosylated (Non-Patent Document 9). Glycosylation is a highly complex posttranslational modification that plays pivotal roles in various biological phenomena such as cell differentiation, intercellular communication, and immunity (Non-Patent Documents 10 and 11). Many proteins reported as biomarkers for diseases, including cancer, are known to be glycosylated (Non-Patent Documents 12 to 14). However, there have been few cases in which biomarkers have been searched by LC-MS focusing on glycoproteins.

Lectin is a general term for proteins that reversibly bind to monosaccharides or glycans. A vast variety of lectins have been discovered from animals, plants, bacteria, viruses, and the like. Among these, plant lectins are classified into seven families as structurally and evolutionarily related proteins (Non-Patent Document 15). These families include (i) amaranthin lectin family, (ii) chitin-binding lectins, (iii) cucurbitaceae phloem lectins, (iv) jacalin-related lectins, (v) legume lectins, (vi) monocot mannose-binding lectins, and (vii) type 2 RIPs. Among these, the chitin-binding lectin family comprises plant proteins having at least one so-called hevein domain and is frequently found in higher plants. Examples of chitin-binding lectins include lectins from Solanaceae plants (such as *Solanum tuberosum, Lycopersicon esculentum,* and *Datura Stramonium*), Wheat Germ Agglutinin (WGA), *Urtica dioica* lectin, *Viscum album* lectin, *Chelidonium majus* lectin, and *Phytolacca americana* lectin (PWM) (Non-Patent Document 16). N-acetylglucosamine oligomer ((GlcNAc)n)-binding lectins are present in the families of chitin-binding lectins having a hevein domain, cucurbit phloem lectins, and legume lectins.

Lectins are useful for isolating and purifying glycoproteins and glycans, and various lectin columns are commercially available.

### PRIOR ART DOCUMENTS

### NON-PATENT DOCUMENTS

Non-Patent Document 1: Methods Mol Biol, 1002, 1-11 (2013)
Non-Patent Document 2: ACS Omega, 7, 7012-7023 (2022)
Non-Patent Document 3: Rheumatology (Oxford), 62, 3501-3506 (2023)
Non-Patent Document 4: Mass Spectrom Rev, 42, 779-795 (2023)
Non-Patent Document 5: Pediatr Hematol Oncol, 39, 658-671 (2022)
Non-Patent Document 6: Nat Commun, 11, 3662 (2020)
Non-Patent Document 7: Nat Commun, 12, 2493 (2021)
Non-Patent Document 8: Nat Commun, 12, 6822 (2021)
Non-Patent Document 9: Biochim Biophys Acta, 1473, 4-8 (1999)
Non-Patent Document 10: Cell, 126, 855-867 (2006)
Non-Patent Document 11: Nat Rev Cancer, 15, 540-555 (2015)
Non-Patent Document 12: Electrophoresis, 33, 1746-1754 (2012)
Non-Patent Document 13: Glycoconj J, 29, 249-258 (2012)
Non-Patent Document 14: Technol Cancer Res Treat, 22, 15330338221148811 (2023)
Non-Patent Document 15: Crit Rev Plant Sci, 17, 575-692 (1998)
Non-Patent Document 16: Trends in Glycoscience and Glycotechnology, 12, No. 64, 83-101 (2000)
Non-Patent Document 17: The Plant Journal, 37, 34-45 (2003)

### SUMMARY OF THE INVENTION

### PROBLEMS TO BE SOLVED BY THE INVENTION

The present invention aims to provide a method for preparing a sample for proteome analysis, which enables efficient recovery of trace proteins in a biological sample while removing highly abundant proteins that hinder deepening of analysis as much as possible in proteome analysis of a biological sample such as serum or plasma.

### MEANS TO SOLVE THE PROBLEMS

The present inventors have conducted intensive studies to solve the above problems and succeeded in recovering trace proteins with high efficiency while minimizing contamination of highly abundant proteins by applying a serum sample to a column on which a N-acetylglucosamine oligomer-binding lectin such as *Solanum tuberosum* lectin (STL), *Lycopersicon esculentum lectin* (LEL), *Datura stramonium* lectin (DSL), and wheat germ agglutinin (WGA), or a fucose-binding lectin such as *Ulex europaeus* agglutinin-1, *Aleuria aurantia* lectin (AAL), and *Aspergillus oryzae* lectin (AOL) are immobilized. Compared with a sample prepared by depletion of 14 highly abundant proteins using an antibody column (TOP14D), a sample prepared using an N-acetylglucosamine oligomer-binding lectin or a fucose-binding lectin enabled identification of a larger number of proteins by LC-MS/MS analysis. Although highly abundant proteins in serum are also glycosylated, their binding to N-acetylglucosamine oligomer-binding lectins or fucose-binding lectins was limited. On the other hand, trace proteins such as membrane proteins released into serum were preferentially enriched. It was expected that, by combining an N-acetylglucosamine oligomer-binding lectin with another lectin having different sugar specificity, glycoproteins having diverse glycan structures would be recovered and the number of identified proteins would be increased; however, unexpectedly, such an effect was hardly observed. Rather, the number of identified proteins was increased by combining a plurality of N-acetylglucosamine oligomer-binding lectins. In particular, when STL and LEL were combined, the number of identified proteins was significantly increased as compared with the case where STL or LEL was used alone, although both STL and LEL are classified as Solanaceae lectins and have similar sugar specificity, structure, and properties (Non-Patent Document 17). When proteins bound to a lectin column were eluted with SDS, recovery of the eluted proteins by an SP3 method was required to remove SDS that inhibits enzymatic reactions. However, by eluting proteins from the lectin column with an acidic aqueous solution and neutralizing the eluate with a buffer to return to a pH optimal for enzymatic digestion, enzymatic digestion and subsequent proteome analysis could be performed without performing the SP3 method step. Furthermore, when LMNG was added to the acidic aqueous solution, the recovery of eluted proteins from the lectin column was improved and the number of identified proteins was increased. Based on these findings, the present inventors have conducted further studies and completed the present invention.

Namely, the present invention relates to the following.
[1] A preparation method of a protein- or peptide-containing sample for identifying proteins contained in a biological sample by mass spectrometry, comprising contacting the biological sample with a carrier on which a lectin selected from the group consisting of N-acetylglucosamine oligomer-binding lectins and fucose-binding lectins, or a binding fragment thereof, is immobilized, to recover glycoproteins having binding affinity to the lectin in the biological sample.
[2] The preparation method of [1], wherein the lectin or the binding fragment thereof is an N-acetylglucosamine oligomer-binding lectin selected from the group consisting of *Solanum tuberosum* lectin, *Lycopersicon esculentum* lectin, *Datura stramonium* lectin, and wheat germ agglutinin, or a binding fragment thereof.
[3] The preparation method of [1], wherein the lectin or the binding fragment thereof is a fucose-binding lectin selected from the group consisting of *Ulex europaeus* agglutinin-1, *Aleuria aurantia* lectin, and *Aspergillus oryzae* lectin, or a binding fragment thereof.
[4] The preparation method of [1], wherein the lectin or the binding fragment thereof is a combination comprising:
   *1) Solanum tuberosum* lectin or a binding fragment thereof; and
   2) a lectin selected from the group consisting of *Lycopersicon esculentum* lectin, *Datura stramonium* lectin, wheat germ agglutinin, and *Aleuria aurantia* lectin, or a binding fragment thereof.
[5] The preparation method of [1], wherein the lectin or the binding fragment thereof is a combination comprising:
   *1) Solanum tuberosum* lectin or a binding fragment thereof; and
   *2) Lycopersicon esculentum* lectin or a binding fragment thereof.
[6] The preparation method of any of [1] to [5], further comprising subjecting the recovered glycoproteins in the biological sample to reductive alkylation.
[7] The preparation method of any of [1] to [6], further comprising digesting the recovered glycoproteins in the biological sample with a protease to obtain a peptide mixture as a digestion product.
[8] The preparation method of any of [1] to [7], further comprising applying the obtained peptide mixture to a desalting column to obtain a purified peptide mixture.
[9] The preparation method of any of [1] to [8], wherein the biological sample is selected from the group consisting of serum, plasma, whole blood, cerebrospinal fluid, ascites, synovial fluid, and lymph.
[10] The preparation method of any of [1] to [9], wherein the glycoproteins having binding affinity to the lectin in the biological sample are recovered by eluting proteins bound to the lectin or the binding fragment thereof with an acidic aqueous solution.
[11] The preparation method of [10], wherein the acidic aqueous solution contains a sugar-based nonionic surfactant.
[12] The preparation method of [11], wherein the sugar-based nonionic surfactant is LMNG.
[13] The preparation method of any of [10] to [12], further comprising neutralizing the eluate and treating it with a protease to digest the recovered glycoproteins in the biological sample in the eluate, thereby obtaining a peptide mixture as a digestion product.
[14] A method for identifying a protein contained in a biological sample by mass spectrometry, comprising the following steps:
   1) contacting the biological sample with a carrier on which a lectin selected from the group consisting of N-acetylglucosamine oligomer-binding lectins and fucose-binding lectins, or a binding fragment thereof is immobilized, to recover glycoproteins having binding affinity to the lectin in the biological sample;
   2) digesting the recovered glycoproteins in the biological sample with a protease to obtain a peptide mixture as a digestion product;
   3) subjecting the obtained peptide mixture to a desalting treatment to obtain a purified peptide mixture;
   4) subjecting the purified peptide mixture to a mass spectrometer to identify peptides contained in the peptide mixture based on the obtained mass spectrometry results; and
   5) identifying a protein in the biological sample from which the peptide is derived based on the information of the identified peptides.
[15] The method of [14], wherein the lectin or the binding fragment thereof is an N-acetylglucosamine oligomer-binding lectin selected from the group consisting of *Solanum tuberosum* lectin, *Lycopersicon esculentum* lectin, *Datura stramonium* lectin, and wheat germ agglutinin, or a binding fragment thereof.
[16] The method of [14], wherein the lectin or the binding fragment thereof is a fucose-binding lectin selected from the group consisting of *Ulex europaeus* agglutinin-1, *Aleuria aurantia* lectin, and *Aspergillus oryzae* lectin, or a binding fragment thereof.
[17] The method of [14], wherein the lectin or the binding fragment thereof is a combination comprising:
   *1) Solanum tuberosum* lectin or a binding fragment thereof; and
   2) a lectin selected from the group consisting of *Lycopersicon esculentum* lectin, *Datura stramonium* lectin, wheat germ agglutinin, and *Aleuria aurantia* lectin, or a binding fragment thereof.
[18] The method of [17], wherein the lectin or the binding fragment thereof is a combination comprising:
   *1) Solanum tuberosum* lectin or a binding fragment thereof; and
   *2) Lycopersicon esculentum* lectin or a binding fragment thereof.
[19] The method of any of [14] to [18], further comprising subjecting the glycoproteins in the biological sample recovered in step 1 to reductive alkylation.
[20] The method of any of [14] to [19], wherein the biological sample is selected from the group consisting of serum, plasma, whole blood, cerebrospinal fluid, ascites, synovial fluid, and lymph.
[21] The preparation method of any of [14] to [20], wherein, in step 1, the glycoproteins having binding affinity to the lectin in the biological sample are recovered by eluting proteins bound to the lectin or the binding fragment thereof with an acidic aqueous solution.
[22] The preparation method of [21], wherein the acidic aqueous solution contains a sugar-based nonionic surfactant.
[23] The preparation method of [22], wherein the sugar-based nonionic surfactant is LMNG.
[24] The preparation method of any of [21] to [23], wherein the eluate is neutralized and subjected to the digestion with the protease in step 2.

### EFFECTS OF THE INVENTION

According to the present invention, highly abundant proteins that hinder deepening of LC-MS analysis can be efficiently removed from a biological sample such as serum or plasma, and trace proteins useful as biomarkers can be preferentially enriched. Since a sample prepared by the method of the present invention contains a large amount of highly enriched trace proteins, contamination of highly abundant proteins is suppressed, and the dynamic range of protein concentrations is reduced, when it is used for LC-MS analysis, the depth of proteome analysis is improved, and a large number of proteins can be detected.

### BRIEF DESCRIPTION OF THE DRAWINGS

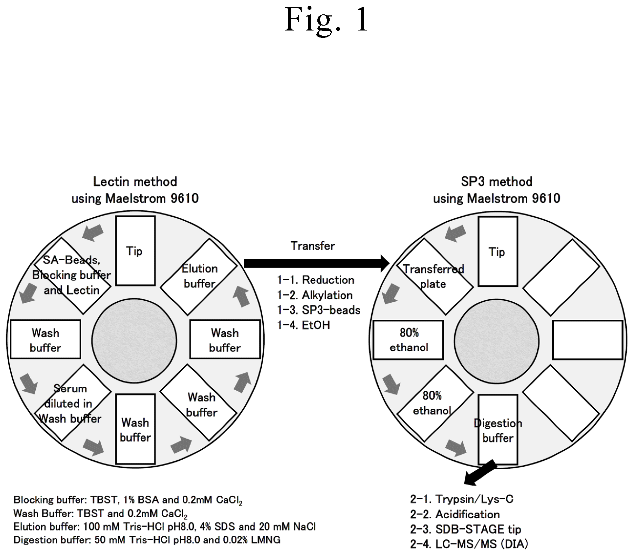

[Figure 1] Figure 1 is a schematic diagram showing an automated method for the enrichment of trace serum proteins using biotinylated lectins. Maelstrom 9610 is equipped to accommodate eight types of 96-deep well plates. One of these plates is required to be provided with a specific chip dedicated for use with this apparatus. Each plate can be rotated to a predetermined position beneath a 96-pin magnetic head. This head descends into the 96-well plate, facilitating the binding, release or mixing of magnetic beads in solution. Using Maelstrom 9610, the reaction of the biotinylated lectin with streptavidin beads, the reaction of the lectin-bound beads with serum, and washing between the respective steps were automatically processed. Serum enriched with lectin was subjected to reduction and alkylation, and then automatically processed by SP3 using Maelstrom 9610.
[Figure 2] Figure 2 is a graph showing the number of proteins detected in sera enriched with 37 different lectins. As a representative of a typical method, serum subjected to TOP14 Abundant Protein Depletion (TOP14D) was also analyzed. The treated samples were analyzed via LC-MS/MS with a 60-minute active gradient for 200 ng of peptides, following trypsin digestion. Horizontally striped, diagonally striped, and gray bars represent lectins having sugar specificity for N-acetylglucosamine, fucose, and others, respectively.
[Figure 3] Figure 3 is a graph showing the number of proteins detected in sera enriched with a combination of two or three lectins. As a control, serum treated with STL alone was also analyzed. Each treated sample was analyzed via LC-MS/MS with a 60-minute active gradient for 200 ng of peptides, followed by trypsin digestion.
[Figure 4] Figure 4 is a graph showing the number of proteins detected in STL/LEL -enriched sera from various species. For each species, 200 ng of trypsin-treated peptides were analyzed by LC-MS/MS with a 60-minute active gradient.
[Figure 5A] Figure 5A is a Venn diagram comparing the number of proteins observed in crude serum, TOP14D-treated serum, and STL/LEL-enriched serum via LC-MS/MS with a 120-minute active gradient. In each case, 500 ng of trypsin-treated peptides were analyzed. The numbers in the figure indicate the overlap of proteins identified by each treatment.
[Figure 5B] Figure 5B shows the cellular component in gene ontology (GO) enrichment analysis for proteins observed only in STL/LEL-enriched serum.
[Figure 6A] Figures 6A to D show analysis results of proteins in the serum of SLE model mice and control mice using STL/LEL method and TOP2D method. Figure 6A is a Pearson correlation coefficient heatmap for individual protein intensities between each sample.
[Figure 6B] Figure 6B is a volcano plot of protein intensities obtained from sera of SLE model mice and control mice. The red dots (dots in the upper right area of each graph) indicate proteins whose expression is increased in the sera of R-SLE model mice, and the blue dots (dots in the upper left area of each graph) indicate proteins whose expression is decreased in the sera of SLE model mice.
[Figure 6C] Figure 6C is a Venn diagram showing the overlap between altered proteins in sera of SLE model mice detected by the STL/LEL method and the TOP2D method.
[Figure 6D] Figure 6D is a graph showing disease ontology enrichment analysis of proteins upregulated in the sera of SLE model mice. Disease terms associated with SLE and its complications are shown.
[Figure 7] Figure 7 is a schematic diagram showing an improved automated enrichment method for trace serum proteins using a biotinylated lectin.
[Figure 8] Figure 8 is a graph comparing the number of proteins identified when proteins bound to a lectin column were eluted by the SDS-SP3 method, 0.2% TFA and 0.2% TFA/0.05% LMNG aqueous solutions, with the SDS-SP3 method.
[Figure 9] Figure 9 is a graph comparing the number of proteins identified when proteins bound to a lectin column were eluted using various types of acidic aqueous solutions.

### MODE FOR CARRYING OUT THE INVENTION

The present invention provides a preparation method of a protein- or peptide-containing sample for identifying proteins contained in a biological sample by mass spectrometry, comprising contacting the biological sample with a carrier on which a lectin selected from the group consisting of N-acetylglucosamine oligomer-binding lectins and fucose-binding lectins, or a binding fragment thereof, is immobilized, to recover glycoproteins having binding affinity to the lectin in the biological sample.

By treating a biological sample with a carrier on which a lectin selected from the group consisting of N-acetylglucosamine oligomer-binding lectins and fucose-binding lectins, or a binding fragment thereof, is immobilized, glycoproteins in the biological sample having binding affinity to the lectin (which may be N-acetylglucosamine oligomer-containing glycoproteins or fucosecontaining glycoproteins) are adsorbed onto the carrier, and the carrier is washed to remove non-adsorbed components, whereby highly abundant proteins that hinder deepening of analysis in proteome analysis are efficiently removed and trace proteins useful as biomarkers are preferentially enriched.

Examples of the biological sample used in the present invention include, but are not limited to, body fluids such as blood (serum, plasma, whole blood), cerebrospinal fluid, ascites, synovial fluid, lymph, saliva, urine, sweat, sputum, tears, nasal discharge, semen, pleural effusion, pericardial fluid, and interstitial fluid; cells; tissues or a part thereof; lysates of cells or tissues; extracts of cells or tissues; biopsy specimens; swab samples; feces; cell cultures; bacteria; viruses; and fungi. According to the preparation method of the present invention, highly abundant proteins such as albumin, immunoglobulin, and transferrin can be efficiently removed from a biological sample, and trace proteins can be preferentially enriched; therefore, the preparation method of the present invention is advantageous for preparing a trace protein-containing sample for proteome analysis from a biological sample containing abundant highly abundant proteins (for example, serum, plasma, whole blood, cerebrospinal fluid, ascites, synovial fluid, and the like), particularly from serum or plasma.

The biological sample may be one in which a specific biological substance fraction has been extracted or concentrated, or one that has been diluted with a buffer or the like. Since removal of highly abundant proteins (for example, albumin, IgG, antitrypsin, IgA, transferrin, haptoglobin, fibrinogen, α2-macroglobulin, α1-acid glycoprotein, IgM, apolipoprotein AI, apolipoprotein AII, complement protein C3, and transthyretin 9) in a biological sample by an affinity depletion system using an antibody column is commonly employed, a biological sample after removal of highly abundant proteins by such an antibody column may be used as a starting material in the present invention; however, according to the preparation method of the present invention, it is possible to prepare a sample for proteome analysis that contains trace proteins abundantly while suppressing contamination of highly abundant proteins without requiring such an antibody column. Accordingly, in one embodiment, the preparation method of the present invention uses a biological sample in which at least one highly abundant protein selected from the group consisting of albumin, IgG, antitrypsin, IgA, transferrin, haptoglobin, fibrinogen, α2-macroglobulin, α1-acid glycoprotein, IgM, apolipoprotein AI, apolipoprotein AII, complement protein C3, and transthyretin 9 has not been removed by antibody column treatment.

The species from which the biological sample is derived is not particularly limited, and examples include animals, plants, bacteria, fungi, and viruses. The kind of animal is also not particularly limited, and examples include mammals, birds, reptiles, amphibians, and fishes, with mammals or birds being preferred. Examples of mammals include rodents such as mice, rats, hamsters, and guinea pigs; lagomorphs such as rabbits; ungulates such as pigs, cattle, goats, horses, and sheep; carnivores such as dogs and cats; and primates such as humans, monkeys, rhesus macaques, cynomolgus monkeys, marmosets, orangutans, and chimpanzees. Mammals are preferably primates (such as humans) or rodents (such as mice).

Examples of N-acetylglucosamine oligomer-binding lectins include lectins from Solanaceae plants (such as *Solanum tuberosum, Lycopersicon esculentum,* and *Datura stramonium),* Wheat Germ agglutinin (WGA), *Urtica dioica* lectin, *Viscum album* lectin, *Chelidonium majus* lectin, and *Phytolacca americana* lectin (PWM) (Non-Patent Document 18) (Trends in Glycoscience and Glycotechnology, vol. 12, No. 64 (March 2000), pp. 83-101), but are not limited thereto. The N-acetylglucosamine oligomer-binding lectin is preferably *Solanum tuberosum* lectin (STL), *Lycopersicon esculentum* Lectin (LEL), *Datura stramonium* Lectin (DSL), or Wheat Germ Agglutinin (WGA), and more preferably *Solanum tuberosum* Lectin (STL) or *Lycopersicon esculentum* Lectin (LEL). A representative amino acid sequence of *Solanum tuberosum* lectin is shown in SEQ ID NO: 1, and a representative amino acid sequence of *Lycopersicon esculentum* lectin is shown in SEQ ID NO: 2. Amino acids from position 22 to position 323 of the amino acid sequence represented by SEQ ID NO: 1 may correspond to the mature *Solanum tuberosum* lectin. In addition, amino acids from position 24 to position 365 of the amino acid sequence represented by SEQ ID NO: 2 may correspond to the mature *Lycopersicon esculentum* lectin (The Plant Journal (2004) 37, 34-45; Biosci. Biotechnol. Biochem., 72, 80310-1-11, 2008).

Examples of fucose-binding lectins include *Ulex europaeus* agglutinin-1 (UEA-1), *Aleuria aurantia* lectin (AAL), and *Aspergillus oryzae* lectin (AOL), but are not limited thereto.

As used herein, N-acetylglucosamine oligomer refers to a polymer of N-acetylglucosamine in which N-acetylglucosamine units are linked to each other via β1-4 linkages. The length of the polymer is not particularly limited and includes dimers, trimers, tetramers, and the like. As used herein, when a lectin has binding affinity to a glycan having a specific structure, the dissociation constant (Kd value) of the binding affinity of the lectin to the glycan is generally 1×10⁻² M or less (for example, 1 × 10⁻³ M or less, 1×10⁻⁴ M or less, or 1 × 10⁻⁵ M or less). The binding affinity of an "N-acetylglucosamine oligomer-binding lectin" can be relatively compared among different types of lectins by evaluating binding affinity to an N-acetylglucosamine oligomer with a specific length (e.g., a tetramer). The binding affinity can be determined, for example, by using surface plasmon resonance (BIAcore(TM)) analysis or the like.

As used herein, a "binding fragment" of a lectin refers to a protein or peptide comprising a part (partial fragment) of the lectin and retaining the glycan-binding property of the parent lectin. In one embodiment, the "binding fragment" of a lectin comprises a contiguous partial sequence having a length of 30% or more (e.g., 40% or more, 50% or more, 60% or more, 70% or more, 80% or more, or 90% or more) of the full-length amino acid sequence of the mature form of the parent lectin.

In the present invention, a lectin selected from the group consisting of N-acetylglucosamine oligomer-binding lectins and fucose-binding lectins, or a binding fragment thereof, may be used alone as a single type; however, by using two or more types of lectins in combination, an increase in the number of proteins identified in proteome analysis can be expected. In one embodiment,
*1) Solanum tuberosum* lectin (STL) or a binding fragment thereof; and
2) a lectin selected from the group consisting of *Lycopersicon esculentum* lectin (LEL), *Datura stramonium* lectin (DSL), Wheat Germ Agglutinin (WGA), and *Aleuria aurantia* lectin (AAL), or a binding fragment thereof
are used in combination.

In particular, both *Solanum tuberosum* lectin (STL) and *Lycopersicon esculentum* lectin (LEL) belong to Solanaceae lectins and have similar sugar specificity, structure, and physicochemical properties (Non-Patent Document 19) (The Plant Journal, 37, 34-45 (2003)); however, as shown in the Examples described below, when STL and LEL are used in combination, the number of identified proteins is unexpectedly and significantly increased as compared with the case where STL or LEL is used alone. Therefore, in a preferred embodiment,
*1) Solanum tuberosum* lectin (STL) or a binding fragment thereof; and
*2) Lycopersicon esculentum* lectin (LEL) or a binding fragment thereof are used in combination.

When a plurality of lectins or binding fragments thereof are used in combination, although the mixing ratio is not particularly limited, preferably, they are combined such that the amount (molar amount) of the lectin or the binding fragment thereof having the highest content is 10 times or less (preferably 5 times or less, more preferably 2 times or less) relative to the amount of the lectin or the binding fragment thereof having the lowest content.

As used herein, the term "carrier" refers to a substance that serves as a base for adsorbing or immobilizing another substance. Examples of the carrier include beads, filters, cartridges, columns, and porous material plates (e.g., multi-well plates), but are not limited thereto. Examples of the beads include silica particles, agarose particles, polymer particles such as polystyrene, polyacrylamide, polydivinylbenzene, silicon or the like, magnetic particles, glass particles or the like, but are not limited thereto. Various functional groups may be bound to the surface of the carrier.

Immobilization of a lectin or a binding fragment thereof onto a carrier can be carried out by a protein immobilization method well known to those skilled in the art. For example, a lectin or a binding fragment thereof is biotinylated and mixed with a carrier having streptavidin bound thereto, whereby biotin on the lectin or the binding fragment thereof is bound to streptavidin on the carrier, and the lectin or the binding fragment thereof is thereby immobilized on the carrier. Alternatively, the lectin or a binding fragment thereof may be immobilized on the carrier using a crosslinking agent such as N-hydroxysuccinimide (NHS) and the like.

When a plurality of lectins or binding fragments thereof are used in combination, a mixture of the plurality of lectins or binding fragments thereof may be immobilized on a carrier, or each lectin or binding fragment thereof may be immobilized on a carrier individually, followed by mixing a plurality of types of carriers on which the lectins or binding fragments thereof have been immobilized.

In order to suppress non-specific binding of proteins to the surface of the carrier, the carrier on which the lectin or a binding fragment thereof is immobilized may be blocked with BSA, FBS, skim milk, Block Ace (manufactured by Dainippon Sumitomo Pharma Co., Ltd.), or the like.

By mixing the obtained carrier on which the lectin or a binding fragment thereof is immobilized with a biological sample, the biological sample is brought into contact with the carrier on which a lectin selected from the group consisting of N-acetylglucosamine oligomer-binding lectins and fucose-binding lectins, or a binding fragment thereof, is immobilized. As a result, glycoproteins in the biological sample having binding affinity to the lectin or the binding fragment thereof on the carrier are adsorbed onto the lectin or the binding fragment thereof on the carrier and are recovered. In order to suppress non-specific adsorption and contamination of highly abundant proteins, the carrier on which the glycoproteins are adsorbed may be washed with an appropriate buffer. Examples of the buffer include Tris-hydrochloric acid buffer, phosphate buffer, citrate buffer, acetate buffer, HEPES buffer, borate buffer, tartrate buffer, and the like, but are not limited thereto. The pH of the buffer is about 6 to 9. For effective washing, a surfactant may be added to the buffer. Examples of the surfactant include Tween 20, Triton X-100, NP-40, and the like, but are not limited thereto. An inorganic salt may also be added to the buffer. Examples of inorganic salt include sodium chloride, potassium chloride, calcium chloride, magnesium sulfate, and ammonium sulfate, and the like, but are not limited thereto.

The carrier on which glycoproteins are adsorbed onto the immobilized lectin or binding fragment thereof may be treated with an elution solution to release and elute the adsorbed glycoproteins into the elution solution. In one embodiment, the elution solution is an aqueous solution of a denaturant. Examples of the available denaturant include sodium dodecyl sulfate (SDS), urea, guanidine hydrochloride, and the like, but are not limited thereto. In another embodiment, the elution solution is an aqueous solution of a hapten sugar corresponding to the lectin or the binding fragment thereof immobilized on the carrier. For example, when an N-acetylglucosamine oligomer-binding lectin or a binding fragment thereof is used, the carrier is treated with an aqueous solution of an N-acetylglucosamine oligomer (e.g., dimer, trimer, tetramer, pentamer, or hexamer of N-acetylglucosamine or the like), and when a fucose-binding lectin or a binding fragment thereof is used, the carrier is treated with an aqueous solution of fucose. A buffer such as Tris-hydrochloric acid, phosphate, citrate, acetate, HEPES, borate, tartrate, and the like, and/or an inorganic salt such as sodium chloride, potassium chloride, calcium chloride, magnesium sulfate, ammonium sulfate, and the like may be added to the elution solution. From the viewpoint of avoiding loss of trace proteins in the sample, subsequent processing may be carried out without an elution step while maintaining N-acetylglucosamine oligomer-containing glycoproteins adsorbed on the carrier.

In a further aspect, the elution solution is an acidic aqueous solution. The pH of the acidic aqueous solution is generally 5 or less, preferably 4 or less, and more preferably 3 or less. The type of acidic aqueous solution is not particularly limited as long as it can release glycoproteins adsorbed to the lectin or the binding fragment thereof, and examples thereof include aqueous solutions of organic acids such as trifluoroacetic acid, acetic acid, formic acid, and the like, and aqueous solutions of inorganic acids such as hydrochloric acid, sulfuric acid, nitric acid, and the like. The concentration of the acid in the acidic aqueous solution is not particularly limited as long as it can release glycoproteins adsorbed to the lectin or the binding fragment thereof, but is usually 0.01 to 1.0% (w/w). The eluate containing the released glycoproteins obtained by elution with the acidic aqueous solution may be neutralized. The pH of the eluate after neutralization is preferably a pH at which the protease described below is active (preferably the optimum pH), and although it varies depending on the type of protease, it is usually within the range of 7.0 to 10.0. Neutralization can be carried out using a buffer well known to those skilled in the art (e.g., Tris-HCl) or an alkaline aqueous solution (e.g., an aqueous NaOH solution).

In order to suppress loss of the eluted glycoproteins and to improve the yield of the glycoproteins to be recovered, a surfactant may be added to the elution solution. The type of surfactant is not particularly limited; however, from the viewpoint of suppressing adsorption of glycoproteins and peptides to containers and the like while avoiding contamination of the mass spectrometer, a sugar-based nonionic surfactant is preferred. The sugar-based nonionic surfactant refers to a nonionic surfactant having a sugar residue as a hydrophilic group unit. Examples of the sugar-based nonionic surfactant include 2,2-didecylpropane-1,3-bis-β-D-maltopyranoside (lauryl maltose neopentyl glycol) (LMNG), 2,2-dioctylpropane-1,3-bis-β-D-maltopyranoside (decyl maltose neopentyl glycol) (DMNG), 2,2-dihexylpropane-1,3-bis-β-D-glucopyranoside (octyl glucose neopentyl glycol), n-dodecyl-β-D-maltoside (DDM), n-undecyl-β-D-maltoside, 3-oxatridecyl-α-D-mannoside, α-D-glucopyranosyl-α-D-glucopyranoside monododecanoate (trehalose C12) and the like, but are not limited thereto. Among these, LMNG has strong affinity for a reversed-phase column and is retained in the column even under elution conditions capable of eluting most peptides from the reversed-phase column; therefore, in the desalting step using the reversed-phase column, LMNG can be removed from peptides while avoiding loss of peptides, thereby minimizing the possibility of contamination of LC-MS instruments (WO 2024/127796 A1). The concentration of the sugar-based nonionic surfactant may be appropriately adjusted in consideration of the type of the sugar-based nonionic surfactant, the concentrations of glycoproteins or peptides in the eluate or the like; for example, it is 0.00125% (w/w) or more, 0.0025% (w/w) or more, 0.005% (w/w) or more, 0.01% (w/w) or more, or 0.05% (w/w) or more. The sugar-based nonionic surfactant is typically used at a concentration of 0.5% (w/w) or less, for example 0.1% (w/w) or less.

In order to cleave intermolecular and intramolecular disulfide bonds between cysteine residues in proteins and to prevent disulfide bond formation, the recovered glycoproteins may be subjected to reductive alkylation. Reduction of the glycoproteins can be carried out by heating the glycoproteins adsorbed on the carrier or eluted from the carrier together with a reducing agent such as 1,4-dithiothreitol (DTT), 2-mercaptoethanol, tris(2-carboxyethyl)phosphine or the like. Alkylation of the glycoproteins can be carried out by reacting the reduced glycoproteins with an alkylating reagent such as iodoacetamide, iodoacetic acid, acrylamide, chloroacetamide and the like. To promote reductive alkylation, a chaotropic agent may be added during the reduction treatment and/or the alkylation treatment. Examples of the chaotropic agent include urea, thiourea, guanidine hydrochloride, thiocyanate, sarcosine and the like, but are not limited thereto. After the reductive alkylation reaction, unreacted reducing agents and alkylating agents may be removed. For example, the carrier on which the reductively alkylated glycoproteins are adsorbed is washed with an appropriate buffer. As the buffer, those mentioned as buffers for washing the carrier on which glycoproteins are adsorbed can be used. The reductive alkylation treatment is an optional step, and may be omitted when emphasis is placed on rapidity and labor saving.

In order to facilitate subsequent processing operations of the eluted glycoproteins and to minimize sample loss during buffer exchange and the like, the glycoproteins may be adsorbed onto a new carrier. For example, an SP3 (singlepot solid-phase-enhanced sample preparation) method, in which glycoproteins are bound to a hydrophilic surface of beads coated with a carboxylic acid, is known (Mol. Syst. Biol. 2014, 10, 757). Adsorption onto the new carrier may be performed either before or after the reductive alkylation treatment.

When an acidic aqueous solution is used as the elution solution, by neutralizing the eluate to a pH at which a protease described below is active (preferably an optimum pH), and treating the eluate with the protease, the recovered proteins in the extract can be digested with the protease in the liquid phase to obtain a peptide mixture as a digestion product. In this case, since it is not necessary to adsorb the eluted proteins onto a new carrier to perform buffer exchange or to remove a denaturant or the like that inhibits the activity of the protease contained in the elution solution, the risk of sample loss associated with adsorption to a carrier may be avoided, and the number of identified proteins may be increased. Accordingly, in one embodiment, the eluted glycoproteins are not adsorbed onto a new carrier.

Next, the recovered glycoproteins (including those subjected to reductive alkylation) are digested with a protease. Specifically, the carrier on which the glycoproteins are adsorbed is incubated in an aqueous solvent containing a protease, or a protease is added to an eluate containing the glycoproteins and incubated. Examples of the protease include endoproteases such as trypsin, Glu-C, Lys-N, Lys-C, Asp-N, chymotrypsin and the like, but are not limited thereto. A plurality of types of proteases may be used in combination. It is preferable to add an appropriate buffer to the aqueous solvent for performing the digestion reaction in order to maintain pH. Examples of the buffer include Tris-hydrochloric acid, phosphate, citrate, acetate, HEPES, borate, and tartrate, but are not limited thereto. An inorganic salt may be added to the aqueous solvent. Examples of the inorganic salt include sodium chloride, potassium chloride, calcium chloride, magnesium sulfate, ammonium sulfate and the like, but are not limited thereto. A preferred aqueous solvent includes a buffered aqueous solution, for example, a Tris-HCl buffer (pH 8.0) and the like, but is not limited thereto. By the protease treatment, the glycoproteins are digested, and peptides as digestion products are released into the aqueous solvent.

When the carrier on which the glycoproteins are adsorbed is incubated in an aqueous solvent containing a protease, the carrier is removed from the digestion reaction mixture to recover a peptide mixture as a digestion product, and the recovered peptide mixture is used as a peptide-containing sample for identifying proteins contained in the biological sample by mass spectrometry. Specifically, for example, a digestion reaction mixture containing the carrier and an aqueous solvent containing the peptide mixture as the digestion product is centrifuged to precipitate the carrier, and the aqueous solvent containing the peptide mixture as the digestion product in the supernatant is recovered.

In order to analyze the obtained peptide mixture by a mass spectrometer, the peptide mixture is usually subjected to a desalting treatment to remove low-molecular-weight substances such as salts, buffers, chaotropic agents and the like, thereby obtaining a purified peptide mixture. Desalting treatment can be carried out by applying the aqueous solvent containing the peptide mixture as a digestion product to a reversed-phase column. Examples of the reversed-phase column include columns in which a hydrophobic group such as an octadecyl group (C18), octyl group (C8), butyl group (C3), phenyl group, or cyanopropyl group is bound to a solid-phase carrier (e.g., silica gel), and styrene-divinylbenzene (SDB) copolymer columns, but are not limited thereto. The reversed-phase column is preferably a column in which an octadecyl group (C18) is bound to a solid-phase carrier (e.g., silica gel) (referred to as a C18 column) or an SDB copolymer column. A commercially available reversed-phase column may be used, and a commercially available kit such as EVOSEP ONE or an SDB-STAGE tip may also be used.

When an aqueous solvent containing a peptide mixture is applied to a reversed-phase column, the peptide mixture is adsorbed onto the reversed-phase column, while low-molecular-weight substances such as salts, buffers, and chaotropic agents are not bound to the column and are eluted and removed. Subsequently, the peptide mixture adsorbed onto the reversed-phase column can be eluted to obtain a purified peptide mixture. The solvent used for elution of the peptides is usually a mixed solvent of water and an organic solvent such as acetonitrile, methanol, tetrahydrofuran, isopropanol, acetone and the like. To promote protonation, it is preferable to add an acid such as trifluoroacetic acid, formic acid, acetic acid, hydrochloric acid and the like to the water.

The eluted peptide mixture is dried to remove the solvent. Drying of the peptide mixture can be carried out, for example, using a centrifugal evaporator.

The obtained dry peptide mixture is re-dissolved in water. In order to promote protonation, an acid such as formic acid, trifluoroacetic acid, acetic acid, hydrochloric acid and the like may be added to the water. An organic solvent such as acetonitrile, methanol, tetrahydrofuran, isopropanol, acetone and the like may also be added to the water. In addition, to accelerate dissolution of the dry peptide to water and to suppress adsorption of the dissolved peptide to a tube, a surfactant such as LMNG, DMNG, DDM and the like may be added to the water. For example, the dry peptide mixture is dissolved in water containing 0.1% (v/v) TFA and 0.01% (w/v) DMNG to obtain an aqueous solution of the peptide mixture.

Next, the obtained aqueous solution of the peptide mixture is subjected to mass spectrometry. Based on the obtained mass spectrometry results, peptides contained in the peptide mixture are identified, and proteins in the biological sample from which the peptides are derived are identified based on the information of the identified peptides. Mass spectrometry of the peptide mixture is usually carried out using a liquid chromatography-mass spectrometer (LC-MS), and preferably carried out using a tandem mass spectrometer (LC-MS/MS) connected online with liquid chromatography. The measurement mode can be appropriately selected depending on the measurement purpose; for example, in comprehensively identifying proteins contained in a biological sample by nontargeted proteomics, measurement modes such as data-dependent acquisition (Data Dependent Acquisition: DDA) and data-independent acquisition (Data Independent Acquisition: DIA) are selected, and in identifying or quantifying specific proteins by targeted proteomics, measurement modes such as S/MRM (Selected or Multiple Reaction Monitoring) may be selected.

In DDA, a plurality of precursor ions having high signal intensities are selected in descending order, and acquisition of product ion spectra is automatically performed. From the obtained data, a file (peak list) is created in which the product ion spectra are linked to peaks on the MS spectrum, and a database search is performed. In the database search, first, peptide sequences obtained by enzymatic digestion of all protein sequences registered in an arbitrary sequence database are theoretically predicted (for example, in the case of trypsin, cleavage occurs at the C-terminal side of K/R), and candidate peptide sequences having masses matching the precursor ions are narrowed down. Next, theoretical product ion spectra obtained by calculating, for all peptide bonds, m/z values of N-terminal ions (b-ions) and C-terminal ions (y-ions) generated when peptide bonds in the candidate peptide sequences are cleaved at one site are compared with measured product ion spectra to identify a specific peptide sequence.

In DIA, without selecting precursor ions, acquisition of mixed product ion spectra of all precursor ions falling within a Q1 window of a set m/z width is repeatedly performed while shifting the window. In DIA, chromatograms of fragment ions derived from peptide sequences of interest are extracted using an MS/MS information library created from separately acquired DDA data, and chromatographic peaks of target peptides are identified based on co-elution patterns of the fragment ions.

In S/MRM, a combination of the m/z of the precursor ion of a target peptide identified by DDA and the m/z of fragments generated by CID is specified in a measurement method, and ions of the target peptide in the sample (signals of fragment ions) are selectively monitored to obtain a chromatogram.

In DDA, when a large number of analytes are eluted simultaneously and their abundances differ greatly, there is a relatively high risk that trace analytes are not detected in the initial MS spectrum, or that the speed of the mass spectrometer cannot keep up with acquiring MS/MS spectra for all peaks detected in MS mode (i.e., it is too slow relative to the complexity of the sample). In contrast, in DIA, all MS/MS spectra of all detectable analytes passing through each Q1 window are collected, and since analysis of the entire mass range is performed within the LC analysis time, complete MS and MS/MS spectra of substantially all detectable peaks in the sample can be obtained, which is advantageous for detection and identification of trace analytes. In the preparation method of the present invention, affinity purification using a carrier on which an N-acetylglucosamine oligomer-binding lectin or a fucose-binding lectin, or a binding fragment thereof, is immobilized enables efficient removal of highly abundant proteins such as albumin from a biological sample while maintaining trace proteins, thereby allowing preparation of a peptide-containing sample having a high content of peptides derived from trace proteins. Therefore, by subjecting the peptide-containing sample prepared by the preparation method of the present invention to LC-MS/MS analysis in a DIA measurement mode, trace proteins contained in the biological sample and peptides derived therefrom can be detected and identified with higher sensitivity, thereby achieving more sensitive and deeper proteome analysis.

Accordingly, the preparation method of the present invention can also be regarded as a method for identifying proteins contained in a biological sample by mass spectrometry, comprising the following steps:
1) contacting the biological sample with a carrier on which a lectin selected from the group consisting of N-acetylglucosamine oligomer-binding lectins and fucose-binding lectins, or a binding fragment thereof is immobilized, to recover glycoproteins having binding affinity to the lectin in the biological sample;
2) digesting the recovered glycoproteins in the biological sample with a protease to obtain a peptide mixture as a digestion product;
3) subjecting the obtained peptide mixture to a desalting treatment to obtain a purified peptide mixture;
4) subjecting the purified peptide mixture to a mass spectrometer to identify peptides contained in the peptide mixture based on the obtained mass spectrometry results; and
5) identifying a protein in the biological sample from which the peptide is derived based on the information of the identified peptides.

In one embodiment, in step 1, glycoproteins having binding affinity to the lectin in the biological sample are recovered by eluting proteins bound to the lectin or a binding fragment thereof with an acidic aqueous solution. The eluate may be neutralized and subjected to digestion with the protease in step 2.

As described in detail in the Examples, by subjecting a peptide-containing sample prepared from human serum by the preparation method of the present invention to mass spectrometry, proteins listed in Tables 2-1 to 2-12, which could not be detected by conventional depletion of highly abundant proteins, could be identified. Accordingly, in one embodiment, by subjecting a peptide-containing sample prepared from a human-derived biological sample (preferably human serum, plasma, or whole blood) by the preparation method of the present invention to mass spectrometry, at least one protein selected from the proteins listed in Tables 2-1 to 2-12 in the biological sample, for example, at least 5, 10, 20, 50, 100, 200, 300, 400, 500, or 1000 proteins, is identified.

All references cited in the present specification, including publication, patent document and the like, are hereby incorporated individually and specifically by reference, to the extent that the entireties thereof have been specifically disclosed herein.

The present invention is explained in more detail in the following by referring to Examples, which are not to be construed as limitative.

### EXAMPLES

### [Example 1]

### 1. Experimental Procedures

### Serum

Three distinct lots of pooled human sera were purchased from KOJIN BIO (Saitama, Japan). These three serum samples were pooled and employed in the experiments. Normal sera from mice, rats, cattle, rabbits, goats, sheep, and chickens were purchased from TK Craft (Gunma, Japan). Sera from female (NZB × NZW) F1 hybrid mice with spontaneous polygenic autoimmune disease, serving as a systemic lupus erythematosus (SLE) model, and sera from presymptomatic mice, serving as control mice, were purchased from Hooke Laboratories (Lawrence, MA, USA).

### Preparation of Serum Samples

In the lectin enrichment method, glycoproteins in serum were automatically enriched with lectins using Maelstrom 9610 (Taiwan Advanced Nanotech, Taoyuan, Taiwan). Initially, 950 µL of blocking buffer (TBST, 1% BSA, 0.2 mM CaCl₂) was added to 50 µL of a streptavidin (SA) bead suspension (CAT# 21152104010350; Cytiva, Marlborough, MA, USA). Subsequently, 40 µL of a 0.5 µg/µL biotinylated lectin (Table 1) was added.

**[Table 1-1]**

| **Lectin name** | **Abbreviated name** | **Sugar Specificity** | **Com pany** | **Cat #** |
|---|---|---|---|---|
| Galanthus Nivalis Lectin | GNL | αMan | VL | B-1245-2 |
| Maackia Amurensis Lectin I | MAL-I | Galβ4GlclNAc | VL | B-1315-2 |
| Griffonia Simplicifolia Lectin I isolectin B4 | GSL-I-B4 | αGal | VL | B-1205.5 |
| Narcissus Pseudonarcissus Lectin | NPL | Man α | VL | B-1375-2 |
| Wisteria Floribunda Lectin | WFA | GlcNAc | VL | B-1355-2 |
| Aleuria Aurantia Lectin | AAL | Fucα 6 GlcNAc | VL | B-1395-1 |
| Maackia Amurensis Lectin II | MAL-II | Neu5Acα3GalβGalNAc | VL | B-1265-1 |
| Vicia Villosa Lectin | VVL | GalNAc | VL | B-1235-2 |
| Griffonia simplicifolia Lectin I | GSL-I | αGal, αGalNAc | VL | B-1105-2 |
| Pisum sativum agglutinin | PSA | αMan, αGlc | VL | B-1055-5 |
| Lens culinaris agglutinin | LCA | αMan, αGlc Galβ4GlcNAcβ2Manα6(Gl | VL | B-1045-5 |
| Phaseolus vulgaris Erythroagglutinin | PHA-E | cNAcβ4), (GlcNAcβ4Manα3), Manβ4 | VL | B-1125-2 |
| Phaseolus vulgaris Leucoagglutinin | PHA-L | Galβ4GlcNAcβ6, GlcNAcβ2 Manα3, Manα3 | VL | B-1115-2 |
| Succinylated Wheat germ agglutinin | sWGA | GlcNAc | VL | B-1025S-5 |
| Datura Stramonium lectin | DSL | (GlcNAc)2-4 | VL | B-1185-2 |
| Erythrina cristagalli lectin | ECL | Galβ4GlcNAc | VL | B-1145-5 |
| Griffonia (Bandeiraea) simplicifolia Lectin II | GSL-II | αGlcNAc 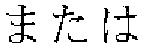 | VL | B-1215-2 |
| | | βGlcNAc | | |
| Jacalin | Jacalin | Galβ3GalNAc | VL | B-1155-5 |
| Lycopersicon esculentum (tomato) lectin | LEL | (GlcNAc)2-4 | VL | B-1175-1 |
| Solanum tuberosum (potato) lectin | STL | (GlcNAc)2-4 | VL | B-1165-2 |
| Concanavalin | Con-A | αMan, αGlc | VL | B-1005-5 |
| Soybean agglutinin | SBA | α>β GalNAc | VL | B-1015-5 |
| Wheat germ agglutinin | WGA | GlcNAc | VL | B-1025-5 |

**[Table 1-2]**

| | | | | |
|---|---|---|---|---|
| Dolichos biflorus agglutinin | DBA | GalNAc α | VL | B-1035-5 |
| Ulex europaeus agglutinin | UEA-I | Fuc α | VL | B-1065-2 |
| Ricinus communis agglutinin | RCA-I | Gal | VL | B-1085-5 |
| Peanut agglutinin | PNA | Galβ3GalNAc | VL | B-1075-5 |
| Sambucus Nigra Lectin | SNA | Neu5Acα6GaIIGaINAc | VL | B-1305-2 |
| Lotus Tetragonolobus Lectin | LTL | αFuc | VL | B-1325-2 |
| Agaricus bisporus agglutinin | ABA | β-D-Gal, D-GalNAc | CB | J202 |
| Erythrina cristagalli agglutinin | ECA | D-GalNAc, D-Gal | CB | J206 |
| Sambucus sieboldiana agglutinin | ABA | Siaα2-6Gal/GalNAc | CB | J218 |
| Aspergillus oryzae lectin | AOL | Fucα(1-6)/α(1-4)/α(1-3)/α(1-2) | TCI | A2659 |
| Griffithsia sp. Lectin | GRFT | Manα(1-2)Man | TCI | R0234 |
| Sclerotium rolfsii lectin | SRL | GlcNAcβ(1-2)Man, Galβ(1-3)GalNAc | TCI | R0233 |
| Marasmius oreades Agglutinin | MOA | Galα(1-3)Gal | TCI | R0232 |
| Polyporus squamosus lectin | PSL1a | Siaα(2-6)Gal | TCI | R0230 |

| | | | | |
|---|---|---|---|---|
| Sugar specificity is described according to the manufacturer's instructions. VL:Vector Laboratories, CB: Cosmo Bio, TCI: Tokyo Chemical Industry | | | | |

For mixtures of two lectins, 20 µL of each lectin was added, and for mixtures of three lectins, 13.3 µL of each lectin was added. The mixture was agitated for 30 minutes, and the beads were washed with a wash buffer (TBST and 0.2 mM CaCl₂). Next, 50 µL of serum, diluted in 150 µL of wash buffer, was mixed with the beads for 60 minutes. The beads were subsequently washed three times with 1000 µL of wash buffer and then mixed in 200 µL of elution buffer (100 mM Tris-HCl pH 8.0, 4% SDS, 20 mM NaCl) for 10 minutes, followed by removal of the beads.

For the depletion of highly abundant proteins, human serum was processed using Top14 Abundant Protein Depletion Mini Spin Columns (Thermo Fisher Scientific, Waltham, MA, USA) according to the manufacturer's instructions, and mouse serum was processed using Proteome Purify 2 Mouse Serum Protein Immunodepletion Resin (R&D Systems, Minneapolis, MN, USA) according to the manufacturer's instructions.

### Protein Digestion

The treated serum was treated with 20 mM tris(2-carboxyethyl)phosphine at 80°C for 10 minutes and then alkylated with 35 mM iodoacetamide at room temperature for 30 minutes and protected from light, followed by cleanup by SP3 and digestion using Maelstrom 9610. Briefly, two types of Sera-Mag SpeedBead carboxylate-modified magnetic particles (hydrophilic particles, CAT# 45152105050250; hydrophobic particles, CAT# 65152105050250; Cytiva) were used. These beads were combined at a ratio of 1:1 (v/v), washed twice with distilled water, and reconstituted with distilled water at a concentration of 8 µg solid/µL. 20 µL of the reconstituted beads (SP3 beads) was added to the alkylated protein sample, followed by addition of 99.5% ethyl alcohol to reach a final concentration of 75% (v/v), and the mixture was agitated for 15 minutes. The supernatant was discarded, and the pellet was washed twice with 80% ethanol. The beads were suspended in 80 µL of 50 mM Tris-HCl (pH 8.0) and 10 mM CaCl₂ (containing 0.02% LMNG) (20). Thereafter, 500 ng of trypsin/Lys-C Mix (CAT# V5072, Promega, Madison, WI, USA) was added, and the mixture was gently mixed at 37°C overnight to digest the proteins. The digested sample was acidified with 20 µL of 5% trifluoroacetic acid (TFA) and then sonicated at room temperature for 5 minutes at high power using a Bioruptor II (Cosmo Bio, Tokyo, Japan). The sample was desalted using an SDB-STAGE tip (GL Sciences, Tokyo, Japan). The SDB-STAGE tip was washed with 25 µL of 80% ACN in 0.1% TFA and equilibrated with 50 µL of 3% ACN in 0.1% TFA. Next, the sample was loaded onto the tip, washed with 80 µL of 3% ACN in 0.1% TFA, and eluted with 50 µL of 36% ACN in 0.1% TFA. The eluate was dried using a centrifugal evaporator (miVac Duo concentrator). The dried sample was re-dissolved in 0.1% TFA containing 0.01% DMNG. The re-dissolved sample was assayed for peptide concentration using a Lunatic instrument (Unchained Labs, Pleasanton, CA, USA), and the sample was transferred to an LC vial (Thermo Fisher Scientific).

### LC-MS/MS and DIA

The re-dissolved peptides were directly injected at 50°C into a 75 µm × 12 cm nanoLC column (Nikko Technos Co., Ltd., Tokyo, Japan) and separated using an UltiMate 3000 RSLCnano LC system at a flow rate of 200 nL/min with a 60-minute gradient consisting of 0 min 6% B, 50 min 36% B, 57 min 70% B, and 60 min 70% B (A = 0.1% FA aqueous solution, B = 0.1% FA in 80% ACN). The peptides eluted from the column were analyzed using a Q-Exactive HF-X with InSpIon system (J Proteome Res 22, 1564-1569, 2023). The precursor range for DIA was defined based on previously reported parameters (J Proteome Res 21, 1418-1427, 2022). MS1 spectra were collected in the range of 495-745 m/z at a resolution of 15,000, with an automatic gain control target of 3 × 10⁶ and a maximum injection time of 23 ms. MS2 spectra were collected at more than 200 m/z at a resolution of 30,000, with an automatic gain control target of 3 × 10⁶, a maximum injection time set to "auto", and a normalized collision energy of 23%. The isolation width for MS2 was set to 4 m/z, and for the 500-740 m/z window pattern, an optimized window arrangement was employed in Scaffold DIA (Proteome Software, Inc., Portland, OR, USA).

For deep proteome analysis, the re-dissolved peptides were directly injected into a 75 µm × 30 cm nanoLC column (CoAnn Technologies, Richland, WA, USA) at 60°C. The separation was conducted using an UltiMate 3000 RSLCnano LC system at a flow rate of 200 nL/min with a 120-minute gradient (A = 0.1% FA aqueous solution, B = 0.1% FA in 80% ACN) consisting of 0 min 3% B, 108 min 33% B, 114 min 65% B, and 120 min 65% B. The peptides eluted from the column were analyzed on an Orbitrap Exploris 480 equipped with an InSpIon system. MS1 spectra were collected in the range of 495-745 m/z at a resolution of 15,000, with an automatic gain control target of 3 × 10⁶ and a maximum injection time set to "auto." MS2 spectra were collected in the a range of 200-1,800 m/z at a resolution of 45,000, with an automatic gain control target of 3 × 10⁶, a maximum injection time set to "auto", and stepped normalized collision energies set at 22%, 26%, and 30%. The isolation width for MS2 was configured at 4 m/z, and for the 500-740 m/z window pattern, an optimized window arrangement was utilized in Scaffold DIA.

### Data Analysis

The DIA-MS files were queried against the in-silico human or mouse spectral library, using DIA-NN (version 1.8.1,
https://github.com/vdemichev/DiaNN) (Nat Methods 17, 41-44, 2020). Initially, the spectral library was generated using DIA-NN from the UniProt database for human or mouse protein sequences.

The parameters for generating the spectral library were as follows:
digestion enzyme, trypsin;
missed cleavages, 1;
peptide length range, 7-35;
precursor charge range, 2-4;
precursor m/z range, 495-745;
fragment ion m/z range, 200--1800; and
"FASTA digest for library-free search/library generation", "deep learning-based spectra, RTs, and IM prediction", "n-term M excision", and "C carbamidomethylation" were enabled.

The DIA-NN search parameters were as follows:
mass accuracy, 10 ppm;
MS1 accuracy, 10 ppm;
protein inference, gene;
neural network classifier, single-pass mode;
quantification strategy, robust LC (high precision);
cross-run normalization, off; and
"unrelated runs", "use isotopologues", "heuristic protein inference", and "no shared spectra" were enabled.

MBR was turned off. The threshold for protein identification was set at 1% or less for both precursor and protein FDR. Protein quantification values were aggregated over the quantification values of unique peptides as calculated by DIA-NN. Protein quantification data were converted to log2 (protein intensity), and filtering was conducted to ensure that for each protein at least one group contained a minimum of 70% valid values. The remaining missing values were imputed with random numbers drawn from a normal distribution (width, 0.3; downshift, 1.8) using Perseus v1.6.15.0 (24). The threshold for identifying altered proteins was defined as a change of more than two-fold between two groups and a difference with p < 0.05 (Welch's t-test). GO enrichment analysis was conducted using DAVID (https://david.ncifcrf.gov/tools.isp). Enrichment analysis of disease ontology (based on the HumanPSD database) was conducted using the geneXplain platform (GeneXplain GmbH, Wolfenbüttel, Germany).

### 2. Results and Discussion

### Enrichment of Trace Proteins from Serum by Lectins

The present inventors first established a method for the automated enrichment of trace proteins in serum using lectins (Figure 1). Employing an automated instrument for magnetic beads, Maelstrom 9610, biotinylated lectins were reacted with streptavidin magnetic beads, and the beads were then reacted with serum to enrich lectin-binding proteins. In addition, the SP3 method was also automated using Maelstrom 9610. This system allowed for the concurrent and automated processing of 96 samples, making it possible to evaluate many conditions. The method established by this system allowed for the concurrent processing of 96 samples, making it suitable for processing many samples such as clinical specimens. Owing to the limited variety of commercially available lectin-immobilized beads, commercially available lectin-immobilized beads were not used in this study; instead, biotinylated lectins, widely available on the market, were bound to streptavidin magnetic beads. Although the enrichment in this study focused on lectins, the same system can be applied to automated affinity purification using other biotinylated substances.

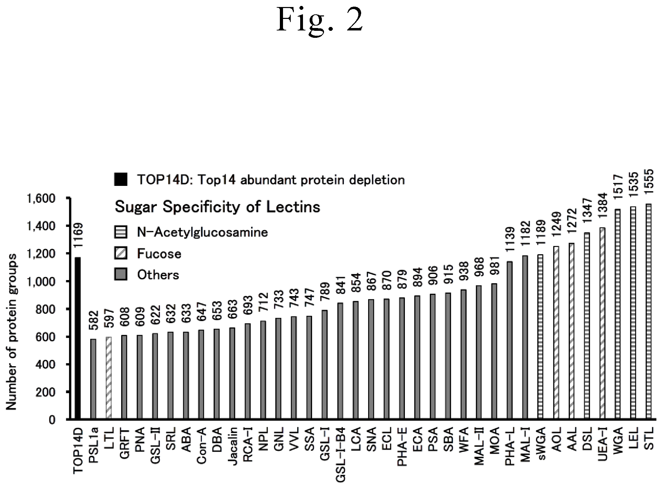

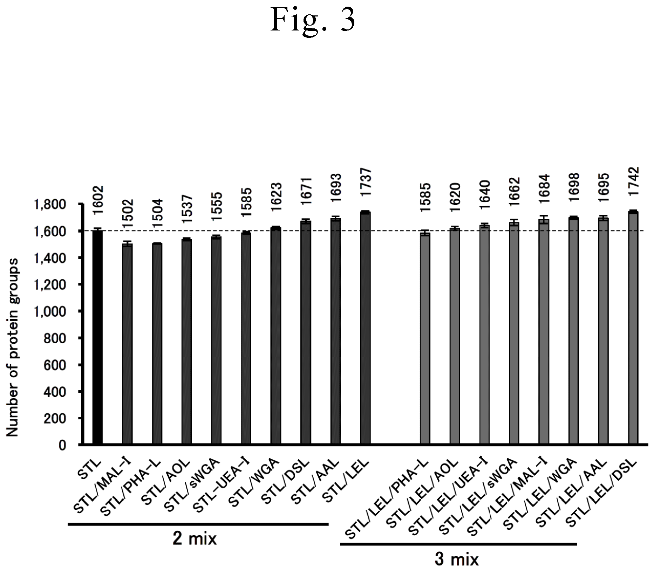

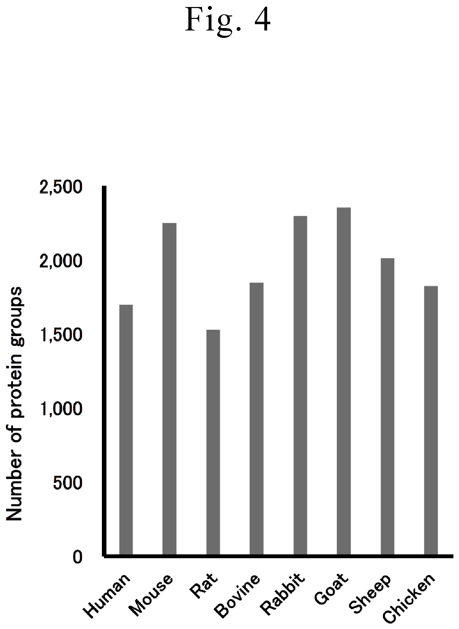

Using this automated method, the present inventors attempted to recover trace proteins in serum using 37 different lectins (Table 1). For this experiment, commercially available pooled sera were employed, and proteins recovered by the lectins were subsequently trypsin-digested and subjected to LC-MS/MS (Figure 2). As a control, serum from which TOP14 proteins had been removed (TOP14D) was used. Lectins that enabled identification of more proteins than TOP14D were STL, LEL, WGA, UEA-I, DSL, AAL, AOL, sWGA, and MAL-I. The present inventors found that these lectins allowed for enrichment of trace proteins beyond the typical performance of TOP14D. These lectins exhibited the property of recognizing N-acetylglucosamine or fucose. Among them, STL and LEL, which showed high numbers of identified proteins (IDs), are lectins that strongly recognize N-acetylglucosamine oligomers, indicating that targeting these proved crucial for recovering trace proteins in serum. Accordingly, the present inventors then examined whether the number of proteins observed could be further increased by combining STL, which showed the highest number of identifications, with other lectins (Figure 3). The combination of STL and LEL surpassed the performance of STL alone in the number of identified proteins and was the best combination with the highest number of identified proteins. Furthermore, the present inventors examined the effect of adding another lectin to STL and LEL. As a result, STL/LEL/DEL showed the highest number of protein IDs; however, the number of identified proteins was almost the same as that of STL/LEL, and there was little advantage in combining three lectins. On the basis of these results, it was concluded that STL/LEL is the optimal combination for enriching trace proteins in serum using lectins. Next, the present inventors further investigated whether this STL/LEL enrichment method could be adapted to use samples from non-human animal species (Figure 4). As a result, 1500 or more proteins were identified in all tested animal species, confirming that trace proteins can be enriched irrespective of the animal species. Since there are limited commercially available antibody-based depletion columns for species other than humans and mice, the applicability of this method to various animal species represents a significant advantage.

### Deep Proteomics Using the STL/LEL Enrichment Method

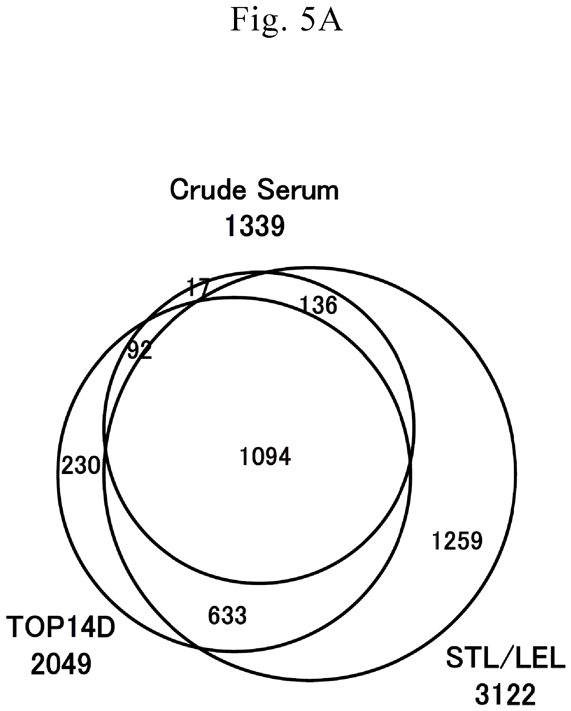

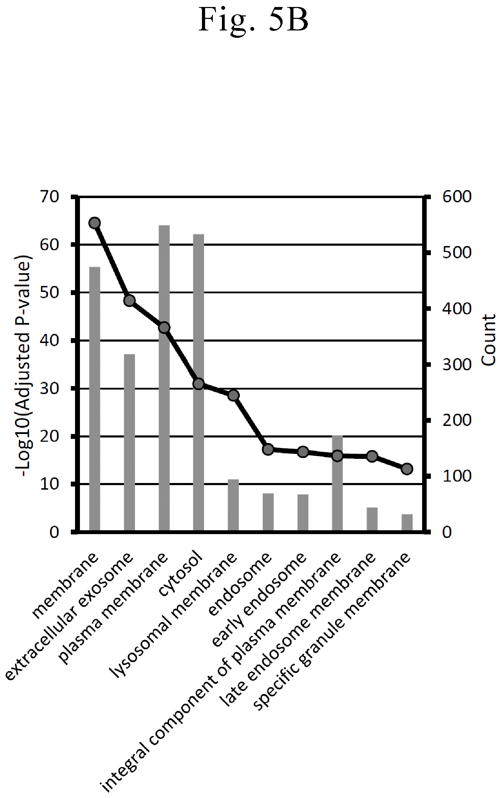

In the experiments for establishing the STL/LEL method, a 60-minute active gradient was employed; however, this was extended to 120 minutes and the conditions were adjusted for detecting trace serum proteins. Figure 5A compares crude serum, TOP14D-treated serum, and STL/LEL-enriched serum within this 120-minute gradient. A total of 1339, 2049, and 3122 proteins were identified in the crude serum, TOP14D-treated serum, and STL/LEL-enriched serum, respectively. Proteins that were not detected in the crude serum or TOP14D-treated serum but were detected only in the STL/LEL-enriched serum are shown in Tables 2-1 to 2-12. GO analysis of proteins exclusively detected in the STL/LEL-enriched serum indicated that membrane proteins were recovered. Since membrane proteins are known for their extensive glycosylation (Electrophoresis 37, 1407-1419, 2016), it was speculated that STL/LEL enriched membrane proteins released into the serum. It was also confirmed that interleukin receptors such as IL1R1, IL2RA, IL2RB, IL2RG, IL3RA, IL10RB, IL15RA, IL17RA, IL17RC, and IL27RA were enriched by STL/LEL.

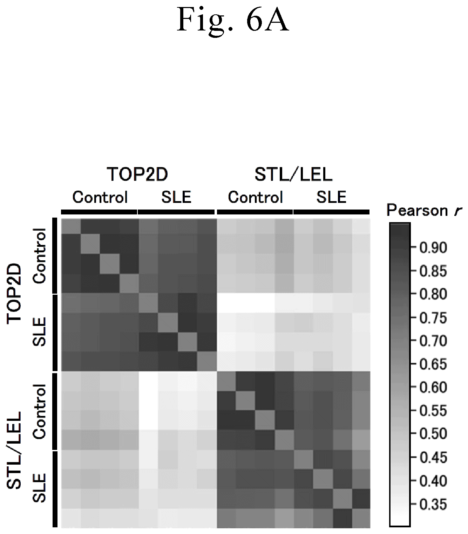

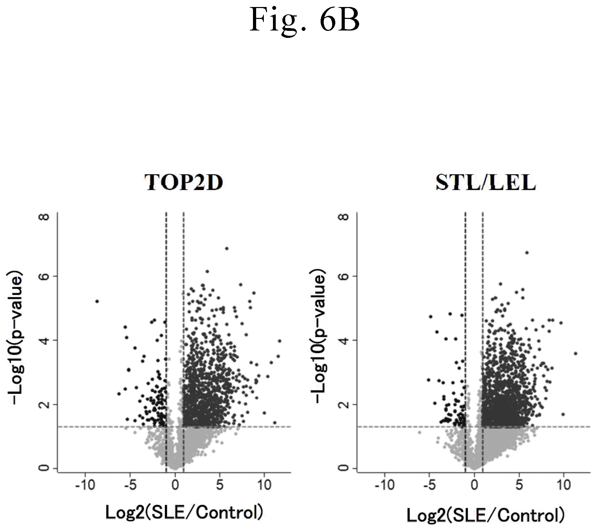

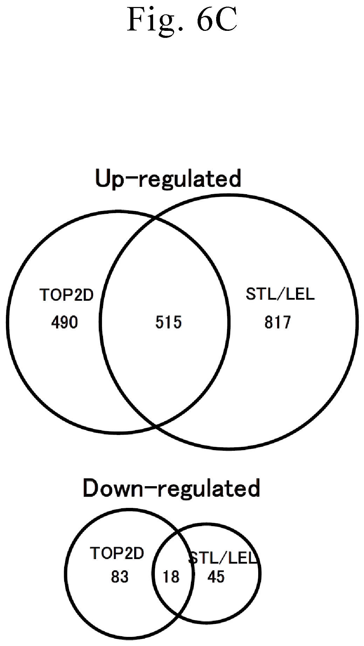

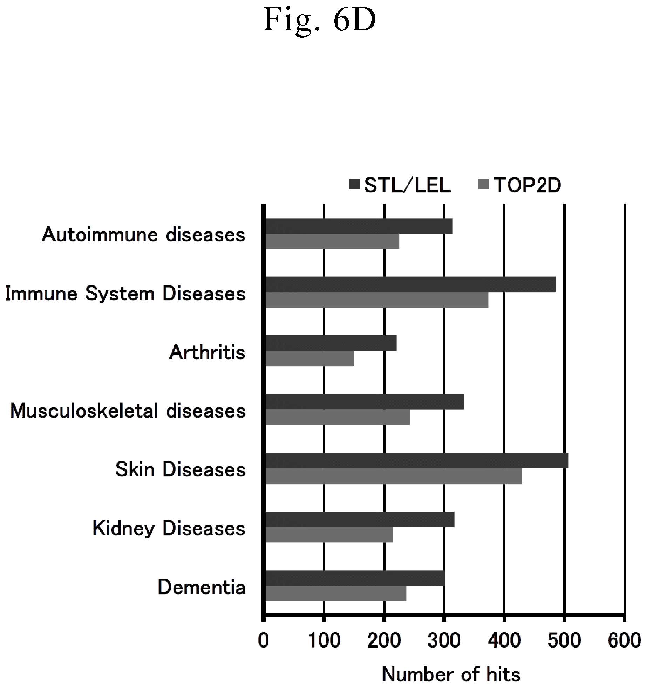

Next, sera from SLE model mice and control mice were compared using the STL/LEL method and a 120-minute active gradient LC-MS/MS method. In addition, to evaluate the STL/LEL method, the same samples were treated with the top 2 abundant protein depletion (TOP2D) method. A comparison of protein profiles in each group showed that the differences between the STL/LEL method and the TOP2D method were more significant than the differences between the groups of mice, confirming that the STL/LEL method differs from the conventional methods (Figure 6A). The number of proteins showing fluctuation in serum of SLE model mice was 1,332 with the STL/LEL method and 1,005 with the TOP2D method, indicating that the STL/LEL method detected a greater number of fluctuations (Figure 6B). In addition, many of the altered proteins that did not overlap between the respective methods were also identified (Figure 6C), indicating that biomarkers can be explored from a different perspective than that of the TOP2D method. In addition, a disease enrichment analysis was conducted for proteins increased in SLE (Supplementary Table 3). Figure 6D displays the results for disease terms associated with SLE and its complications. In all diseases shown, a greater number of proteins were categorized by the STL/LEL method, indicating that the STL/LEL method has a higher potential to capture the essence of the disease compared to the TOP2D method. These results suggest that the STL/LEL enrichment method is useful for biomarker discovery.

### 3. Conclusion

To broaden the scope of serum biomarker discovery, an automated method that combines STL and LEL for enriching trace proteins in serum was established. The STL/LEL method demonstrated an approximately 1.5-fold increase in the detection of serum proteins compared to the TOP14D method. Furthermore, comparing sera from SLE model mice and control mice via the STL/LEL enrichment method revealed over 1300 differentially expressed proteins. By collecting proteins with N-acetylglucosamine oligomer using STL and LEL, variations different from those obtained by conventional depletion methods could be observed, demonstrating that this method is effective for biomarker discovery.

**Table 2-1**

| Uniprot Accession Number | Protein Name | Gene Symbol | MW(kDa) |
|---|---|---|---|
| P21964 | Catechol O-methyltransferase | COMT | 30.037 |
| P08962 | CD63 antigen | CD63 | 25.636 |
| Q9NYQ8 | Protocadherin Fat 2 | FAT2 | 479.324 |
| Q96S97 | Myeloid-associated differentiation marker | MYADM | 35.275 |
| Q658P3 | Metalloreductase STEAP3 | STEAP3 | 54.601 |
| Q15075 | Early endosome antigen 1 | EEA1 | 162.468 |
| Q9Y6C2 | EMILIN-1 | EMILIN1 | 106.696 |
| Q93050 | V-type proton ATPase 116 kDa subunit a 1 | ATP6V0A1 | 96.415 |
| Q9Y613 | FH1/FH2 domain-containing protein 1 | FHOD1 | 126.554 |
| Q4KMQ2 | Anoctamin-6 | ANO6 | 106.167 |
| Q9Y2A7 | Neck-associated protein 1 | NCKAP1 | 128.79 |
| Q8WUT4 | Leucine-rich repeat neuronal protein 4 | LRRN4 | 78.844 |
| P60201 | Myelin proteolipid protein | PLP1 | 30.076 |
| Q99808 | Equilibrative nucleoside transporter 1 | SLC29A1 | 50.222 |
| P23560 | Brain-derived neurotrophic factor | BDNF | 27.818 |
| Q14764 | Major vault protein | MVP | 99.328 |
| P07948 | Tyrosine-protein kinase Lyn | LYN | 58.575 |
| P30711 | Glutathione S-transferase theta-1 | GSTT1 | 27.333 |
| Q9ULA0 | Aspartyl aminopeptidase | DNPEP | 53.412 |
| Q14644 | Ras GTPase-activating protein 3 | RASA3 | 95.7 |
| Q09327 | Beta-1,4-mannosyl-glycoprotein 4-beta-N-acetylglucosa | MGAT3 | 61.314 |
| P51888 | Prolargin | PRELP | 43.812 |
| P18433 | Receptor-type tyrosine-protein phosphatase alpha | PTPRA | 90.721 |
| Q9BXX0 | EMILIN-2 | EMILIN2 | 115.686 |
| P30622 | CAP-Gly domain-containing linker protein 1 | CLIP1 | 162.249 |
| Q15758 | Neutral amino acid transporter B(0) | SLC1A5 | 56.599 |
| Q6UWD8 | Transmembrane protein C16orf54 | C16orf54 | 24.359 |
| P55160 | Nck-associated protein 1-like | NCKAP1L | 128.156 |
| Q9HCM3 | UPF0606 protein KIAA1549 | KIAA1549 | 210.759 |
| P06870 | Kallikrein-1 | KLK1 | 28.89 |
| P61421 | V-type proton ATPase subunit d 1 | ATP6V0D1 | 40.329 |
| O96466 | Formin-like protein 1 | FMNL1 | 121.856 |
| 000501 | Claudin-5 | CLDN5 | 23.146 |
| Q9NP58 | ATP-binding cassette sub-family B member 6 | ABCB6 | 93.885 |
| P47972 | Neuronal pentraxin-2 | NPTX2 | 47.043 |
| Q9HD45 | Transmembrane 9 superfamily member 3 | TM9SF3 | 67.89 |
| P01266 | Thyroglobulin | TG | 304.794 |
| P62873 | Guanine nucleotide-binding protein G(I)/G(S)/G(T) subu | GNB1 | 37.377 |
| P15848 | Arylsulfatase B | ARSB | 59.688 |
| Q9Y2Q0 | Phospholipid-transporting ATPase IA | ATP8A1 | 131.369 |
| Q8WZ42 | Titin | TTN | 3816.111 |
| Q08431 | Lactadherin | MFGE8 | 43.105 |
| Q14980 | Nuclear mitotic apparatus protein 1 | NUMA1 | 238.263 |
| O66988 | Keratocan | KERA | 40.507 |
| P50148 | Guanine nucleotide-binding protein G(q) subunit alpha | GNAQ | 42.143 |
| Q8NOV5 | N-acetyllactosaminide beta-1,6-N-acetylglucosaminyl-tra | GCNT2 | 45.875 |
| P22748 | Carbonic anhydrase 4 | CA4 | 35.031 |
| Q9UKJ1 | Paired immunoglobulin-like type 2 receptor alpha | PILRA | 34.005 |
| P05023 | Sodium/potassium-transporting ATPase subunit alpha-1 | ATP1A1 | 112.897 |
| P78539 | Sushi repeat-containing protein SRPX | SRPX | 51573 |
| P19086 | Guanine nucleotide-binding protein G(z) subunit alpha | GNAZ | 40.924 |
| Q16555 | Dihydropyrimidinase-related protein 2 | DPYSL2 | 62.294 |
| P09543 | 2',3'-cyclic-nucleotide 3'-phosphodiesterase | CNP | 41.58 |
| Q86UF1 | Tetraspanin-33 | TSPAN33 | 31.539 |
| Q9UGN4 | CMRF35-like molecule 8 | CD300A | 33.202 |
| Q04656 | Copper-transporting ATPase 1 | ATP7A | 163.376 |
| P04839 | Cytochrome b-245 heavy chain | CYBB | 65.334 |
| B0l1T2 | Unconventional myosin-Ig | MYO1G | 116.444 |
| Q9NTJ4 | Alpha-mannosidase 2C1 | MAN2C1 | 115.836 |
| Q9UKV8 | Protein argonaute-2 | AGO2 | 97.209 |
| O00159 | Unconventional myosin-Ic | MYO1C | 121.682 |
| Q9NZC2 | Triggering receptor expressed on myeloid cells 2 | TREM2 | 25.445 |
| Q11201 | CMP-N-acetylneuraminate-beta-galactosarnide-alpha-2,3 | ST3GAL1 | 39.077 |
| Q5JWF2 | Guanine nucleotide-binding protein G(s) subunit alpha isc | GNAS | 111.025 |
| Q14344 | Guanine nucleotide-binding protein subunit alpha-13 | GNA13 | 44.05 |
| O75578 | Integrin alpha-10 | ITGA10 | 127.602 |
| P02686 | Myelin basic protein | MBP | 33.117 |
| P98194 | Calcium-transporting ATPase type 2C member 1 | ATP2C1 | 100.577 |
| P51575 | P2X purinoceptor 1 | P2RX1 | 44.983 |
| Q9Y6W5 | Actin-binding protein WASF2 | WASF2 | 54.284 |
| P18084 | Integrin beta-5 | ITGB5 | 88.056 |
| Q9Y639 | Neuroplastin | NPTN | 44.388 |
| Q6AZY7 | Scavenger receptor class A member 3 | SCARA3 | 65.137 |
| Q9NR16 | Scavenger receptor cysteine-rich type 1 protein M160 | CD163L1 | 159.242 |
| P13612 | Integrin alpha-4 | ITGA4 | 114.902 |
| P12956 | X-ray repair cross-complementing protein 6 | XRCC6 | 69.844 |
| P13498 | Cytochrome b-245 light chain | CYBA | 21.011 |
| Q9Y4D1 | Disheveled-associated activator of morphogenesis 1 | DAAM1 | 123.474 |
| Q12889 | Oviduct-specific glycoprotein | OVGP1 | 75.421 |
| P23634 | Plasma membrane calcium-transporting ATPase 4 | ATP2B4 | 137.923 |
| P61020 | Ras-related protein Rab-5B | RAB5B | 23.706 |
| Q8NAL2 | Type 2 phosphatidylinositol 4,5-bisphosphate 4-phosphat | PIP4P2 | 28.082 |
| Q96F07 | Cytoplasmic FMRl-interacting protein 2 | CYFIP2 | 145.402 |
| Q96PB7 | Noelin-3 | OLFM3 | 54.931 |
| Q96JP9 | Cadherin-related family member 1 | CDHR1 | 93.596 |
| Q9BY15 | Adhesion G protein-coupled receptor E3 | ADGRE3 | 72.621 |
| Q9Y6M5 | Zinc transporter 1 | SLC30A1 | 56.302 |
| Q96PL5 | Erythroid membrane-associated protein | ERMAP | 52.604 |
| Q9UQ53 | Alpha-1,3-mannosyl-glycoprotein 4-beta-N-acetylglucosa | MGAT4B | 63.198 |
| Q13336 | Urea transporter 1 | SLC14A1 | 42.528 |
| Q8NG06 | E3 ubiquitin-protein ligase TRIM58 | TRIM58 | 54.766 |
| O95782 | AP-2 complex subunit alpha-1 | AP2A1 | 107.547 |
| Q9P2B2 | Prostaglandin F2 receptor negative regulator | PTGFRN | 98.558 |
| P62879 | Guanine nucleotide-binding protein G(I)/G(S)/G(T) subun | GNB2 | 37.331 |
| Q99828 | Calcium and integrin-binding protein 1 | CIB1 | 21.702 |
| P09769 | Tyrosine-protein kinase Fgr | FGR | 59.478 |
| Q9NUM4 | Transmembrane protein 106B | TMEM106B | 31.13 |
| Q9BV40 | Vesicle-associated membrane protein 8 | VAMP8 | 11.434 |
| P13010 | X-ray repair cross-complementing protein 5 | XRCC5 | 82.705 |
| P14136 | Glial fibrillary acidic protein | GFAP | 49.88 |
| Q9H115 | Beta-soluble NSF attachment protein | NAPB | 33.558 |
| Q9HBH0 | Rho-related GTP-binding protein RhoF | RHOF | 23.625 |
| O15439 | ATP-binding cassette sub-family C member 4 | ABCC4 | 149.528 |
| P19474 | E3 ubiquitin-protein ligase TRIM21 | TRIM21 | 54.171 |
| Q14242 | P-selectin glycoprotein ligand 1 | SELPLG | 43.2 |
| Q9H444 | Charged multivesicular body protein 4b | CHMP4B | 24.951 |

**Table 2-2**

| Uniprot Accession Number | Protein -Name | Gene Symbol | MW(kDa) |
|---|---|---|---|
| P10915 | Hyaluronan and proteoglycan link protein 1 | HAPLN1 | 40.166 |
| Q969P0 | Immunoglobulin superfamily member 8 | IGSF8 | 65.035 |
| Q9BTU6 | Phosphatidylinositol 4-kinase type 2-alpha | PI4K2A | 54.021 |
| Q5T0T0 | E3 ubiquitin-protein ligase MARCHF8 | MARCHF8 | 32.965 |
| P49281 | Natural resistance-associated macrophage protein 2 | SLC11A2 | 62.267 |
| Q8N5I2 | Arrestin domain-containing protein 1 | ARRDC1 | 45.982 |
| Q13530 | Serine incorporator 3 | SERINC3 | 52.58 |
| Q6IAA8 | Ragulator complex protein LAMTOR1 | LAMTOR1 | 17.743 |
| O66636 | Tetraspanin-2 | TSPAN2 | 24.145 |
| Q9UQC9 | Calcium-activated chloride channel regulator 2 | CLCA2 | 103.94 |
| P51790 | H(+)/Cl(-) exchange transporter 3 | CLCN3 | 90.966 |
| P78504 | Protein jagged-1 | JAG1 | 133.801 |
| P62834 | Ras-related protein Rap-1A | RAP1A | 20.987 |
| Q9H336 | Cysteine-rich secretory protein LCCL domain-containing | CRISPLD1 | 56.89 |
| P13637 | Sodium/potassium-transporting ATPase subunit alpha-3 | ATP1A3 | 111.75 |
| O95841 | Angiopoietin-related protein 1 | ANGPTL1 | 56.72 |
| Q8NBZ7 | UDP-glucuronic acid decarboxylase 1 | UXS1 | 47.579 |
| Q96BS2 | Calcineurin B homologous protein 3 | TESC | 24.749 |
| O15123 | Angiopoietin-2 | ANGPT2 | 56.918 |
| Q14141 | Septin-6 | SEPTIN6 | 49.716 |
| P05165 | Propionyl-CoA carboxylase alpha chain, mitochondrial | PCCA | 80.06 |
| P08754 | Guanine nucleotide-binding protein G(i) subunit alpha-3 | GNAI3 | 40.534 |
| Q14392 | Transforming growth factor beta activator LRRC32 | LRRC32 | 71.98 |
| Q99747 | Gamma-soluble NSF attachment protein | NAPG | 34.746 |
| Q9Y210 | Short transient receptor potential channel 6 | TRPC6 | 106.328 |
| Q9H4B8 | Dipeptidase 3 | DPEP3 | 53.688 |
| Q9UM07 | Protein-arginine deiminase type-4 | PADI4 | 74.082 |
| Q96J02 | E3 ubiquitin-protein ligase Itchy homolog | ITCH | 102.801 |
| Q92696 | Geranylgeranyl transferase type-2 subunit alpha | RABGGTA | 65.075 |
| P05166 | Propionyl-CoA carboxylase beta chain, mitochondrial | PCCB | 58.215 |
| P40199 | Carcinoembryonic antigen-related cell adhesion molecule | CEACAM6 | 37.236 |
| A6NHR9 | Structural maintenance of chromosomes flexible hinge do | SMCHD1 | 226.377 |
| Q9UK41 | Vacuolar protein sorting-associated protein 28 homolog | VPS28 | 25.422 |
| O15162 | Phospholipid scramblase 1 | PLSCR1 | 35.049 |
| P30825 | High affinity cationic amino acid transporter 1 | SLC7A1 | 67.638 |
| Q9Y2G3 | Phospholipid-transporting ATPase IF | ATP11B | 134.189 |
| O94898 | Leucine-rich repeats and immunoglobulin-like domains p | LRIG2 | 118.966 |
| Q96QK1 | Vacuolar protein sorting-associated protein 35 | VPS35 | 91.707 |
| O00587 | Beta-1,3-N-acetylglucosaminyltransferase manic fringe | MFNG | 36.203 |
| O14798 | Tumor necrosis factor receptor superfamily member 10C | TNFRSF10C | 27.407 |
| Q6PJF5 | Inactive rhomboid protein 2 | RHBDF2 | 96.686 |
| P51159 | Ras-related protein Rab-27A | RAB27A | 24.865 |
| O75351 | Vacuolar protein sorting-associated protein 4B | VPS4B | 49.303 |
| O94804 | Serine/threonine-protein kinase 10 | STK10 | 112.136 |
| Q6P5S2 | Protein LEG1 homolog | LEG1 | 31.927 |
| O60229 | Kalirin | KALRN | 340.265 |
| O95858 | Tetraspanin-15 | TSPAN15 | 33.163 |
| Q14683 | Structural maintenance of chromosomes protein 1A | SMC1A | 143.236 |
| O75752 | UDP-GalNAc:beta-1,3-N-acetylgalactosaminyltransferas | B3GALNT1 | 39.513 |
| P14778 | Interleukin-1 receptor type 1 | IL1R1 | 65.404 |
| P21730 | C5a anaphylatoxin chemotactic receptor 1 | C5AR1 | 39.336 |
| P08631 | Tyrosine-protein kinase HCK | HCK | 59.601 |
| Q9NRX5 | Serine incorporator 1 | SERINC1 | 50.493 |
| Q7Z6M3 | Allergin-1 | MILR1 | 38.737 |
| P61764 | Syntaxin-binding protein 1 | STXBP1 | 67.569 |
| B9EJG8 | Transmembrane protein 150C | TMEM150C | 27.889 |
| Q9NV96 | Cell cycle control protein 50A | TMEM30A | 40.686 |
| Q6ZUX7 | LHFPL tetraspan subfamily member 2 protein | LHFPL2 | 24.483 |
| O75923 | Dysferlin | DYSF | 237.298 |
| P78330 | Phosphoserine phosphatase | PSPH | 25.009 |
| Q5T3F8 | CSC1-like protein 2 | TMEM63B | 94.958 |
| O75387 | Large neutral amino acids transporter small subunit 3 | SLC43A1 | 61.474 |
| Q8IU68 | Transmembrane channel-like protein 8 | TMC8 | 81.641 |
| Q92608 | Dedicator of cytokinesis protein 2 | DOCK2 | 211.95 |
| P07947 | Tyrosine-protein kinase Yes | YES1 | 60.8 |
| Q9HCM2 | Plexin-A4 | PLXNA4 | 212.459 |
| P21281 | V-type proton ATPase subunit B, brain isoform | ATP6V1B2 | 56.5 |
| Q13219 | Pappalysin-1 | PAPPA | 180.977 |
| Q96L08 | Sushi domain-containing protein 3 | SUSD3 | 27.12 |
| Q9P265 | Disco-interacting protein 2 homolog B | DIP2B | 171.492 |
| Q96AX2 | Ras-related protein Rab-37 | RAB37 | 24.815 |
| Q96RI0 | Proteinase-activated receptor 4 | F2RL3 | 41.132 |
| Q9NYQ6 | Cadherin EGF LAG seven-pass G-type receptor 1 | CELSR1 | 329.49 |
| Q9BX67 | Junctional adhesion molecule C | JAM3 | 35.021 |
| Q9Y6N6 | Laminin subunit gamma-3 | LAMC3 | 171.231 |
| Q9NQ34 | Transmembrane protein 9B | TMEM9B | 22.529 |
| Q9NRW7 | Vacuolar protein sorting-associated protein 45 | VPS45 | 65.075 |
| Q8NE00 | Transmembrane protein 104 | TMEM104 | 55.68 |
| P31641 | Sodium- and chloride-dependent taurine transporter | SLC6A6 | 69.832 |
| O75095 | Multiple epidermal growth factor-like domains protein 6 | MEGF6 | 161.19 |
| Q9BZZ2 | Sialoadhesin | SIGLEC1 | 182.624 |
| Q86X55 | Histone-arginine methyltransferase CARM1 | CARM1 | 65.854 |
| P04350 | Tubulin beta-4A chain | TUBB4A | 49.585 |
| Q969X1 | Protein lifeguard 3 | TMBIM1 | 34.607 |
| Q96FE5 | Leucine-rich repeat and immunoglobulin-like domain-con | LINGO1 | 69.876 |
| Q12846 | Syntaxin-4 | STX4 | 34.18 |
| P20020 | Plasma membrane calcium-transporting ATPase 1 | ATP2B1 | 134.687 |
| Q8N9N7 | Leucine-rich repeat-containing protein 57 | LRRC57 | 26.754 |
| Q13464 | Rho-associated protein kinase 1 | ROCK1 | 158.178 |
| Q8NDH3 | Probable aminopeptidase NPEPL1 | NPEPL1 | 55.859 |
| Q96QS1 | Tetraspanin-32 | TSPAN32 | 34.629 |
| P17213 | Bactericidal permeability-increasing protein | BPI | 53.901 |
| O15484 | Calpain-5 | CAPN5 | 73.171 |
| Q6GP\|1 | Chymotrypsinogen B2 | CTRB2 | 27.923 |
| Q15276 | Rab GTPase-binding effector protein 1 | RABEP1 | 99.291 |
| Q15722 | Leukotriene B4 receptor 1 | LTB4R | 37.556 |
| Q7Z403 | Transmembrane channel-like protein 6 | TMC6 | 90.047 |
| Q9H269 | Vacuolar protein sorting-associated protein 16 homolog | VPS16 | 94.691 |
| O96009 | Napsin-A | NAPSA | 45.386 |
| Q86T03 | Type 1 phosphatidylinositol 4,5-bisphosphate 4-phosphat | PIP4P1 | 29.468 |
| O69669 | Monocarboxylate transporter 2 | SLC16A7 | 52.199 |
| P21802 | Fibroblast growth factor receptor 2 | FGFR2 | 92.026 |
| P55060 | Exportin-2 | CSE1L | 110.419 |
| Q8TCT8 | Signal peptide peptidase-like 2A | SPPL2A | 58.144 |
| O94973 | AP-2 complex subunit alpha-2 | AP2A2 | 103.961 |
| Q8WUW1 | Protein BRICK1 | BRK1 | 8.74 |

**Table 2-3**

| Uniprot Accession Number | Protein Name | Gene Symbol | MW(kDa) |
|---|---|---|---|
| Q9H741 | SREBP regulating gene protein | SPRING1 | 23.596 |
| P17600 | Synapsin-1 | SYN1 | 74.112 |
| P23276 | Kell blood group glycoprotein | KEL | 82.827 |
| P16234 | Platelet-derived growth factor receptor alpha | PDGFRA | 122.669 |
| Q96RT1 | Erbin | ERBIN | 158.299 |
| Q15818 | Neuronal pentraxin-1 | NPTX1 | 47.122 |
| Q9BX97 | Plasmalemma vesicle-associated protein | PLVAP | 50.596 |
| Q504Y2 | Extracellular tyrosine-protein kinase PKDCC | PKDCC | 54.13 |
| P36888 | Receptor-type tyrosine-protein kinase FLT3 | FLT3 | 112.905 |
| Q14114 | Low-density lipoprotein receptor-related protein 8 | LRP8 | 105.636 |
| Q9NUQ9 | CYFIP-related Rac1 interactor B | CYRIB | 36.75 |
| Q9NP59 | Solute carrier family 40 member 1 | SLC40A1 | 62.54 |
| O48747 | AP-1 complex subunit gamma-1 | AP1G1 | 91.353 |
| O00421 | C-C chemokine receptor-like 2 | CCRL2 | 39.513 |
| P54709 | Sodium/potassium-transporting ATPase subunit beta -3 | ATP1B3 | 31.514 |
| Q5VXI9 | Lipase member N | LIPN | 45.534 |
| P08069 | Insulin-like growth factor 1 receptor | IGF1R | 154.798 |
| Q7Z304 | MAM domain-containing protein 2 | MAMDC2 | 71.556 |
| P10301 | Ras-related protein R-Ras | RRAS | 23.479 |
| P34910 | Protein EVI2B | EVI2B | 48.668 |
| Q9UQE7 | Structural maintenance of chromosomes protein 3 | SMC3 | 141.543 |
| O00453 | Leukocyte-specific transcript 1 protein | LST1 | 10.793 |
| Q05193 | Dynamin-1 | DNM1 | 97.409 |
| O00182 | Galectin-9 | LGALS9 | 39.518 |
| P08217 | Chymotrypsin-like elastase family member 2A | CELA2A | 28.886 |
| Q9NVA2 | Septin-11 | SEPTIN11 | 49.399 |
| O60784 | Target of Myb protein 1 | TOM1 | 53.817 |
| P43004 | Excitatory amino acid transporter 2 | SLC1A2 | 62.105 |
| Q9Y487 | V-type proton ATPase 116 kDa subunit a 2 | ATP6V0A2 | 98.085 |
| Q9HB40 | Retinoid-inducible serine carboxypeptidase | SCPEP1 | 50.829 |
| P00813 | Adenosine deaminase | ADA | 40.765 |
| Q96BY6 | Dedicator of cytokinesis protein 10 | DOCK10 | 249.535 |
| P53985 | Monocarboxylate transporter 1 | SLC16A1 | 53.943 |
| Q9NQ84 | G-protein coupled receptor family C group 5 member C | GPRC5C | 48.195 |
| Q9ULQ1 | Two pore channel protein 1 | TPCN1 | 94.148 |
| Q7Z3C6 | Autophagy-related protein 9A | ATG9A | 94.449 |
| P17812 | CTP synthase 1 | CTPS1 | 66.691 |
| Q9BXS5 | AP-1 complex subunit mu-1 | AP1M1 | 48.586 |
| Q8N131 | Porimin | TMEM123 | 21.532 |
| P53801 | Pituitary tumor-transforming gene 1 protein-interacting p | PTTG1IP | 20.323 |
| Q15846 | Clusterin-like protein 1 | CLUL1 | 54.216 |
| O43306 | Adenylate cyclase type 6 | ADCY6 | 130.619 |
| P09651 | Heterogeneous nuclear ribonucleoprotein A1 | HNRNPA1 | 38.744 |
| P51153 | Ras-related protein Rab-13 | RAB13 | 22.773 |
| Q15599 | Na(+)/H(+) exchange regulatory cofactor NHE-RF2 | SLC9A3R2 | 37.414 |
| P17643 | 5,6-dihydroxyindole-2-carboxylic acid oxidase | TYRP1 | 60.725 |
| Q5lJ48 | Protein crumbs homolog 2 | CRB2 | 134.266 |
| Q16666 | Gamma-interferon-inducible protein 16 | IFI16 | 88.258 |
| Q6UWE0 | E3 ubiquitin-protein ligase LRSAM1 | LRSAM1 | 83.593 |
| Q92752 | Tenascin-R | TNR | 149.564 |
| Q5M8T2 | Solute carrier family 35 member D3 | SLC35D3 | 44.184 |
| P16885 | 1-phosphatidylinositol 4,5-bisphosphate phosphodiestera | PLCG2 | 147.873 |
| P24071 | Immunoglobulin alpha Fc receptor | FCAR | 32.265 |
| P57075 | Ubiquitin-associated and SH3 domain-containing protein | UBASH3A | 74.124 |
| Q86SJ2 | Amphoterin-induced protein 2 | AMIGO2 | 57.935 |
| P62266 | 40S ribosomal protein S23 | RPS23 | 15.807 |
| P09936 | Ubiquitin carboxyl-terminal hydrolase isozyme L1 | UCHL1 | 24.825 |
| O15084 | Serine/threonine-protein phosphatase 6 regulatory ankyri | ANKRD28 | 112.967 |
| O75019 | Leukocyte immunoglobulin-like receptor subfamily A merr | LILRA1 | 53.273 |
| P35475 | Alpha-L-iduronidase | IDUA | 72.67 |
| O60488 | Long-chain-fatty-acid--CoA ligase 4 | ACSL4 | 79.189 |
| Q8N5C1 | Calcium homeostasis modulator protein 5 | CALHMS | 35.169 |
| Q99798 | Aconitate hydratase, mitochondrial | ACO2 | 85.426 |
| A8K7I4 | Calcium-activated chloride channel regulator 1 | CLCA1 | 100.226 |
| P33527 | Multidrug resistance-associated protein 1 | ABCC1 | 171.593 |
| P51798 | H(4-)/Cl(-) exchange transporter 7 | CLCN7 | 88.68 |
| Q7Z5G4 | Golgin subfamily A member 7 | GOLGA7 | 15.823 |
| P43250 | G protein-coupled receptor kinase 6 | GRK6 | 65.992 |
| Q9UQV4 | Lysosome-associated membrane glycoprotein 3 | LAMP3 | 44.345 |
| P06239 | Tyrosine-protein kinase Lck | LCK | 57.999 |
| Q96CS7 | Pleckstrin homology domain-containing family B member | PLEKHB2 | 24.737 |
| A6NC98 | Coiled-coil domain-containing protein 88B | CCDC88B | 164.81 |
| Q14642 | Inositol polyphosphate-5-phosphatase A | INPP5A | 47.818 |
| Q9UHW9 | Solute carrier family 12 member 6 | SLC12A6 | 127.62 |
| Q9H756 | Leucine-rich repeat-containing protein 19 | LRRC19 | 42.336 |
| Q9H2A7 | C-X-C motif chemokine 16 | CXCL16 | 27.579 |
| P28906 | Hematopoietic progenitor cell antigen CD34 | CD34 | 40.716 |
| Q9UQM7 | Calcium/calmodulin-dependent protein kinase type II sub | CAMK2A | 54.089 |
| Q8N9U0 | Tandem C2 domains nuclear protein | TC2N | 55.283 |
| P46776 | 60S ribosomal protein L27a | RPL27A | 16.561 |
| P05771 | Protein kinase C beta type | PRKCB | 76.869 |
| O15551 | Claudin-3 | CLDN3 | 23.318 |
| P33316 | Deoxyuridine 5'-triphosphate nucleotidohydrolase, mitoch | DUT | 26.564 |
| P50213 | Isocitrate dehydrogenase [NAD] subunit alpha, mitochonc | IDH3A | 39.59 |
| O60486 | Plexin-C1 | PLXNC1 | 175.744 |
| Q68D85 | Natural cytotoxicity triggering receptor 3 ligand 1 | NCR3LG1 | 50.826 |
| P35556 | Fibrillin-2 | FBN2 | 314.781 |
| Q96BM9 | ADP-ribosylation factor-like protein 8A | ARL8A | 21.417 |
| Q9H2K8 | Serine/threonine-protein kinase TAO3 | TAOK3 | 105.405 |
| Q15046 | Lysine--tRNA ligase | KARS1 | 68.048 |
| P57729 | Ras-related protein Rab-38 | RAB38 | 23.713 |
| P35241 | Radixin | RDX | 68.564 |
| P63167 | Dynein light chain 1, cytoplasmic | DYNLL1 | 10.365 |
| O60888 | Protein CutA | CUTA | 19.114 |
| P01817 | Immunoglobulin heavy variable 2-5 | IGHV2-5 | 13.231 |
| P08237 | ATP-dependent 6-phosphofructokinase, muscle type | PFKM | 86.184 |
| P42356 | Phosphatidylinositol 4-kinase alpha | PI4KA | 236.834 |
| O43820 | Hyaluronidase-3 | HYAL3 | 46.503 |
| Q7L7X3 | Serine/threonine-protein kinase TAO1 | TAOK1 | 116.072 |
| P20339 | Ras-related protein Rab-5A | RAB5A | 23.659 |
| Q99615 | DnaJ homolog subfamily C member 7 | DNAJC7 | 56.439 |
| O76036 | Natural cytotoxicity triggering receptor 1 | NCR1 | 34.48 |
| Q5JXA9 | Signal-regulatory protein beta-2 | SIRPB2 | 36.966 |
| Q8N697 | Solute carrier family 15 member 4 | SLC15A4 | 62.032 |
| P21757 | Macrophage scavenger receptor types I and II | MSR1 | 49.764 |
| Q32P28 | Prolyl 3-hydroxylase 1 | P3H1 | 83.395 |

**Table 2-4**

| Uniprot Accession Number | Protein Name | Gene Symbol | MW(kDa) |
|---|---|---|---|
| Q9NPH2 | Inositol-3-phosphate synthase 1 | ISYNA1 | 61.068 |
| O96721 | Synaptosomal-associated protein 29 | SNAP29 | 28.971 |
| Q01433 | AMP deaminase 2 | AMPD2 | 100.69 |
| Q92841 | Probable ATP-dependent RNA helicase DDX17 | DDX17 | 80.276 |
| P15104 | Glutamine synthetase | GLUL | 42.066 |
| Q14156 | Protein EFR3 homolog A | EFR3A | 92.925 |
| P08559 | Pyruvate dehydrogenase E1 component subunit alpha, so | PDHA1 | 43.295 |
| O95502 | Neuronal pentraxin receptor | NPTXR | 52.846 |
| Q8WUH2 | Transforming growth factor-beta receptor-associated pro | TGFBRAP1 | 97.158 |
| O95741 | Copine-6 | CPNE6 | 61.991 |
| Q8N5J2 | Ubiquitin carboxyl-terminal hydrolase MINDY-1 | MINDY1 | 51.778 |
| Q8NCB2 | CaM kinase-like vesicle-associated protein | CAMKV | 54.355 |
| Q14088 | Ras-related protein Rab-33A | RAB33A | 26.591 |
| Q53TN4 | Plasma membrane ascorbate-dependent reductase CYB | CYBRD1 | 31.639 |
| A1A5C7 | Solute carrier family 22 member 23 | SLC22A23 | 73.75 |
| P50993 | Sodium/potassium-transporting ATPase subunit alpha-2 | ATP1A2 | 112.265 |
| Q92556 | Engulfment and cell motility protein 1 | ELMO1 | 83.832 |
| Q5JSL3 | Dedicator of cytokinesis protein 11 | DOCK11 | 237.675 |
| Q15907 | Ras-related protein Rab-11B | RAB11B | 24.487 |
| Q8IY22 | C-Maf-inducing protein | CMIP | 86.331 |
| Q9NR97 | Toll-like receptor 8 | TLR8 | 119.829 |
| P22102 | Trifunctional purine biosynthetic protein adenosine-3 | GART | 107.766 |
| A6NM11 | Leucine-rich repeat-containing protein 37A2 | LRRC37A2 | 188.395 |
| P26368 | Splicing factor U2AF 65 kDa subunit | U2AF2 | 53.501 |
| Q5T4S7 | E3 ubiquitin-protein ligase UBR4 | UBR4 | 573.853 |
| Q96PU5 | E3 ubiquitin-protein ligase NEDD4-like | NEDD4L | 111.933 |
| P40818 | Ubiquitin carboxyl-terminal hydrolase 8 | USP8 | 127.523 |
| O75643 | U5 small nuclear ribonucleoprotein 200 kDa helicase | SNRNP200 | 244.511 |
| P27694 | Replication protein A 70 kDa DNA-binding subunit | RPA1 | 68.139 |
| Q9NPA2 | Matrix metalloproteinase-25 | MMP25 | 62.556 |
| Q86YD3 | Transmembrane protein 25 | TMEM25 | 39.282 |
| Q9UMR5 | Lysosomal thioesterase PPT2 | PPT2 | 34.224 |
| Q9UNQ0 | Broad substrate specificity ATP-binding cassette transpo | ABCG2 | 72.315 |
| Q10589 | Bone marrow stromal antigen 2 | BST2 | 19.77 |
| Q9Y3E7 | Charged multivesicular body protein 3 | CHMP3 | 25.071 |
| Q15303 | Receptor tyrosine-protein kinase erbB-4 | ERBB4 | 146.812 |
| Q9H3S7 | Tyrosine-protein phosphatase non-receptor type 23 | PTPN23 | 178.974 |
| Q9UNK0 | Syntaxin-8 | STX8 | 26.905 |
| Q8NHG7 | Small VCP/p97-interacting protein | SVIP | 8.44 |
| P00387 | NADH-cytochrome b5 reductase 3 | CYB5R3 | 34.236 |
| Q8TDQ1 | CMRF35-like molecule 1 | CD300LF | 32.336 |
| P54619 | 5'-AMP-activated protein kinase subunit gamma-1 | PRKAG1 | 37.579 |
| Q92973 | Transportin-1 | TNPO1 | 102.357 |
| Q8IXJ6 | NAD-dependent protein deacetylase sirtuin-2 | SIRT2 | 43.182 |
| P20336 | Ras-related protein Rab-3A | RAB3A | 24.984 |
| Q8NB49 | Phospholipid-transporting ATPase IG | ATP11C | 129.48 |
| Q8IVB4 | Sodium/hydrogen exchanger 9 | SLC9A9 | 72.565 |
| Q9UN37 | Vacuolar protein sorting-associated protein 4A | VPS4A | 48.899 |
| P50851 | Lipopolysaccharide-responsive and beige-like anchor pro | LRBA | 319.112 |
| Q8NE01 | Metal transporter CNNM3 | CNNM3 | 76.121 |
| P57057 | Glucose-6-phosphate exchanger SLC37A1 | SLC37A1 | 57.651 |
| P53621 | Coatomer subunit alpha | COPA | 138.349 |
| Q9HCJ1 | Progressive ankylosis protein homolog | ANKH | 54.243 |
| Q9P2J5 | Leucine--tRNA ligase, cytoplasmic | LARS1 | 134.468 |
| Q16352 | Alpha- internexin | INA | 55.392 |
| Q9NRQ2 | Phospholipid scramblase 4 | PLSCR4 | 37.006 |
| A5D8V6 | Vacuolar protein sorting-associated protein 37C | VPS37C | 38.66 |
| P63096 | Guanine nucleotide-binding protein G(i) subunit alpha-1 | GNAI1 | 40.358 |
| Q9H479 | Fructosamine-3-kinase | FN3K | 35.172 |
| Q99719 | Septin-5 | SEPTIN5 | 42.777 |
| P38570 | Integrin alpha-E | ITGAE | 130.158 |
| Q9H9H4 | Vacuolar protein sorting-associated protein 37B | VPS37B | 31.308 |
| P50570 | Dynamin-2 | DNM2 | 98.067 |
| Q14435 | Polypeptide N-acetylgalactosaminyltransferase 3 | GALNT3 | 72.611 |
| P22694 | cAMP-dependent protein kinase catalytic subunit beta | PRKACB | 40.622 |
| P07196 | Neurofilament light polypeptide | NEFL | 61.519 |
| P11137 | Microtubule-associated protein 2 | MAP2 | 199.529 |
| Q9NRA2 | Sialin | SLC17A5 | 54.639 |
| Q8NCG7 | Diacylglycerol lipase-beta | DAGLB | 73.733 |
| O60331 | Phosphatidylinositol 4-phosphate 5-kinase type-1 gamma | PIP5K1C | 73.26 |
| Q14773 | Intercellular adhesion molecule 4 | ICAM4 | 29.265 |
| Q13641 | Trophoblast glycoprotein | TPBG | 46.031 |
| P40763 | Signal transducer and activator of transcription 3 | STAT3 | 88.068 |
| P78536 | Disintegrin and metalloproteinase domain-containing prot | ADAM17 | 93.022 |
| P07814 | Bifunctional glutamate/proline--tRNA ligase | EPRS1 | 170.594 |
| Q9UI12 | V-type proton ATPase subunit H | ATP6V1H | 55.885 |
| P62333 | 26S proteasome regulatory subunit 10B | PSMC6 | 44.172 |
| P19526 | Galactoside alpha-(1,2)-fucosyltransferase 1 | FUT1 | 41.251 |
| P36542 | ATP synthase subunit gamma, mitochondrial | ATP5F1C | 32.995 |
| P09471 | Guanine nucleotide-binding protein G(o) subunit alpha | GNAO1 | 40.048 |
| O00483 | Cytochrome c oxidase subunit NDUFA4 | NDUFA4 | 9.368 |
| Q9UIA9 | Exportin-7 | XPO7 | 123.908 |
| P61073 | C-X-C chemokine receptor type 4 | CXCR4 | 39.745 |
| Q14108 | Lysosome membrane protein 2 | SCARB2 | 54.294 |
| P51679 | C-C chemokine receptor type 4 | CCR4 | 41.404 |
| Q81ZY2 | Phospholipid-transporting ATPase ABCA7 | ABCA7 | 234.353 |
| Q7Z2W4 | Zinc finger CCCH-type antiviral protein 1 | ZC3HAV1 | 101.433 |
| P51674 | Neuronal membrane glycoprotein M6-a | GPM6A | 31.211 |
| P61221 | ATP-binding cassette sub-family E member 1 | ABCE1 | 67.315 |
| Q9H7D0 | Dedicator of cytokinesis protein 5 | DOCK5 | 215.312 |
| P13747 | HLA class I histocompatibility antigen, alpha chain E | HLA-E | 40.058 |
| O43426 | Synaptojanin-1 | SYNJ1 | 173.105 |
| P50591 | Tumor necrosis factor ligand superfamily member 10 | TNFSF10 | 32.511 |
| Q86Y82 | Syntaxin-12 | STX12 | 31.64 |
| P49591 | Serine--tRNA ligase, cytoplasmic | SARS1 | 58.778 |
| Q99598 | Translin-associated protein X | TSNAX | 33.112 |
| Q68CP4 | Heparan-alpha-glucosaminide N-acetyltransferase | HGSNAT | 73.294 |
| Q96LA6 | Fc receptor-like protein 1 | FCRL1 | 46.937 |
| Q9H3U5 | Major facilitator superfamily domain-containing protein 1 | MFSD1 | 51.209 |
| Q9NXU5 | ADP-ribosylation factor-like protein 15 | ARL15 | 22.875 |
| Q9BX74 | TM2 domain-containing protein 1 | TM2D1 | 22.326 |
| Q15393 | Splicing factor 3B subunit 3 | SF3B3 | 135.578 |
| Q8N3P4 | Vacuolar protein sorting-associated protein 8 homolog | VPS8 | 161.754 |
| O15431 | High affinity copper uptake protein 1 | SLC31A1 | 21.09 |
| Q9NX76 | CKLF-like MARVEL transmembrane domain-containing pr | CMTM6 | 20.419 |
| P49961 | Ectonucleoside triphosphate diphosphohydrolase 1 | ENTPD1 | 57.966 |

**Table 2-5**

| Uniprot Accession Number | Protein Name | Gene Symbol | MW(kDa) |
|---|---|---|---|
| O76696 | Protein XRP2 | RP2 | 39.641 |
| P0C7P3 | Protein SLFN14 | SLFN14 | 103.906 |
| O95772 | STARD3 N-terminal-like protein | STARD3NL | 26.657 |
| P31930 | Cytochrome b-c1 complex subunit 1, mitochondrial | UQCRC1 | 52.649 |
| Q9Y4F9 | Rho family-interacting cell polarization regulator 2 | RIPOR2 | 118.52 |
| P25024 | C-X-C chemokine receptor type 1 | CXCR1 | 39.789 |
| Q13444 | Disintegrin and metalloproteinase domain-containing pro | ADAM15 | 92.961 |
| O66487 | 26S proteasome non-ATPase regulatory subunit 14 | PSMD14 | 34.577 |
| O48668 | Prostate stem cell antigen | PSCA | 11.957 |
| P43007 | Neutral amino acid transporter A | SLC1A4 | 55.721 |
| Q99895 | Chymotrypsin-C | CTRC | 29.483 |
| P01732 | T-cell surface glycoprotein CD8 alpha chain | CD8A | 25.73 |
| Q3ZCM7 | Tubulin beta-8 chain | TUBB8 | 49.774 |
| P25205 | DNA replication licensing factor MCM3 | MCM3 | 90.981 |
| P17612 | cAMP-dependent protein kinase catalytic subunit alpha | PRKACA | 40.59 |
| Q9H3U1 | Protein unc-45 homolog A | UNC45A | 103.077 |
| Q99873 | Protein arginine N-methyltransferase 1 | PRMT1 | 42.462 |
| O94907 | Dickkopf-related protein 1 | DKK1 | 28.669 |
| P32121 | Beta-arrestin-2 | ARRB2 | 46.107 |
| Q9BYG0 | Lactosylceramide 1,3-N-acetyl-beta-D-glucosaminyltrans | B3GNT5 | 44.052 |
| Q68CJ9 | Cyclic AMP-responsive element-binding protein 3-like pr | CREB3L3 | 49.077 |
| A0A0G2JMI3 | Immunoglobulin heavy variable 1-69-2 | IGHV1-69-2 | 12.87 |
| P21731 | Thromboxane A2 receptor | TBXA2R | 37.43 |
| P21579 | Synaptotagmin-1 | SYT1 | 47.573 |
| Q9P289 | Serine/threonine-protein kinase 26 | STK26 | 46.528 |
| P38919 | Eukaryotic initiation factor 4A-III | EIF4A3 | 46.871 |
| P40616 | ADP-ribosylation factor-like protein 1 | ARL1 | 20.413 |
| P16949 | Stathmin | STMN1 | 17.305 |
| 014744 | Protein arginine N-methyltransferase 5 | PRMT5 | 72.684 |
| P36873 | Serine/threonine-protein phosphatase PP1-gamma catal | PPP1CC | 36.982 |
| Q13443 | Disintegrin and metalloproteinase domain-containing pro | ADAM9 | 90.559 |
| Q9Y666 | Solute carrier family 12 member 7 | SLC12A7 | 119.107 |
| Q92832 | Protein kinase C-binding protein NELL1 | NELL1 | 89.638 |
| Q86WC4 | Osteopetrosis-associated transmembrane protein 1 | OSTM1 | 37.256 |
| P50897 | Palmitoyl-protein thioesterase 1 | PPT1 | 34.193 |
| 014657 | Torsin-1B | TOR1B | 37.979 |
| Q9NZH0 | G-protein coupled receptor family C group 5 member B | GPRC5B | 44.794 |
| Q9NQ75 | Cas scaffolding protein family member 4 | CASS4 | 87.144 |
| P31997 | Carcinoembryonic antigen-related cell adhesion molecule | CEACAMS | 38.155 |
| Q08209 | Protein phosphatase 3 catalytic subunit alpha | PPP3CA | 58.688 |
| P35221 | Catenin alpha-1 | CTNNA1 | 100.073 |
| Q8WZ73 | E3 ubiquitin-protein ligase rififylin | RFFL | 40.512 |
| Q5JTH9 | RRP12-like protein | RRP12 | 143.703 |
| O75746 | Electrogenic aspartate/glutamate antiporter SLC25A12, | SLC25A12 | 74.762 |
| Q9HCE7 | E3 ubiquitin-protein ligase SMURF1 | SMURF1 | 86.116 |
| 075348 | V-type proton ATPase subunit G 1 | ATP6V1G1 | 13.756 |
| Q96D96 | Voltage-gated hydrogen channel 1 | HVCN1 | 31.633 |
| P25025 | C-X-C chemokine receptor type 2 | CXCR2 | 40.758 |
| Q14CN2 | Calcium-activated chloride channel regulator 4 | CLCA4 | 101.285 |
| Q86X52 | Chondroitin sulfate synthase 1 | CHSY1 | 91.787 |
| Q9Y286 | Sialic acid-binding Ig-like lectin 7 | SIGLEC7 | 51.144 |
| P23458 | Tyrosine-protein kinase JAK1 | JAK1 | 133.279 |
| Q92522 | Histone H1-10 | H1-10 | 22.484 |
| P21439 | Phosphatidylcholine translocator ABCB4 | ABCB4 | 141.524 |
| O43301 | Heat shock 70 kDa protein 12A | HSPA12A | 74.978 |
| Q9BZQ8 | Protein Niban 1 | NIBAN1 | 103.136 |
| P41091 | Eukaryotic translation initiation factor 2 subunit 3 | EIF2S3 | 51.109 |
| Q8IUX7 | Adipocyte enhancer-binding protein 1 | AEBP1 | 130.931 |
| P41252 | Isoleucine--tRNA ligase, cytoplasmic | IARS1 | 144.498 |
| P35080 | Profilin-2 | PFN2 | 15.046 |
| Q08334 | Interleukin-10 receptor subunit beta | IL10RB | 36.995 |
| Q9Y6W3 | Calpain-7 | CAPN7 | 92.654 |
| P14555 | Phospholipase A2, membrane associated | PLA2G2A | 16.084 |
| Q9Y394 | Dehydrogenase/reductase SDR family member 7 | DHRS7 | 38.295 |
| Q9H1A3 | Protein-L-histidine N-pros-methyltransferase | METTL9 | 36.536 |
| Q9H7P6 | Multivesicular body subunit 12B | MVB12B | 35.621 |
| Q7RTW8 | Otoancorin | OTOA | 128.534 |
| O75390 | Citrate synthase, mitochondrial | CS | 51.714 |
| Q8N386 | Leucine-rich repeat-containing protein 25 | LRRC25 | 33.179 |
| Q15208 | Serine/threonine-protein kinase 38 | STK38 | 54.19 |
| P63098 | Calcineurin subunit B type 1 | PPP3R1 | 19.299 |
| Q96F46 | Interleukin-17 receptor A | IL17RA | 96.122 |
| 043809 | Cleavage and polyadenylation specificity factor subunit 5 | NUDT21 | 26.228 |
| Q961G2 | F-box/LRR-repeat protein 20 | FBXL20 | 48.424 |
| P32241 | Vasoactive intestinal polypeptide receptor 1 | VIPR1 | 51.546 |
| P19525 | Interferon-induced, double-stranded RNA-activated prote | EIF2AK2 | 62.095 |
| P41226 | Ubiquitin-like modifier-activating enzyme 7 | UBA7 | 111.695 |
| Q8TAA9 | Vang-like protein 1 | VANGL1 | 59.975 |
| Q9NPA0 | ER membrane protein complex subunit 7 | EMC7 | 26.471 |
| Q9ULF5 | Zinc transporter ZIP10 | SLC39A10 | 94.13 |
| Q8TDZ2 | [F-actin]-monooxygenase MICAL1 | MICAL1 | 117.875 |
| Q96ID5 | Immunoglobulin superfamily member 21 | IGSF21 | 51.832 |
| Q5JU69 | Torsin-2A | TOR2A | 35-711 |
| Q9H3G5 | Probable serine carboxypeptidase CPVL | CPVL | 54.163 |
| P08243 | Asparagine synthetase [glutamine-hydrolyzing] | ASNS | 64.371 |
| Q8N4C8 | Misshapen-like kinase 1 | MINK1 | 149.824 |
| O00154 | Cytosolic acyl coenzyme A thioester hydrolase | ACOT7 | 41.792 |
| Q9NRR1 | Cytokine-like protein 1 | CYTL1 | 15.577 |
| O75354 | Ectonucleoside triphosphate diphosphohydrolase 6 | ENTPD6 | 53.249 |
| Q8TB96 | T-cell immunomodulatory protein | ITFG1 | 68.108 |
| P51570 | Galactokinase | GALK1 | 42.273 |
| O60674 | Tyrosine-protein kinase JAK2 | JAK2 | 130.674 |
| O15127 | Secretory carrier-associated membrane protein 2 | SCAMP2 | 36.647 |
| Q9Y2J2 | Band 4.1-like protein 3 | EPB41L3 | 120.678 |
| Q9BYB0 | SH3 and multiple ankyrin repeat domains protein 3 | SHANK3 | 184.669 |
| O15440 | ATP-binding cassette sub-family C member 5 | ABCC5 | 160.663 |
| Q9ULH0 | Kinase D-interacting substrate of 220 kDa | KIDINS220 | 196.545 |
| P07306 | Asialoglycoprotein receptor 1 | ASGR1 | 33.185 |
| Q96DZ9 | CKLF-like MARVEL transmembrane domain-containing pr | rCMTM5 | 24.654 |
| Q9NYH9 | U3 small nucleolar RNA-associated protein 6 homolog | UTP6 | 70.195 |
| Q96K49 | Transmembrane protein 87B | TMEM87B | 63.536 |
| Q9UQB8 | Brain-specific angiogenesis inhibitor 1-associated protein | BAIAP2 | 60.868 |
| P46777 | 60S ribosomal protein L5 | RPL5 | 34.361 |
| P04181 | Ornithine aminotransferase, mitochondrial | OAT | 48.533 |
| P61266 | Syntaxin-1B | STX1B | 33-245 |
| Q96CW1 | AP-2 complex subunit mu | AP2M1 | 49.657 |

**Table 2-6**

| Uniprot Accession Number | Protein Name | Gene Symbol | MW(kDa) |
|---|---|---|---|
| Q92900 | Regulator of nonsense transcripts 1 | UPF1 | 124.346 |
| Q7Z4F1 | Low-density lipoprotein receptor-related protein 10 | LRP10 | 76.194 |
| P62753 | 40S ribosomal protein S6 | RPS6 | 28.679 |
| 060716 | Catenin delta-1 | CTNND1 | 108.171 |
| 015118 | NPC intracellular cholesterol transporter 1 | NPC1 | 142.169 |
| P51572 | B-cell receptor-associated protein 31 | BCAP31 | 27.99 |
| P61812 | Transforming growth factor beta-2 proprotein | TGFB2 | 47.747 |
| Q01523 | Defensin alpha 5 | DEFA5 | 10.07 |
| Q8N0Z9 | V-set and immunoglobulin domain-containing protein 10 | VSIG10 | 59.218 |
| Q9Y5B9 | FACT complex subunit SPT16 | SUPT16H | 119.916 |
| P24752 | Acetyl-CoA acetyltransferase, mitochondrial | ACAT1 | 45.197 |
| Q8NE71 | ATP-binding cassette sub-family F member 1 | ABCF1 | 95.927 |
| Q4GOF5 | Vacuolar protein sorting-associated protein 26B | VPS26B | 39.155 |
| 095831 | Apoptosis-inducing factor 1, mitochondrial | AIFM1 | 66.9 |
| P50336 | Protoporphyrinogen oxidase | PPOX | 50.764 |
| Q8WV92 | MIT domain-containing protein 1 | MITD1 | 29.314 |
| P02462 | Collagen alpha-1(IV) chain | COL4A1 | 160.612 |
| P47871 | Glucagon receptor | GCGR | 54.009 |
| P78423 | Fractalkine | CX3CL1 | 42.202 |
| 000522 | Krev interaction trapped protein 1 | KRIT1 | 84.348 |
| 060884 | DnaJ homolog subfamily A member 2 | DNAJA2 | 45.748 |
| P18754 | Regulator of chromosome condensation | RCC1 | 44.969 |
| P62316 | Small nuclear ribonucleoprotein Sm D2 | SNRPD2 | 13.525 |
| Q08945 | FACT complex subunit SSRP1 | SSRP1 | 81.075 |
| P21283 | V-type proton ATPase subunit C 1 | ATP6V1C1 | 43.943 |
| Q9H1C7 | Cysteine-rich and transmembrane domain-containing pro | CYSTM1 | 10.63 |
| Q9Y3C5 | RING finger protein 11 | RNF11 | 17.443 |
| P0DP07 | Immunoglobulin heavy variable 4-31 | IGHV4-31 | 13.093 |
| P17252 | Protein kinase C alpha type | PRKCA | 76.753 |
| P10155 | RNA-binding protein R060 | RO60 | 60.67 |
| Q96RD7 | Pannexin-1 | PANX1 | 48.051 |
| Q9NUG4 | Cerebral cavernous malformations 2 protein-like | CCM2L | 62.179 |
| Q9Y264 | Angiopoietin-4 | ANGPT4 | 56.849 |
| P42858 | Huntingtin | HTT | 347-607 |
| P05388 | 60S acidic ribosomal protein P0 | RPLP0 | 34.272 |
| P43003 | Excitatory amino acid transporter 1 | SLC1A3 | 59.571 |
| Q6ZNJ1 | Neurobeachin-like protein 2 | NBEAL2 | 302.521 |
| Q99653 | Calcineurin B homologous protein 1 | CHP1 | 22.456 |
| 075915 | PRA1 family protein 3 | ARL6IPS | 21615 |
| 043491 | Band 4.1-like protein 2 | EPB41L2 | 112.588 |
| Q6P589 | Tumor necrosis factor alpha-induced protein 8-like protei | TNFAIP8L2 | 20.556 |
| Q8NC42 | E3 ubiquitin-protein ligase RNF149 | RNF149 | 43.163 |
| P25942 | Tumor necrosis factor receptor superfamily member 5 | CD40 | 30.619 |
| Q14554 | Protein disulfide-isomerase A5 | PDIA5 | 59.595 |
| Q9BRN9 | TM2 domain-containing protein 3 | TM2D3 | 27.118 |
| Q14699 | Raftlin | RFTN1 | 63.145 |
| P49458 | Signal recognition particle 9 kDa protein | SRP9 | 10.111 |
| P53611 | Geranylgeranyl transferase type-2 subunit beta | RABGGTB | 36.925 |
| P53680 | AP-2 complex subunit sigma | AP2S1 | 17.017 |
| P42229 | Signal transducer and activator of transcription 5A | STAT5A | 90.652 |
| P08708 | 40S ribosomal protein S17 | RPS17 | 15.55 |
| 075533 | Splicing factor 3B subunit 1 | SF3B1 | 145-831 |
| P30408 | Transmembrane 4 L6 family member 1 | TM4SF1 | 21.632 |
| Q9UNM6 | 26S proteasome non-ATPase regulatory subunit 13 | PSMD13 | 42.944 |
| P35244 | Replication protein A 14 kDa subunit | RPA3 | 13-569 |
| P14415 | Sodium/potassium-transporting ATPase subunit beta-2 | ATP1B2 | 33-368 |
| P07197 | Neurofilament medium polypeptide | NEFM | 102.474 |
| Q5T5CO | Syntaxin-binding protein 5 | STXBP5 | 127.575 |
| P36543 | V-type proton ATPase subunit E 1 | ATP6V1E1 | 26.145 |
| P98196 | Phospholipid-transporting ATPase IH | ATP11A | 129.758 |
| Q9BRG1 | Vacuolar protein-sorting-associated protein 25 | VPS25 | 20.748 |
| Q14289 | Protein-tyrosine kinase 2-beta | PTK2B | 115.875 |
| Q16891 | MICOS complex subunit MIC60 | IMMT | 83.679 |
| Q01844 | RNA-binding protein EWS | EWSR1 | 68.481 |
| P36957 | Dihydrolipoyllysine-residue succinyltransferase component | DLST | 48.758 |
| Q03692 | Collagen alpha-1(X) chain | COL10A1 | 66.158 |
| P01730 | T-cell surface glycoprotein CD4 | CD4 | 51.112 |
| Q9NYB9 | AbI interactor 2 | ABI2 | 55.662 |
| Q8NCR9 | Clarin-3 | CLRN3 | 25.321 |
| Q3ZCQ8 | Mitochondrial import inner membrane translocase subunit | TIMM50 | 39.647 |
| Q15036 | Sorting nexin-17 | SNX17 | 52.9 |
| A6NFX1 | Sphingosine-1-phosphate transporter MFSD2B | MFSD2B | 53.743 |
| Q9ULP9 | TBC1 domain family member 24 | TBC1D24 | 62.922 |
| P17844 | Probable ATP-dependent RNA helicase DDX5 | DDX5 | 69-148 |
| Q15049 | Membrane protein MLC1 | MLC1 | 41.141 |
| Q9H6Y7 | E3 ubiquitin-protein ligase RNF167 | RNF167 | 38.296 |
| P22001 | Potassium voltage-gated channel subfamily A member 3 | KCNA3 | 63-843 |
| Q13263 | Transcription intermediary factor 1-beta | TRIM28 | 88.553 |
| 060568 | Multifunctional procollagen lysine hydroxylase and glycosy | PLOD3 | 84.787 |
| Q8NFP4 | MAM domain-containing glycosylphosphatidylinositol anc | MDGA1 | 105.792 |
| A6NFN3 | RNA binding protein fox-1 homolog 3 | RBFOX3 | 33-871 |
| Q8WWB7 | Glycosylated lysosomal membrane protein | GLMP | 43.863 |
| Q9UPR5 | Sodium/calcium exchanger 2 | SLC8A2 | 100.369 |
| Q8WW52 | Protein FAM151A | FAM151A | 64.025 |
| Q9H7B2 | Ribosome production factor 2 homolog | RPF2 | 35.585 |
| P49795 | Regulator of G-protein signaling 19 | RGS19 | 24.637 |
| Q96QH2 | PML-RARA-regulated adapter molecule 1 | PRAM1 | 73.969 |
| Q6UWY5 | Olfactomedin-like protein 1 | OLFML1 | 45.95 |
| Q9UHA4 | Ragulator complex protein LAMTOR3 | LAMTOR3 | 13.623 |
| P43353 | Aldehyde dehydrogenase family 3 member B1 | ALDH3B1 | 51.839 |
| P83731 | 60S ribosomal protein L24 | RPL24 | 17.778 |
| Q8IWB7 | WD repeat and FYVE domain-containing protein 1 | WDFY1 | 46.323 |
| Q9H7Y0 | Divergent protein kinase domain 2B | DIPK2B | 48.554 |
| P01111 | GTPase NRas | NRAS | 21.228 |
| Q86Y38 | Xylosyltransferase 1 | XYLT1 | 107.568 |
| Q9P2R7 | Succinate--CoA ligase [ADP-forming] subunit beta, mitoc | SUCLA2 | 50.316 |
| P05198 | Eukaryotic translation initiation factor 2 subunit 1 | EIF2S1 | 36-114 |
| P05997 | Collagen alpha-2(V) chain | COL5A2 | 144.912 |
| Q6P1X5 | Transcription initiation factor TFIID subunit 2 | TAF2 | 136.974 |
| Q8TEM1 | Nuclear pore membrane glycoprotein 210 | NUP210 | 205.115 |
| Q8NAC3 | Interleukin-17 receptor C | IL17RC | 86.24 |
| Q99714 | 3-hydroxyacyl-CoA dehydrogenase type-2 | HSD17B10 | 26.923 |
| P61326 | Protein mago nashi homolog | MAGOH | 17.163 |
| A2RU67 | Protein FAM234B | FAM234B | 67.041 |
| P24390 | ER lumen protein-retaining receptor 1 | KDELR1 | 24.543 |
| P26951 | Interleukin-3 receptor subunit alpha | IL3RA | 43.331 |

**Table 2-7**

| Uniprot Accession Number | Protein Name | Gene Symbol | MW(kDa) |
|---|---|---|---|
| Q15771 | Ras-related protein Rab-30 | RAB30 | 23.057 |
| Q8N392 | Rho GTPase-activating protein 18 | ARHGAP18 | 74.977 |
| 043567 | E3 ubiquitin-protein ligase RNF13 | RNF13 | 42.812 |
| 014576 | Cytoplasmic dynein 1 intermediate chain 1 | DYNC1I1 | 72.956 |
| Q9C0K1 | Metal cation symporter ZIP8 | SLC39A8 | 49.631 |
| P62745 | Rho-related GTP-binding protein RhoB | RHOB | 22.121 |
| Q9NPF0 | CD320 antigen | CD320 | 28.99 |
| Q709C8 | Intermembrane lipid transfer protein VPS13C | VPS13C | 422.398 |
| Q13303 | Voltage-gated potassium channel subunit beta-2 | KCNAB2 | 41 |
| Q9HD40 | O-phosphoseryl-tRNA(Sec) selenium transferase | SEPSECS | 55.726 |
| Q9UQN3 | Charged multivesicular body protein 2b | CHMP2B | 23.905 |
| P28907 | ADP-ribosyl cyclase/cyclic ADP-ribose hydrolase 1 | CD38 | 34.328 |
| P46778 | 60S ribosomal protein L21 | RPL21 | 18.564 |
| Q13636 | Ras-related protein Rab-31 | RAB31 | 21.57 |
| Q14761 | Protein tyrosine phosphatase receptor type C-associated | PTPRCAP | 21.194 |
| Q96NT5 | Proton-coupled folate transporter | SLC46A1 | 49.772 |
| Q9UHD8 | Septin-9 | SEPTING | 65.402 |
| P36776 | Lon protease homolog, mitochondrial | LONP1 | 106.492 |
| Q13098 | C0P9 signalosome complex subunit 1 | GPS1 | 55.537 |
| P49643 | DNA primase large subunit | PRIM2 | 58.807 |
| 043813 | Glutathione S-transferase LANCL1 | LANCL1 | 45.284 |
| P30203 | T-cell differentiation antigen CD6 | CD6 | 71.804 |
| P51828 | Adenylate cyclase type 7 | ADCY7 | 120.309 |
| Q93008 | Probable ubiquitin carboxyl-terminal hydrolase FAF-X | USP9X | 290.467 |
| P40238 | Thrombopoietin receptor | MPL | 71.244 |
| Q96AJ9 | Vesicle transport through interaction with t-SNAREs hom | VTI1A | 25.218 |
| Q68DH5 | G-protein coupled receptor-associated protein LMBRD2 | LMBRD2 | 81.174 |
| Q8NBF2 | NHL repeat-containing protein 2 | NHLRC2 | 79.446 |
| P55769 | NHP2-like protein 1 | SNU13 | 14.173 |
| Q7Z6Z7 | E3 ubiquitin-protein ligase HUWE1 | HUWE1 | 481.899 |
| Q9UEY8 | Gamma-adducin | ADD3 | 79.157 |
| Q14558 | Phosphoribosyl pyrophosphate synthase-associated prot | PRPSAP1 | 39.394 |
| Q6UWB1 | Interleukin-27 receptor subunit alpha | IL27RA | 69.475 |
| Q6UWY2 | **Serine** protease 57 | PRSS57 | 30.336 |
| Q9P253 | Vacuolar protein sorting-associated protein 18 homolog | VPS18 | 110.186 |
| Q86VW1 | Solute carrier family 22 member 16 | SLC22A16 | 64.613 |
| 015347 | High mobility group protein B3 | HMGB3 | 22.979 |
| P31785 | Cytokine receptor common subunit gamma | IL2RG | 42.287 |
| P48539 | Calmodulin regulator protein PCP4 | PCP4 | 6.789 |
| P53992 | Protein transport protein Sec24C | SEC24C | 118.325 |
| Q9Y5S2 | Serine/threonine-protein kinase MRCK beta | CDC42BPB | 194.317 |
| Q9H8M5 | Metal transporter CNNM2 | CNNM2 | 96.623 |
| P07305 | Histone H1.0 | H1-0 | 20.861 |
| 015389 | Sialic acid-binding Ig-like lectin 5 | SIGLEC5 | 60.715 |
| Q9BT78 | COP9 signalosome complex subunit 4 | COPS4 | 46.271 |
| Q9BX10 | GTP-binding protein 2 | GTPBP2 | 65.767 |
| Q05940 | Synaptic vesicular amine transporter | SLC18A2 | 55.713 |
| 014976 | Cyclin-G-associated kinase | GAK | 143-193 |
| Q00577 | Transcriptional activator protein Pur-alpha | PURA | 34.908 |
| Q13155 | Aminoacyl tRNA synthase complex-interacting multifuncti | AIMP2 | 35.348 |
| Q9BUT1 | 3-hydroxybutyrate dehydrogenase type 2 | BDH2 | 26.723 |
| P0CG47 | Polyubiquitin-B | UBB | 25.76 |
| Q9UL15 | BAG family molecular chaperone regulator 5 | BAG5 | 51.2 |
| Q9BUJ0 | Protein ABHD14A | ABHD14A | 29-765 |
| P12270 | Nucleoprotein TPR | TPR | 267.298 |
| Q14739 | Delta(14)-sterol reductase LBR | LBR | 70.702 |
| Q8TCG2 | Phosphatidylinositol 4-kinase type 2-beta | PI4K2B | 54.745 |
| P42702 | Leukemia inhibitory factor receptor | LIFR | 123.745 |
| Q16602 | Calcitonin gene-related peptide type 1 receptor | CALCRL | 52.977 |
| Q8WY54 | Protein phosphatase 1E | PPM1E | 83.952 |
| P55084 | Trifunctional enzyme subunit beta, mitochondrial | HADHB | 51.296 |
| A6Q L63 | Ankyrin repeat and BTB/POZ domain-containing protein E | BTBD11 | 120.884 |
| Q9H2X3 | C-type lectin domain family 4 member M | CLEC4M | 45.351 |
| 015371 | Eukaryotic translation initiation factor 3 subunit D | EIF3D | 63.974 |
| P13688 | Carcinoembryonic antigen-related cell adhesion molecule | CEACAM1 | 57.561 |
| P26196 | Probable ATP-dependent RNA helicase DDX6 | DDX6 | 54.418 |
| Q04537 | Eukaryotic translation initiation factor 4 gamma 1 | EIF4G1 | 175.493 |
| 043657 | Tetraspanin-6 | TSPAN6 | 27.564 |
| Q9BZG1 | Ras-related protein Rab-34 | RAB34 | 29.045 |
| Q86TP1 | Exopolyphosphatase PRUNE1 | PRUNE1 | 50.201 |
| Q14566 | DNA replication licensing factor MCM6 | MCM6 | 92.889 |
| P43243 | Matrin-3 | MATR3 | 94.624 |
| Q8WW22 | DnaJ homolog subfamily A member 4 | DNAJA4 | 44.797 |
| Q96KP1 | Exocyst complex component 2 | EXOC2 | 104.066 |
| Q8WVY7 | Ubiquitin-like domain-containing CTD phosphatase 1 | UBLCP1 | 36.805 |
| P46379 | Large proline-rich protein BAG6 | BAG6 | 119.41 |
| P20936 | Ras GTPase-activating protein 1 | RASA1 | 116.405 |
| Q13884 | Beta-1-syntrophin | SNTB1 | 58.063 |
| Q04837 | Single-stranded DNA-binding protein, mitochondrial | SSBP1 | 17.257 |
| Q9H2G2 | STE20-like serine/threonine-protein kinase | SLK | 142.697 |
| Q8TDN6 | Ribosome biogenesis protein BRX1 homolog | BRIX1 | 41.403 |
| Q17RC7 | Exocyst complex component 3-like protein 4 | EXOC3L4 | 79.898 |
| 075900 | Matrix metalloproteinase-23 | MMP23B | 43.935 |
| Q9BVH7 | Alpha-N-acetylgalactosaminide alpha-2,6-sialyltransferas | ST6GALNAC5 | 38.441 |
| Q14194 | Dihydropyrimidinase-related protein 1 | CRMP1 | 62.185 |
| Q9H4E7 | Differentially expressed in FDCP 6 homolog | DEF6 | 73.911 |
| Q96HA4 | Uncharacterized protein C1orf159 | C1orf159 | 40.283 |
| Q15642 | Cdc42-interacting protein 4 | TRIP10 | 68.351 |
| Q6UXF1 | Transmembrane protein 108 | TMEM108 | 59.948 |
| P09496 | Clathrin light chain A | CLTA | 27.075 |
| P32971 | Tumor necrosis factor ligand superfamily member 8 | TNFSF8 | 26.017 |
| Q9NTJ5 | Phosphatidylinositol-3-phosphatase SAC1 | SACM1L | 66.968 |
| P19623 | Spermidine synthase | SRM | 33.824 |
| Q9UHV9 | Prefoldin subunit 2 | PFDN2 | 16.647 |
| 015554 | Intermediate conductance calcium-activated potassium cl | KCNN4 | 47.695 |
| P11168 | Solute carrier family 2, facilitated glucose transporter mer | SLC2A2 | 57.49 |
| Q9BPX5 | Actin-related protein 2/3 complex subunit 5-like protein | ARPC5L | 16.941 |
| Q00796 | Sorbitol dehydrogenase | SORD | 38.324 |
| 060313 | Dynamin-like 120 kDa protein, mitochondrial | OPA1 | 111.631 |
| 043324 | Eukaryotic translation elongation factor 1 epsilon-1 | EEF1E1 | 19.807 |
| P48454 | Serine/threonine-protein phosphatase 2B catalytic subun | PPP3CC | 58.129 |
| Q02153 | Guanylate cyclase soluble subunit beta-1 | GUCY1B1 | 70.514 |
| P37235 | Hippocalcin-like protein 1 | HPCALL | 22.312 |
| Q8IZ52 | Chondroitin sulfate synthase 2 | CHPF | 85.466 |
| Q9UKE5 | TRAF2 and NCK-interacting protein kinase | TNIK | 154.947 |
| Q92930 | Ras-related protein Rab-8B | RAB8B | 23.582 |

**Table 2-8**

| Uniprot Accession Number | Protein Name | Gene Symbol | MW(kDa) |
|---|---|---|---|
| O00178 | GTP-binding protein 1 | GTPBP1 | 72.457 |
| Q9UBQ7 | Glyoxylate reductase/hydroxypyruvate reductase | GRHPR | 35-668 |
| Q01085 | Nucleolysin TIAR | TIAL1 | 41.589 |
| Q13547 | Histone deacetylase 1 | HDAC1 | 55,104 |
| Q4KMP7 | TBC1 domain family member 10B | TBC1010B | 87.2 |
| P08183 | ATP-dependent translocase ABCB1 | ABCB1 | 141.479 |
| 060268 | Uncharacterized protein KIAA0513 | KIAA0513 | 46.639 |
| Q07666 | KH domain-containing, RNA-binding, signal transduction- | KHDRBS1 | 48.225 |
| P47992 | Lymphotactin | XCL1 | 12-513 |
| P62191 | 26S proteasome regulatory subunit 4 | PSMC1 | 49.185 |
| 015194 | CTD small phosphatase-like protein | CTDSPL | 31 127 |
| 075190 | Dnal homolog subfamily B member 6 | DNAJB6 | 36.086 |
| P49184 | Deoxyribonuclease-1-like 1 | DNASE1L1 | 33.892 |
| O43633 | Charged multivesicular body protein 2a | CHMP2A | 25.104 |
| Q13018 | Secretory phospholipase A2 receptor | PLA2R1 | 168.604 |
| Q02543 | 60S ribosomal protein L18a | RPL18A | 20.76 |
| O00622 | CCN family member 1 | CCN1 | 42.026 |
| P27708 | CAD protein | CAD | 242-985 |
| P63027 | Vesicle-associated membrane protein 2 | VAMP2 | 12.661 |
| Q96QE2 | Proton myo-inositol cotransporter | SLC2A13 | 70.37 |
| Q8N3D4 | EH domain-binding protein 1-like protein 1 | EHBP1L1 | 161.858 |
| Q9UKK3 | Protein mono-ADP-ribosyltransferase PARP4 | PARP4 | 192.598 |
| Q9P0T7 | Proton-transporting V-type ATPase complex assembly re | TMEM9 | 20.572 |
| P31040 | Succinate dehydrogenase [ubiquinone] flavoprotein subu | SDHA | 72.69 |
| P18077 | 60S ribosomal protein L35a | RPL35A | 12.536 |
| Q9UNH7 | Sorting nexin-6 | SNX6 | 46.647 |
| Q9UNE7 | E3 ubiquitin-protein ligase CHIP | STUB1 | 34.854 |
| P55259 | Pancreatic secretory granule membrane major glycoprote | GP2 | 59.479 |
| 075410 | Transforming acidic coiled-coil-containing protein 1 | TACC1 | 87-795 |
| 000308 | NEDD4-like E3 ubiquitin-protein ligase WWP2 | WWP2 | 98-914 |
| Q9P258 | Protein RCC2 | RCC2 | 56.085 |
| Q96T76 | MMS19 nucleotide excision repair protein homolog | MMS19 | 113.292 |
| P04271 | Protein S100-B | S100B | 10.709 |
| Q8WUX1 | Sodium-coupled neutral amino acid transporter 5 | SLC38A5 | 51.468 |
| Q9Y281 | Cofilin-2 | CFL2 | 18.737 |
| Q8IUN9 | C-type lectin domain family 10 member A | CLEC10A | 35.445 |
| Q9ULC4 | Malignant T-cell-amplified sequence 1 | MCTS1 | 20.555 |
| 060343 | TBC1 domain family member 4 | TBC1D4 | 146-567 |
| Q9UIG8 | Solute carrier organic anion transporter family member 3 | SLCO3A1 | 76.554 |
| Q7Z5R6 | Amyloid beta A4 precursor protein-binding family B mem | APBB1IP | 73.18 |
| Q9BV20 | Methylthioribose-1-phosphate isomerase | MRI1 | 39-147 |
| Q61A69 | Glutamine-dependent NAD(+) synthetase | NADSYN1 | 79.284 |
| Q6PCB5 | Lysine-specific demethylase RSBN1L | RSBN1L | 94.871 |
| 075508 | Claudin-11 | CLDN11 | 21.992 |
| Q9HB90 | Ras-related GTP-binding protein C | RRAGC | 44.223 |
| Q9Y4H4 | G-protein-signaling modulator 3 | GPSM3 | 17.862 |
| Q13488 | V-type proton ATPase 116 kDa subunit a 3 | TCIRG1 | 92.969 |
| Q9UBB6 | Neurochondrin | NCDN | 78.864 |
| 043143 | ATP-dependent RNA helicase DHX15 | DHX15 | 90.934 |
| 095971 | CD160 antigen | CD160 | 19.807 |
| 095816 | BAG family molecular chaperone regulator 2 | BAG2 | 23.771 |
| P40429 | 60S ribosomal protein L13a | RPL13A | 23.576 |
| P51811 | Endoplasmic reticulum membrane adapter protein XK | XK | 50.903 |
| Q8NBN3 | Transmembrane protein 87A | TMEM87A | 63.43 |
| P98171 | Rho GTPase-activating protein 4 | ARHGAP4 | 105.025 |
| 014617 | AP-3 complex subunit delta-1 | AP3D1 | 130.159 |
| 060749 | Sorting nexin-2 | SNX2 | 58.471 |
| PODMM9 | Sulfotransferase 1A3 | SULT1A3 | 34.196 |
| P52849 | Bifunctional heparan sulfate N-deacetylase/N-sulfotransf | NDST2 | 100.878 |
| Q9H270 | Vacuolar protein sorting-associated protein 11 homolog | VPS11 | 107.839 |
| Q9H425 | Uncharacterized protein C1orf198 | C1orf198 | 36.346 |
| Q9H0M0 | NEDD4-like E3 ubiquitin-protein ligase WWP1 | WWP1 | 105.203 |
| P06127 | T-cell surface glycoprotein CD5 | CD5 | 54.579 |
| Q9BZV1 | UBX domain-containing protein 6 | UBXN6 | 49.756 |
| P10515 | Dihydrolipoyllysine-residue acetyltransferase component | DLAT | 68.996 |
| P01889 | HLA class I histocompatibility antigen, B alpha chain | HLA-B | 40.46 |
| P43115 | Prostaglandin E2 receptor EP3 subtype | PTGER3 | 43.311 |
| Q9HBJ8 | Collectrin | CLTRN | 25.235 |
| 094826 | Mitochondrial import receptor subunit TOM70 | TOMM70 | 67-454 |
| 043286 | Beta-1,4-galactosyltransferase 5 | B4GALT5 | 45-12 |
| 000303 | Eukaryotic translation initiation factor 3 subunit F | EIF3F | 37.562 |
| P25098 | Beta-adrenergic receptor kinase 1 | GRK2 | 79.574 |
| Q92783 | Signal transducing adapter molecule 1 | STAM | 59-182 |
| P61952 | Guanine nucleotide-binding protein G(I)/G(S),/G(O) subur | GNG11 | 8.48 |
| Q15813 | Tubulin-specific chaperone E | TBCE | 59.348 |
| Q14116 | Interleukin-18 | IL18 | 22.328 |
| Q86VE9 | Serine incorporator 5 | SERINC5 | 47.01 |
| Q12933 | TNF receptor-associated factor 2 | TRAF2 | 55.859 |
| Q81WU5 | Extracellular sulfatase Sulf-2 | SULF2 | 100.456 |
| P43246 | DNA mismatch repair protein Msh2 | MSH2 | 104.745 |
| Q86YQ8 | Copine-8 | CPNE8 | 63.108 |
| P04637 | Cellular tumor antigen p53 | TP53 | 43.653 |
| Q9H7D7 | WD repeat-containing protein 26 | WDR26 | 72.125 |
| P10114 | Ras-related protein Rap-2a | RAP2A | 20.615 |
| Q687X5 | Metalloreductase STEAP4 | STEAP4 | 51.98 |
| Q8TB73 | Protein NDNF | NDNF | 64.674 |
| Q04609 | Glutamate carboxypeptidase 2 | FOLH1 | 84.332 |
| Q6ZSS7 | Major facilitator superfamily domain-containing protein 6 | MFSD6 | 88.089 |
| Q13258 | Prostaglandin D2 receptor | PTGDR | 40.271 |
| Q9UNS2 | COP9 signalosome complex subunit 3 | COPS3 | 47.874 |
| Q13554 | Calcium/calmodulin-dependent protein kinase type II sub | CAMK2B | 72.678 |
| Q8N766 | ER membrane protein complex subunit 1 | EMC1 | 111.761 |
| Q99962 | Endophilin-A1 | SH3GL2 | 39.96 |
| Q9Y262 | Eukaryotic translation initiation factor 3 subunit L | EIF3L | 66.726 |
| Q9UIJ5 | Palmitoyltransferase ZDHHC2 | ZDHHC2 | 42.022 |
| P42704 | Leucine-rich PPR motif-containing protein, mitochondrial | LRPPRC | 157.907 |
| P52306 | Rap1 GTPase-GDP dissociation stimulator 1 | RAP1GDS1 | 66.316 |
| Q99733 | Nucleosome assembly protein 1-like 4 | NAP1L4 | 42.824 |
| Q9H3M7 | Thioredoxin-interacting protein | TXNIP | 43.662 |
| Q5VZM2 | Ras-related GTP-binding protein B | RRAGB | 43.252 |
| 095777 | U6 snRNA-associated Sm-like protein LSm8 | LSM8 | 10.399 |
| 000148 | ATP-dependent RNA helicase DDX39A | DDX39A | 49.129 |
| P25685 | DnaJ homolog subfamily B member 1 | DNAJB1 | 38.043 |
| Q9H314 | DnaJ homolog subfamily C member 5 | DNAJC5 | 22.148 |
| Q02218 | 2-oxoglutarate dehydrogenase complex component E1 | OGDH | 115.937 |
| Q12873 | Chromodomain-helicase-DNA-binding protein 3 | CHD3 | 226.596 |

**Table 2-9**

| Uniprot Accession Number | Protein Name | Gene Symbol | MW(kDa) |
|---|---|---|---|
| Q2KHR3 | Glutamine and serine-rich protein 1 | QSER1 | 189.974 |
| Q96B97 | SH3 domain-containing kinase-binding protein 1 | SH3KBP1 | 73.126 |
| P35659 | Protein DEK | DEK | 42.675 |
| Q13492 | Phosphatidylinositol-binding clathrin assembly protein | PICALM | 70.755 |
| Q9NY12 | H/ACA ribonucleoprotein complex subunit 1 | GAR1 | 22.347 |
| Q9NPR9 | Protein GPR108 | GPR108 | 60.633 |
| P11387 | DNA topoisomerase 1 | TOP1 | 90.726 |
| Q9NR46 | Endophilin-B2 | SH3GLB2 | 43.977 |
| P42285 | Exosome RNA helicase MTR4 | MTREX | 117.807 |
| Q9H3N1 | Thioredoxin-related transmembrane protein 1 | TMX1 | 31.789 |
| A0A494C071 | PWWP domain-containing 4 | PWWP4 | 217.776 |
| Q7Z478 | ATP-dependent RNA helicase DHX29 | DHX29 | 155.239 |
| Q9Y3A6 | Transmembrane emp24 domain-containing protein 5 | TMED5 | 26.005 |
| P20702 | Integrin alpha-X | ITGAX | 127.832 |
| P33992 | DNA replication licensing factor MCM5 | MCM5 | 82.284 |
| Q92597 | Protein NDRG1 | NDRG1 | 42.836 |
| 014662 | Syntaxin-16 | STX16 | 37.031 |
| P53609 | Geranylgeranyl transferase type-1 subunit beta | PGGT1B | 42.369 |
| Q9Y5X1 | Sorting nexin-9 | SNX9 | 66.593 |
| 014495 | Phospholipid phosphatase 3 | PLPP3 | 35.117 |
| Q86VZ1 | P2Y purinoceptor 8 | P2RY8 | 40.636 |
| Q93034 | Cullin-5 | CUL5 | 90.956 |
| 060832 | H/ACA ribonucleoprotein complex subunit DKC1 | DKC1 | 57.673 |
| P20645 | Cation-dependent mannose-6-phosphate receptor | M6PR | 30.993 |
| 060739 | Eukaryotic translation initiation factor 1b | EIF1B | 12.82 |
| Q9Y6E2 | elF5-mimic protein 1 | BZW2 | 48.163 |
| Q9NUU7 | ATP-dependent RNA helicase DDX19A | DDX19A | 53.977 |
| P49758 | Regulator of G-protein signaling 6 | RGS6 | 54.424 |
| 075306 | NADH dehydrogenase [ubiquinone] iron-sulfur protein 2, | NDUFS2 | 52.545 |
| P19634 | Sodium/hydrogen exchanger 1 | SLC9A1 | 90.765 |
| 015438 | ATP-binding cassette sub-family C member 3 | ABCC3 | 169.344 |
| P56377 | AP-1 complex subunit sigma-2 | AP1S2 | 18.616 |
| 095825 | Quinone oxidoreductase-like protein 1 | CRYZL1 | 38.697 |
| P01911 | HLA class II histocompatibility antigen, DRB1 beta chain | HLA-DRB1 | 29.966 |
| P62917 | 60S ribosomal protein L8 | RPL8 | 28.026 |
| P42261 | Glutamate receptor 1 | GRIA1 | 101.508 |
| Q92747 | Actin-related protein 2/3 complex subunit 1A | ARPC1A | 41.57 |
| Q96AC1 | Fermitin family homolog 2 | FERMT2 | 77.86 |
| Q9Y5S1 | Transient receptor potential cation channel subfamily V | TRPV2 | 85.981 |
| Q19T08 | Endothelial cell-specific chemotaxis regulator | ECSCR | 21.293 |
| Q9BSK0 | MARVEL domain-containing protein 1 | MARVELD1 | 18.915 |
| Q8IUR7 | Armadillo repeat-containing protein 8 | ARMC8 | 75.511 |
| Q99758 | Phospholipid-transporting ATPase ABCA3 | ABCA3 | 191.365 |
| Q15436 | Protein transport protein Sec23A | SEC23A | 86.16 |
| Q9NQW7 | Xaa-Pro aminopeptidase 1 | XPNPEP1 | 69.92 |
| Q96EP5 | DAZ-associated protein 1 | DAZAP1 | 43.384 |
| Q9HA92 | Radical S-adenosyl methionine domain-containing protei | RSAD1 | 48.712 |
| Q9Y411 | Unconventional myosin-Va | MY05A | 215.409 |
| Q9NS93 | Transmembrane 7 superfamily member 3 | TM7SF3 | 64.167 |
| Q9UKY0 | Prion-like protein doppel | PRND | 20.293 |
| Q9HAV7 | GrpE protein homolog 1, mitochondrial | GRPEL1 | 24.281 |
| 060603 | Toll-like receptor 2 | TLR2 | 89.836 |
| Q13177 | Serine/threonine-protein kinase PAK 2 | PAK2 | 58.044 |
| P57088 | Transmembrane protein 33 | TMEM33 | 27.978 |
| Q99704 | Docking protein 1 | DOK1 | 52.391 |
| P54886 | Delta-1-pyrroline-5-carboxylate synthase | ALDH18A1 | 87.304 |
| Q9UBQ0 | Vacuolar protein sorting-associated protein 29 | VPS29 | 20.504 |
| 043760 | Synaptogyrin-2 | SYNGR2 | 24.81 |
| Q16836 | Hydroxyacyl-coenzyme A dehydrogenase, mitochondrial | HADH | 34.293 |
| 015372 | Eukaryotic translation initiation factor 3 subunit H | EIF3H | 39.931 |
| Q15417 | Calponin-3 | CNN3 | 36.414 |
| P20290 | Transcription factor BTF3 | BTF3 | 22.168 |
| Q15061 | WD repeat-containing protein 43 | WDR43 | 74.889 |
| Q715K2 | Wings apart-like protein homolog | WAPL | 132.946 |
| Q9UDY4 | DnaJ homolog subfamily B member 4 | DNAJB4 | 37.807 |
| Q9HBG7 | T-lymphocyte surface antigen Ly-9 | LY9 | 72-141 |
| 094811 | Tubulin polymerization-promoting protein | TPPP | 23.693 |
| P40939 | Trifunctional enzyme subunit alpha, mitochondrial | HADHA | 82.999 |
| Q96BI3 | Gamma-secretase subunit APH-1A | APH1A | 28.996 |
| P61966 | AP-1 complex subunit sigma-1A | AP1S1 | 18.733 |
| Q9BQI4 | Coiled-coil domain-containing protein 3 | CCDC3 | 30.732 |
| P18075 | Bone morphogenetic protein 7 | BMP7 | 49.314 |
| 095562 | Vesicle transport protein SFT2B | SFT2D2 | 17.775 |
| 043731 | ER lumen protein-retaining receptor 3 | KDELR3 | 25.028 |
| O00273 | DNA fragmentation factor subunit alpha | DFFA | 36.521 |
| Q09161 | Nuclear cap-binding protein subunit 1 | NCBP1 | 91.842 |
| Q9Y5P6 | Mannose-1-phosphate guanyltransferase beta | GMPPB | 39.834 |
| Q8N5K1 | CDGSH iron-sulfur domain-containing protein 2 | CISD2 | 15.278 |
| Q7L0J3 | Synaptic vesicle glycoprotein 2A | SV2A | 82.697 |
| Q13595 | Transformer-2 protein homolog alpha | TRA2A | 32.688 |
| P56192 | Methionine--tRNA ligase, cytoplasmic | MARS1 | 101.116 |
| P50225 | Sulfotransferase 1A1 | SULT1A1 | 34.166 |
| Q13261 | Interleukin-15 receptor subunit alpha | IL15RA | 28-232 |
| P51665 | 26S proteasome non-ATPase regulatory subunit 7 | PSMD7 | 37.025 |
| Q96D71 | RalBP1-associated Eps domain-containing protein 1 | REPS1 | 86.663 |
| Q8WTVO | Scavenger receptor class B member 1 | SCARB1 | 60.878 |
| P04439 | HLA class I histocompatibility antigen, A alpha chain | HLA-A | 40.84 |
| 000764 | Pyridoxal kinase | PDXK | 35.102 |
| P60866 | 40S ribosomal protein S20 | RPS20 | 13.369 |
| B5ME19 | Eukaryotic translation initiation factor 3 subunit C-like | pro EIF3CL | 105.475 |
| Q8NEZ2 | Vacuolar protein sorting-associated protein 37A | VPS37A | 44.314 |
| Q9NPQ8 | Synembryn-A | RIC8A | 59.708 |
| P14672 | Solute carrier family 2, facilitated glucose transporter | SLC2A4 | 54.788 |
| Q9UHX1 | Poly(U)-binding-splicing factor PUF60 | PUF60 | 59.876 |
| Q86Y34 | Adhesion G protein-coupled receptor G3 | ADGRG3 | 60.862 |
| Q6DD88 | Atlastin-3 | ATL3 | 60.542 |
| 043615 | Mitochondrial import inner membrane translocase | TIMMA4 | 51.356 |
| Q92558 | Actin-binding protein WASF1 | WASF1 | 61.652 |
| P42262 | Glutamate receptor 2 | GRIA2 | 98-823 |
| P78559 | Microtubule-associated protein 1A | MAP1A | 305.49 |
| Q86U42 | Polyadenylate-binding protein 2 | PABPN1 | 32.75 |
| Q9HOU4 | Ras-related protein Rab-1B | RAB1B | 22.171 |
| Q6UB35 | Monofunctional C1-tetrahydrofolate synthase, mitochondr | MTHFD1L | 105.788 |
| Q15397 | Pumilio homolog 3 | PUM3 | 73.585 |
| P02458 | Collagen alpha-1(II) chain | COL2A1 | 141.787 |
| Q03169 | Tumor necrosis factor alpha-induced protein 2 | TNFAIP2 | 72.663 |

**Table 2-10**

| Uniprot Accession Number | Protein Name | Gene Symbol | MW(kDa) |
|---|---|---|---|
| Q15459 | Splicing factor 3A subunit 1 | SF3A1 | 88887 |
| P43304 | Glycerol-3-phosphate dehydrogenase, mitochondrial | GFPD2 | 80.852 |
| O95757 | Heat shock 70 kDa protein 4L | HSPA4L | 94.515 |
| P57737 | Coronin-7 | CORO7 | 100.607 |
| Q8NB37 | Glutamine amidotransferase-like class 1 domain-containi | GATD1 | 23.298 |
| O14828 | Secretory carrier-associated membrane protein 3 | SCAMP3 | 38.286 |
| Q96MM6 | Heat shock 70 kDa protein 12B | HSPA12B | 75.689 |
| P27986 | Phosphatidylinositol 3-kinase regulatory subunit alpha | PIK3R1 | 83.6 |
| Q9P2R3 | Rabankyrin-5 | ANKFY1 | 128.399 |
| Q8IW52 | SLIT and NTRK-like protein 4 | SLITRK4 | 94.33 |
| P25105 | Platelet-activating factor receptor | PTAFR | 39.206 |
| Q92905 | COP9 signalosome complex subunit 5 | COPS5 | 37.579 |
| Q3V5L5 | Alpha-1,6-mannosylglycoprotein 6-beta-N-acetylglucosa | MGAT5B | 89.537 |
| P55036 | 26S proteasome non-ATPase regulatory subunit 4 | PSMD4 | 40.738 |
| Q14444 | Caprin-1 | CAPRIN1 | 78.364 |
| Q6PCE3 | Glucose 1,6-bisphosphate synthase | PGM2L1 | 70.441 |
| O14775 | Guanine nucleotide-binding protein subunit beta-5 | GNB5 | 43.568 |
| Q9NNX6 | CD209 antigen | CD209 | 45.774 |
| Q9Y310 | RNA-splicing ligase RtcB homolog | RTCB | 55.209 |
| Q02108 | Guanylate cyclase soluble subunit alpha-1 | GUCY1A1 | 77.452 |
| P01589 | Interleukin-2 receptor subunit alpha | IL2RA | 30.82 |
| Q6PUV4 | Complexin-2 | CPLX2 | 15.393 |
| Q52LJ0 | Protein FAM98B | FAM98B | 45.546 |
| Q6PI48 | Aspartate--tRNA ligase, mitochondrial | DARS2 | 73.563 |
| Q01629 | interferon-induced transmembrane protein 2 | IFITM2 | 14.633 |
| Q9HBI0 | Gamma-parvin | PARVG | 37.486 |
| O15078 | Centrosomal protein of 290 kDa | CEP290 | 290391 |
| O95342 | Bile salt export pump | ABCB11 | 146.409 |
| O43427 | Acidic fibroblast growth factor intracellular-binding protei | FIBP | 41.879 |
| Q14258 | E3 ubiquitin/ISG15 ligase TRIM25 | TRIM25 | 70.97% |
| Q9BVG4 | Protein PBDC1 | PBDC1 | 26.054 |
| Q9H6P5 | Threonine aspartase 1 | TASP1 | 44.457 |
| P19404 | NADH dehydrogenase [ubiquinone] flavoprotein 2, mitoch | NDUFV2 | 27.39 |
| Q9H9S4 | Calcium-binding protein 39-like | CAB39L | 39.088 |
| Q6IA17 | Single Ig IL-1-related receptor | SIGIRR | 45.679 |
| Q0VGL1 | Ragulator complex protein LAMTOR4 | LAMTOR4 | 10.74 |
| P49419 | Alpha-aminoadipic semialdehyde dehydrogenase | ALDH7A1 | 58.487 |
| Q11206 | CMP-N-acetylneuraminate-beta-galactosamide-alpha-2, | ST3GAL4 | 38-046 |
| P07204 | Thrombomodulin | THBD | 60.329 |
| Q9UHX3 | Adhesion G protein-coupled receptor E2 | ADGRE2 | 90.472 |
| Q13242 | Serine/arginine-rich splicing factor 9 | SRSF9 | 25.544 |
| P62760 | Visinin-like protein 1 | VSNL1 | 22.144 |
| O43583 | Density-regulated protein | DENR | 22.089 |
| P61163 | Alpha-centractin | ACTR1A | 42.613 |
| Q9NXL6 | SID1 transmembrane family member 1 | SIDT1 | 93.841 |
| Q9NX63 | MICOS complex subunit MIC19 | CHCHD3 | 26.152 |
| Q12929 | Epidermal growth factor receptor kinase substrate 8 | EPS8 | 91.884 |
| P51795 | H(+)/Ci(-) exchange transporter 5 | CLCN5 | 90.785 |
| P48729 | Casein kinase I isoform alpha | CSNK1A1 | 38.915 |
| P14784 | Interleukin-2 receptor subunit beta | IL2RB | 61.119 |
| P62304 | Small nuclear ribonucleoprotein E | SNRPE | 10.8 |
| O95573 | Fatty acid CoA ligase Acsl3 | ACSL3 | 80.422 |
| Q13509 | Tubulin beta-3 chain | TUBB3 | 50.432 |
| P49788 | Retinoic acid receptor responder protein 1 | RARRES1 | 33.285 |
| Q96AX1 | Vacuolar protein sorting-associated protein 33A | VPS33A | 67.613 |
| Q9UKA9 | Polypyrimidine tract-binding protein 2 | PTBP2 | 57.491 |
| O43823 | A-kinase anchor protein 8 | AKAP8 | 76.109 |
| P55263 | Adenosine kinase | ADK | 40.544 |
| Q9UP95 | Solute carrier family 12 member 4 | SLC12A4 | 120.65 |
| Q99504 | Eyes absent homolog 3 | EYA3 | 62.662 |
| Q8ND76 | Cyclin-Y | CCNY | 39.336 |
| Q9C0I1 | Myotubularin-related protein 12 | MTMR12 | 86.148 |
| Q9Y4P8 | WD repeat domain phosphoinositide-interacting protein 2 | WIPI2 | 49.408 |
| Q7Z7H5 | Transmembrane emp24 domain-containing protein 4 | TMED4 | 25.943 |
| Q9H583 | HEAT repeat-containing protein 1 | HEATR1 | 242.375 |
| Q9P109 | Beta-1,3-galactosyl-O-glycosyl-glycoprotein beta-1,6-N-a | GCNT4 | 53.052 |
| Q15738 | Sterol-4-alpha-carboxylate 3-dehydrogenase, decarboxyla | NSDHL | 41.9 |
| Q15019 | Septin-2 | SEPTIN2 | 41.488 |
| Q16222 | UDP-N-acetylhexosamine pyrophosphorylase | UAP1 | 58.769 |
| P35580 | Myosin-10 | MYH10 | 229.002 |
| Q9NRW4 | Dual specificity protein phosphatase 22 | DUSP22 | 20.911 |
| Q9UBX8 | Beta-1,4-galactosyltransferase 6 | B4GALT6 | 44.915 |
| Q9UPN7 | Serine/threonine-protein phosphatase 6 regulatory subun | PPP6R1 | 96.724 |
| O14787 | Transportin-2 | TNPO2 | 101.39 |
| Q7Z4V5 | Hepatoma-derived growth factor-related protein 2 | HDGFL2 | 74.317 |
| Q9NZ45 | CDGSH iron-sulfur domain-containing protein 1 | CISD1 | 12.199 |
| Q00765 | Receptor expression-enhancing protein 5 | REEP5 | 21.494 |
| P61011 | Signal recognition particle 54 kDa protein | SRP54 | 55.703 |
| Q9NTM9 | Copper homeostasis protein cutC homolog | CUTC | 29.34 |
| Q96AE4 | Far upstream element-binding protein 1 | FUBP1 | 67-561 |
| Q6IQ22 | Ras-related protein Rab-12 | RAB12 | 27.248 |
| Q00059 | Transcription factor A, mitochondrial | TFAM | 29.097 |
| Q13148 | TAR DNA-binding protein 43 | TARDBP | 44.74 |
| P38117 | Electron transfer flavoprotein subunit beta | ETFB | 27.842 |
| Q6UXN9 | WD repeat-containing protein 82 | WDR82 | 35.078 |
| P55735 | Protein SEC13 homolog | SEC13 | 35.542 |
| Q07817 | Bcl-2-like protein 1 | BCL2L1 | 26.049 |
| Q9NRG1 | Phosphoribosyltransferase domain-containing protein 1 | PRTFDC1 | 25.672 |
| P27695 | DNA-(apurinic or apyrimidinic site) endonuclease | APEX1 | 35.555 |
| Q96CG8 | Collagen triple helix repeat-containing protein 1 | CTHRC1 | 26.222 |
| P48147 | Prolyl endopeptidase | PREP | 80.699 |
| Q5VYK3 | Proteasome adapter and scaffold protein ECM29 | ECPAS | 204.294 |
| Q96C36 | Pyrroline-5-carboxylate reductase 2 | PYCR2 | 33.637 |
| P35670 | Copper-transporting ATPase 2 | ATP7B | 157.262 |
| Q9UL26 | Ras-related protein Rab-22A | RAB22A | 21.856 |
| Q9NSD9 | Phenylalanine--tRNA ligase beta subunit | FARSB | 66.117 |
| Q6NVY1 | 3-hydroxyisobutyryl-CoA hydrolase, mitochondrial | HIBCH | 43.484 |
| Q9H221 | ATP-binding cassette sub-family G member 8 | ABCG8 | 75.678 |
| Q9Y2R4 | Probable ATP-dependent RNA helicase DDX52 | DDX52 | 67.466 |
| Q29983 | MHC class I polypeptide-related sequence A | MICA | 42.915 |
| Q6ZMI0 | Protein phosphatase 1 regulatory subunit 21 | PPP1R21 | 88.315 |
| P48029 | Sodium- and chloride-dependent creatine transporter 1 | SLC6A8 | 70.525 |
| Q02750 | Dual specificity mitogen-activated protein kinase kinase 1 | MAP2K1 | 43.438 |
| O76021 | Ribosomal L1 domain-containing protein 1 | RSL1D1 | 54.974 |
| Q96EC8 | Protein YIPF6 | YIPF6 | 26.257 |
| P46779 | 60S ribosomal protein L28 | RPL28 | 15.746 |

**Table 2-11**

| Uniprot Accession Number | Protein Name | Gene Symbol | MW(kDa) |
|---|---|---|---|
| O60763 | General vesicular transport factor p115 | USO1 | 107.895 |
| Q9Y450 | HBS1-like protein | HBS1L | 75.475 |
| Q02083 | N-acylethanolamine-hydrolyzing acid amidase | NAAA | 40.066 |
| P42695 | Condensin-2 complex subunit D3 | NCAPD3 | 168.893 |
| Q96BF3 | Transmembrane and immunoglobulin domain-containing | TMIGD2 | 30.675 |
| Q14152 | Eukaryotic translation initiation factor 3 subunit A | EIF3A | 166.569 |
| Q13616 | Cullin-1 | CUL1 | 89.679 |
| P48067 | Sodium- and chloride-dependent glycine transporter 1 | SLC6A9 | 78.26 |
| Q96MU7 | YTH domain-containing protein 1 | YTHDC1 | 84.701 |
| Q6UXK2 | Immunoglobulin superfamily containing leucine-rich repe | ISLR2 | 78.992 |
| Q8NES3 | Beta-1,3-N-acetylglucosaminyltransferase lunatic fringe | LFNG | 41.773 |
| Q14155 | Rho guanine nucleotide exchange factor 7 | ARHGEF7 | 90.013 |
| O95436 | Sodium-dependent phosphate transport protein 2B | SLC34A2 | 75.76 |
| Q8NBI5 | Equilibrative nucleobase transporter 1 | SLC43A3 | 54.527 |
| O43169 | Cytochrome b5 type B | CYB5B | 16.697 |
| P49137 | MAP kinase-activated protein kinase 2 | MAPKAPK2 | 45.569 |
| A5YKK6 | CCR4-NOT transcription complex subunit 1 | CNOT1 | 266.945 |
| Q9C0C4 | Semaphorin-4C | SEMA4C | 92.624 |
| P13861 | cAMP-dependent protein kinase type II-alpha regulatory | PRKAR2A | 45.52 |
| P06731 | Carcinoembryonic antigen-related cell adhesion molecule | CEACAM5 | 76.795 |
| O14494 | Phospholipid phosphatase 1 | PLPP1 | 32.155 |
| Q99519 | Sialidase-1 | NEU1 | 45.468 |
| P11388 | DNA topoisomerase 2-alpha | TOP2A | 174.387 |
| Q8TC12 | Retinol dehydrogenase 11 | RDH11 | 35.386 |
| O15305 | Phosphomannomutase 2 | PMM2 | 28.082 |
| P78310 | Coxsackievirus and adenovirus receptor | CXADR | 40.032 |
| Q17RW2 | Collagen alpha-1(XXIV) chain | COL24A1 | 175.498 |
| O43182 | Rho GTPase-activating protein 6 | ARHGAP6 | 105.948 |
| Q5SW96 | Low density lipoprotein receptor adapter protein 1 | LDLRAP1 | 33.883 |
| Q8TB36 | Ganglioside-induced differentiation-associated protein 1 | GDAP1 | 41.347 |
| P78325 | Disintegrin and metalloproteinase domain-containing pro | ADAM8 | 88.772 |
| Q4G0M1 | Erythroferrone | ERFE | 37.28 |
| Q8N3J6 | Cell adhesion molecule 2 | CADM2 | 47.552 |
| P53602 | Diphosphomevalonate decarboxylase | MVD | 43.404 |
| Q9UGI8 | Testin | TES | 47.998 |
| O95456 | Proteasome assembly chaperone 1 | PSMG1 | 32.855 |
| Q8N1G2 | Cap-specific mRNA (nucleoside-2'-O-)-methyltransferas | CMTR1 | 95.321 |
| Q96RS6 | NudC domain-containing protein 1 | NUDCD1 | 66.755 |
| Q96RFO | Sorting nexin-18 | SNX18 | 68.895 |
| P42263 | Glutamate receptor 3 | GRIA3 | 101.157 |
| Q9BPX3 | Condensin complex subunit 3 | NCAPG | 114.337 |
| P26006 | Integrin alpha-3 | ITGA3 | 116.612 |
| Q5W0Z9 | Palmitoyltransferase ZDHHC20 | ZDHHC20 | 42.278 |
| O15243 | Leptin receptor gene-related protein | LEPROT | 14.253 |
| Q9Y2I8 | WD repeat-containing protein 37 | WDR37 | 54.665 |
| P49019 | Hydroxycarboxylic acid receptor 3 | HCAR3 | 44.477 |
| O60513 | Beta-1,4-galactosyltransferase 4 | B4GALT4 | 40.041 |
| O75694 | Nuclear pore complex protein Nup155 | NUP155 | 155.203 |
| O43865 | S-adenosylhomocysteine hydrolase-like protein 1 | AHCYL1 | 58.953 |
| Q9Y3B4 | Splicing factor 3B subunit 6 | SF3B6 | 14.585 |
| Q68EM7 | Rho GTPase-activating protein 17 | ARHGAP17 | 95.437 |
| Q9H773 | dCTP pyrophosphatase 1 | DCTPP1 | 18.682 |
| Q8NBI2 | Lysosomal membrane ascorbate-dependent ferrireductas | CYB561A3 | 27.213 |
| Q16795 | NADH dehydrogenase [ubiquinone] 1 alpha subcomplex s | NDUFA9 | 42.509 |
| Q69YW2 | Protein stum homolog | STUM | 15.007 |
| Q12767 | Transmembrane protein 94 | TMEM94 | 151.153 |
| P20585 | DNA mismatch repair protein Msh3 | MSH3 | 127.415 |
| O15355 | Protein phosphatase 1G | PPM1G | 59.271 |
| Q86TV6 | Tetratricopeptide repeat protein 7B | TTC7B | 94.182 |
| O43447 | Peptidyl-prolyl cis-trans isomerase H | PPIH | 19.207 |
| O00458 | Interferon-related developmental regulator 1 | IFRD1 | 50.268 |
| Q9UBU9 | Nuclear RNA export factor 1 | NXF1 | 70.183 |
| O94915 | Protein furry homolog-like | FRYL | 339.603 |
| P00749 | Urokinase-type plasminogen activator | PLAU | 48.525 |
| Q9NVP1 | ATP-dependent RNA helicase DDX18 | DDX18 | 75.408 |
| Q6P3W7 | SCY1-like protein 2 | SCYL2 | 103.711 |
| O00232 | 26S proteasome non-ATPase regulatory subunit 12 | PSMD12 | 52.906 |
| O15552 | Free fatty acid receptor 2 | FFAR2 | 37.144 |
| P41440 | Reduced folate transporter | SLC19A1 | 64.869 |
| P63151 | Serine/threonine-protein phosphatase 2A 55 kDa regulate | PPP2R2A | 51.692 |
| Q9UHR4 | Brain-specific angiogenesis inhibitor 1-associated protein | BAIAP2L1 | 56.884 |
| O14727 | Apoptotic protease-activating factor 1 | APAF1 | 141.843 |
| Q9UBE0 | SUMO-activating enzyme subunit 1 | SAE1 | 38.449 |
| P20674 | Cytochrome c oxidase subunit 5A, mitochondrial | COX5A | 16.756 |
| A6NDG6 | Glycerol-3-phosphate phosphatase | PGP | 34.003 |
| P09884 | DNA polymerase alpha catalytic subunit | POLA1 | 165.915 |
| Q2M389 | WASH complex subunit 4 | WASHC4 | 136.404 |
| Q8WWK9 | Cytoskeleton-associated protein 2 | CKAP2 | 76.989 |
| O14979 | Heterogeneous nuclear ribonucleoprotein D-like | HNRNPDL | 46.439 |
| P27448 | MAP/microtubule affinity-regulating kinase 3 | MARK3 | 84.429 |
| Q92478 | C-type lectin domain family 2 member B | CLEC2B | 17.307 |
| P46977 | Dolichyl-diphosphooligosaccharide--protein glycosyltrans | STT3A | 80.53 |
| Q16401 | 26S proteasome non-ATPase regulatory subunit 5 | PSMD5 | 56.195 |
| Q69YN4 | Protein virilizer homolog | VIRMA | 202.027 |
| Q8N0U8 | Vitamin K epoxide reductase complex subunit 1-like | VKORC1L1 | 19.835 |
| Q96F15 | GTPase IMAP family member 5 | GIMAP5 | 34.847 |
| Q9P260 | RAB11-binding protein RELCH | RELCH | 134.633 |
| Q9H9S5 | Ribitol 5-phosphate transferase FKRP | FKRP | 54.569 |
| Q9H2U2 | Inorganic pyrophosphatase 2, mitochondrial | PPA2 | 37.92 |
| Q8TB61 | Adenosine 3'-phospho 5'-phosphosulfate transporter 1 | SLC35B2 | 47.515 |
| A6NI72 | Putative neutrophil cytosol factor 1B | NCF1B | 44.819 |
| Q9UPY6 | Actin-binding protein WASF3 | WASF3 | 55.295 |
| P68400 | Casein kinase II subunit alpha | CSNK2A1 | 45.142 |
| Q9BXW9 | Fanconi anemia group D2 protein | FANCD2 | 164.13 |
| Q8NF50 | Dedicator of cytokinesis protein 8 | DOCK8 | 238.534 |
| O14966 | Ras-related protein Rab-7L1 | RAB29 | 23.152 |
| Q12999 | Tetraspanin-31 | TSPAN31 | 23.063 |
| P21462 | fMet-Leu-Phe receptor | FPR1 | 38.444 |
| O43508 | Tumor necrosis factor ligand superfamily member 12 | TNFSF12 | 27.215 |
| Q14690 | Protein RRP5 homolog | PDCD11 | 208.704 |
| Q9NZQ3 | NCK-interacting protein with SH3 domain | NCKIPSD | 78.961 |
| AOFGR8 | Extended synaptotagmin-2 | ESYT2 | 102.358 |
| Q92688 | Acidic leucine-rich nuclear phosphoprotein 32 family | ANP32B | 28.786 |
| Q7Z2W9 | 39S ribosomal protein L21, mitochondrial | MRPL21 | 22.816 |
| P08247 | Synaptophysin | SYP | 33.845 |
| O60841 | Eukaryotic translation initiation factor 5B | EIF5B | 138.829 |

**Table 2-12**

| Uniprot Accession Number | Protein Name | Gene Symbol | MW(kDa) |
|---|---|---|---|
| O94832 | Unconventional myosin-Id | MYO1D | 116.205 |
| P23352 | Anosmin-1 | ANOS1 | 76.115 |
| Q6P9G4 | Transmembrane protein 154 | TMEM154 | 20.497 |
| P13051 | Uracil-DNA glycosylase | UNG | 34.645 |
| Q13247 | Serine/arginine-rich splicing factor 6 | SRSF6 | 39.589 |
| O75165 | DnaJ homolog subfamily C member 13 | DNAJC13 | 254.422 |
| Q15029 | 116 kDa U5 small nuclear ribonucleoprotein component | EFTUD2 | 109.439 |
| P42338 | Phosphatidylinositol 4,5-bisphosphate 3-kinase catalytic | PIK3CB | 122.764 |
| O15321 | Transmembrane 9 superfamily member 1 | TM9SF1 | 68.861 |
| Q9NSE4 | Isoleucine--tRNA ligase, mitochondrial | IARS2 | 113.793 |
| Q8NI60 | Atypical kinase COQ8A, mitochondrial | COQ8A | 71.949 |
| Q14240 | Eukaryotic initiation factor 4A-II | EIF4A2 | 46.403 |
| O75795 | UDP-glucuronosyltransferase 2B17 | UGT2B17 | 61.095 |
| Q8IZL8 | Proline-, glutamic acid- and leucine-rich protein 1 | PELP1 | 119.704 |
| Q99961 | Endophilin-A2 | SH3GL1 | 41.49 |
| Q15027 | Arf-GAP with coiled-coil, ANK repeat and PH domain-con | ACAP1 | 81-537 |
| O00469 | Procollagen-lysine,2-oxoglutarate 5-dioxygenase 2 | PLOD2 | 84.686 |
| O43566 | Regulator of G-protein signaling 14 | RGS14 | 61.446 |
| P22059 | Oxysterol-binding protein 1 | OSBP | 89.422 |
| Q02817 | Mucin-2 | MUC2 | 540.315 |
| P43686 | 26S proteasome regulatory subunit 6B | PSMC4 | 47.367 |
| Q9Y295 | Developmentally-regulated GTP-binding protein 1 | DRG1 | 40.543 |
| P42680 | Tyrosine-protein kinase Tec | TEC | 73.581 |
| Q01970 | 1-phosphatidylinositol 4,5-bisphosphate phosphodiestera | PLCB3 | 138.802 |
| A0AVT1 | Ubiquitin-like modifier-activating enzyme 6 | UBA6 | 117.972 |
| Q9NV70 | Exocyst complex component 1 | EXOC1 | 101.984 |
| O60645 | Exocyst complex component 3 | EXOC3 | 85.567 |
| Q13241 | Natural killer cells antigen CD94 | KLRD1 | 20.512 |
| Q7Z2Q7 | Leucine-rich repeat-containing protein 70 | LRRC70 | 70.303 |
| Q6WRI0 | Immunoglobulin superfamily member 10 | IGSF10 | 290.843 |
| Q96P47 | Arf-GAP with GTPase, ANK repeat and PH domain-contai | AGAP3 | 95.046 |
| P51151 | Ras-related protein Rab-9A | RAB9A | 22.838 |
| Q9BQS7 | Hephaestin | HEPH | 130.451 |
| O43561 | Linker for activation of T-cells family member 1 | LAT | 27.929 |
| Q9HC35 | Echinoderm microtubule-associated protein-like 4 | EML4 | 108.916 |
| Q92615 | La-related protein 4B | LARP4B | 80.552 |
| Q9NZN4 | EH domain-containing protein 2 | EHD2 | 61.16 |
| Q92888 | Rho guanine nucleotide exchange factor 1 | ARHGEF1 | 102.435 |
| P62306 | Small nuclear ribonucleoprotein F | SNRPF | 9.722 |
| P42345 | Serine/threonine-protein kinase mTOR | MTOR | 288.896 |
| Q86YS7 | C2 domain-containing protein 5 | C2CD5 | 110.448 |
| Q9NZ32 | Actin-related protein 10 | ACTR10 | 46.307 |
| P22307 | Sterol carrier protein 2 | SCP2 | 58-995 |
| Q14684 | Ribosomal RNA processing protein 1 homolog B | RRP1B | 84.428 |
| P60903 | Protein S100-A10 | S100A10 | 11.2 |
| Q9BZZ5 | Apoptosis inhibitor 5 | API5 | 59.005 |
| Q8WWY8 | Lipase member H | LIPH | 50.863 |
| P51805 | Plexin-A3 | PLXNA3 | 207.719 |
| Q6YN16 | Hydroxysteroid dehydrogenase-like protein 2 | HSDL2 | 45.398 |
| Q9BXP2 | Solute carrier family 12 member 9 | SLC12A9 | 96.111 |
| P12314 | High affinity immunoglobulin gamma Fc receptor I | FCGR1A | 42.63 |
| Q86XS5 | Angiopoietin-related protein 5 | ANGPTL5 | 44.144 |
| Q8NBU5 | Outer mitochondrial transmembrane helix translocase | ATAD1 | 40.744 |
| P11274 | Breakpoint cluster region protein | BCR | 142.823 |
| Q12789 | General transcription factor 3C polypeptide 1 | GTF3C1 | 238.879 |
| P43403 | Tyrosine-protein kinase ZAP-70 | ZAP70 | 69.875 |
| P32019 | Type II inositol 1,4,5-trisphosphate 5-phosphatase | INPP5B | 112.853 |
| P05981 | Serine protease hepsin | HPN | 45.011 |
| O95299 | NADH dehydrogenase [ubiquinone] 1 alpha subcomplex s | NDUFA10 | 40.751 |
| P04920 | Anion exchange protein 2 | SLC4A2 | 137.012 |
| Q6UX27 | V-set and transmembrane domain-containing protein 1 | VSTM1 | 26.11 |
| O00471 | Exocyst complex component 5 | EXOC5 | 81.85 |
| Q8N568 | Serine/threonine-protein kinase DCLK2 | DCLK2 | 83.608 |
| Q86XE3 | Calcium uptake protein 3, mitochondrial | MICU3 | 60.713 |
| Q9H497 | Torsin-3A | TOR3A | 46.199 |
| O14958 | Calsequestrin-2 | CASQ2 | 46.436 |
| O75607 | Nucleoplasmin-3 | NPM3 | 19.344 |
| Q9H305 | Cell death-inducing p53-target protein 1 | CDIP1 | 21.892 |
| P51812 | Ribosomal protein S6 kinase alpha-3 | RPS6KA3 | 83.737 |
| Q92959 | Solute carrier organic anion transporter family member | 2ASLCO2A1 | 70.044 |
| O14802 | DNA-directed RNA polymerase III subunit RPC1 | POLR3A | 155.645 |
| Q07157 | Tight junction protein ZO-1 | TJP1 | 195.461 |
| P12081 | Histidine--tRNA ligase, cytoplasmic | HARS1 | 57.411 |
| Q9H1K6 | Talin rod domain-containing protein 1 | TLNRD1 | 37.757 |
| Q5T447 | E3 ubiquitin-protein ligase HECTD3 | HECTD3 | 97.114 |
| Q01581 | Hydroxymethylglutaryl-CoA synthase, cytoplasmic | HMGCS1 | 57.291 |
| Q8N8S7 | Protein enabled homolog | ENAH | 66.51 |
| Q9NZD2 | Glycolipid transfer protein | GLTP | 23.849 |
| Q13620 | Cullin-4B | CUL4B | 103.981 |
| Q9UBS9 | SUN domain-containing ossification factor | SUCO | 139.432 |
| Q9BXB4 | Oxysterol-binding protein-related protein 11 | OSBPL11 | 83.642 |
| Q92979 | Ribosomal RNA small subunit methyltransferase NEP1 | EMG1 | 26.718 |
| Q9P2E5 | Chondroitin sulfate glucuronyltransferase | CHPF2 | 85.949 |
| Q6P1N0 | Coiled-coil and C2 domain-containing protein 1A | CC2D1A | 104.061 |
| Q96P70 | Importin-9 | IPO9 | 115.964 |
| O43818 | U3 small nucleolar RNA-interacting protein 2 | RRP9 | 51.841 |
| Q9UHB9 | Signal recognition particle subunit SRP68 | SRP68 | 70.73 |
| 095633 | Follistatin-related protein 3 | FSTL3 | 27.665 |
| Q9UJX2 | Cell division cycle protein 23 homolog | CDC23 | 68.834 |
| Q9Y219 | Protein jagged-2 | JAG2 | 133.368 |
| Q96K17 | Transcription factor BTF3 homolog 4 | BTF3L4 | 17.268 |
| O75155 | Cullin-associated NEDD8-dissociated protein 2 | CAND2 | 135.259 |
| P52732 | Kinesin-like protein KIF11 | KIF11 | 119.16 |

### [Example 2]

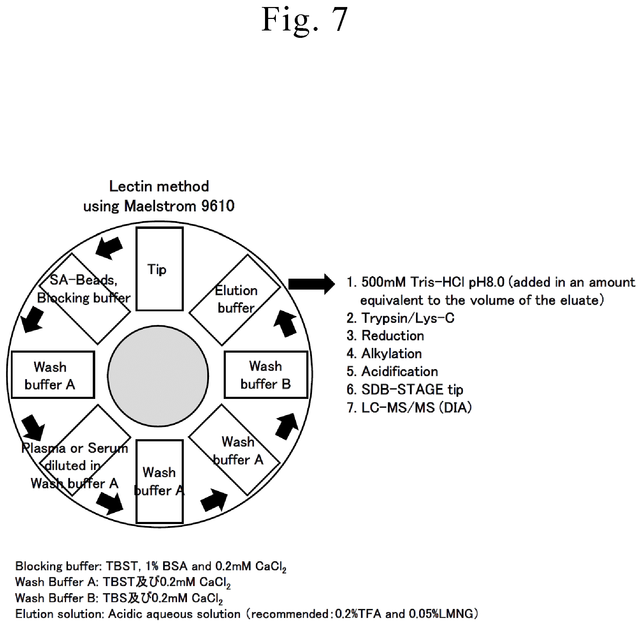

In Example 1, as shown in Figure 1, lectins were denatured with SDS to elute bound proteins from the lectin column; however, since SDS inhibits enzymatic digestion, SDS was removed from the eluted proteins by the SP3 method after elution. In this Example, an acidic aqueous solution (0.2% TFA) was used as the elution solution. After acid-denaturing the lectin column to elute bound proteins, the obtained eluate was neutralized with a buffer to return to a pH optimal for enzymatic digestion, thereby enabling proteome analysis without the step for SP3 method. A schematic diagram of a method for the enrichment of trace serum proteins using a biotinylated lectin column according to this improved method is shown in Figure 7. To prevent contamination of the eluted proteins with surfactants unsuitable for pretreatment in proteome analysis, it is necessary to wash the lectin column with an appropriate buffer before applying the elution solution. In this Example, TBST containing 0.2 mM CaCl₂ was used as a washing buffer, and an aqueous solution of 0.2% TFA was used as an acidic aqueous solution for elution. This acidic aqueous solution for elution contained 0.05% LMNG depending on the experimental conditions. LMNG is a surfactant that suppresses non-specific adsorption of proteins to columns and tubes without inhibiting enzymatic digestion. The eluted protein solution was neutralized by adding an equal volume of 500 mM Tris-HCl aqueous solution (pH 8.0), and then directly digested with trypsin/Lys-C without SP3 treatment. Other experimental conditions were the same as those in Example 1, and the numbers of identified proteins were compared between the improved method and the method of Example 1.

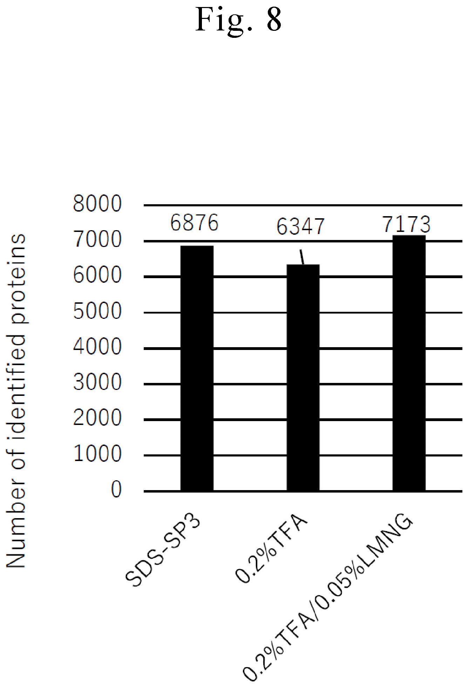

The results are shown in Figure 8. When 0.2% TFA aqueous solution was used as the elution solution and the SP3 treatment was omitted, the number of identified proteins decreased by only about 8% compared with the method of Example 1 in which proteins were eluted with SDS followed by SP3 treatment (SDS-SP3). Furthermore, addition of LMNG to the 0.2% TFA aqueous solution resulted in the detection of a greater number of proteins compared to the SDS-SP3 method. Since LMNG can be easily removed by a reversed-phase spin column, no additional pretreatment steps are required. The elution conditions of 0.2% TFA/0.05% LMNG made it possible to reduce the processing steps as compared with the SDS-SP3 method and to increase the number of identified proteins by recovering and digesting the eluate without losses associated with SP3 treatment.

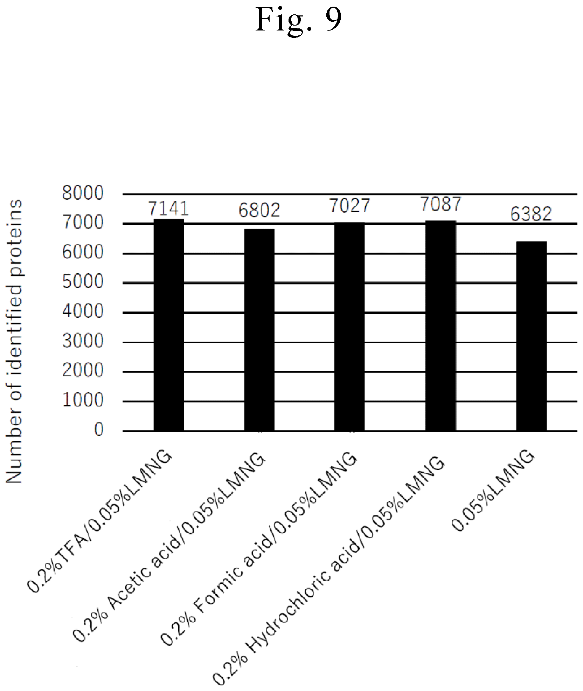

Next, differences in the number of identified proteins eluted depending on the type of acid were investigated. The results are shown in Figure 9. A tendency was observed in that a greater number of proteins were detected with strongly acidic solutions such as TFA and hydrochloric acid; however, it was confirmed that protein elution from the lectin column was enhanced with any of the acids tested.

From the above, it was suggested that elution of lectin-bound proteins using an acidic aqueous solution containing LMNG enables simple and high-depth proteome analysis.

### INDUSTRIAL APPLICABILITY

According to the present invention, highly abundant proteins that hinder deepening of LC-MS analysis can be efficiently removed from a biological sample such as serum and plasma, while trace proteins useful as biomarkers can be preferentially enriched. Since a sample prepared by the method of the present invention contains a large amount of highly enriched trace proteins, contamination of highly abundant proteins is suppressed, and the dynamic range of protein concentrations is reduced, when it is used for LC-MS analysis, the depth of proteome analysis is improved, and a large number of proteins can be detected.

This application is based on Japanese Patent Application No. 2023-211819 (filed on December 15, 2023), the entire contents of which are incorporated herein by reference.

## Claims

1. A preparation method of a protein- or peptide-containing sample for identifying proteins contained in a biological sample by mass spectrometry, comprising contacting the biological sample with a carrier on which a lectin selected from the group consisting of N-acetylglucosamine oligomer-binding lectins and fucose-binding lectins, or a binding fragment thereof, is immobilized, to recover glycoproteins having binding affinity to the lectin in the biological sample.

2. The preparation method according to claim 1, wherein the lectin or the binding fragment thereof is an N-acetylglucosamine oligomer-binding lectin selected from the group consisting of *Solanum tuberosum* lectin, *Lycopersicon esculentum* lectin, *Datura stramonium* lectin, and wheat germ agglutinin, or a binding fragment thereof.

3. The preparation method according to claim 1, wherein the lectin or the binding fragment thereof is a fucose-binding lectin selected from the group consisting of *Ulex europaeus* agglutinin-1, *Aleuria aurantia* lectin, and *Aspergillus oryzae* lectin, or a binding fragment thereof.

4. The preparation method according to claim 1, wherein the lectin or the binding fragment thereof is a combination comprising:
*1) Solanum tuberosum* lectin or a binding fragment thereof; and
2) a lectin selected from the group consisting of *Lycopersicon esculentum* lectin, *Datura stramonium* lectin, wheat germ agglutinin, and *Aleuria aurantia* lectin, or a binding fragment thereof.

5. The preparation method according to claim 4, wherein the lectin or the binding fragment thereof is a combination comprising:
1) *Solanum tuberosum* lectin or a binding fragment thereof; and
*2) Lycopersicon esculentum* lectin or a binding fragment thereof.

6. The preparation method according to claim 1, further comprising subjecting the recovered glycoproteins in the biological sample to reductive alkylation.

7. The preparation method according to claim 1, further comprising digesting the recovered glycoproteins in the biological sample with a protease to obtain a peptide mixture as a digestion product.

8. The preparation method according to claim 7, further comprising applying the obtained peptide mixture to a desalting column to obtain a purified peptide mixture.

9. The preparation method according to any one of claims 1 to 8, wherein the biological sample is selected from the group consisting of serum, plasma, whole blood, cerebrospinal fluid, ascites, synovial fluid, and lymph.

10. The preparation method according to any one of claims 1 to 9, wherein the glycoproteins having binding affinity to the lectin in the biological sample are recovered by eluting proteins bound to the lectin or the binding fragment thereof with an acidic aqueous solution.

11. The preparation method according to claim 10, wherein the acidic aqueous solution contains a sugar-based nonionic surfactant.

12. The preparation method according to claim 11, wherein the sugar-based nonionic surfactant is LMNG.

13. The preparation method according to any one of claims 10 to 12, further comprising neutralizing the eluate and treating it with a protease to digest the recovered glycoproteins in the biological sample in the eluate, thereby obtaining a peptide mixture as a digestion product.

14. A method for identifying a protein contained in a biological sample by mass spectrometry, comprising the following steps:
1) contacting the biological sample with a carrier on which a lectin selected from the group consisting of N-acetylglucosamine oligomer-binding lectins and fucose-binding lectins, or a binding fragment thereof is immobilized, to recover glycoproteins having binding affinity to the lectin in the biological sample;
2) digesting the recovered glycoproteins in the biological sample with a protease to obtain a peptide mixture as a digestion product;
3) subjecting the obtained peptide mixture to a desalting treatment to obtain a purified peptide mixture;
4) subjecting the purified peptide mixture to a mass spectrometer to identify peptides contained in the peptide mixture based on the obtained mass spectrometry results; and
5) identifying a protein in the biological sample from which the peptide is derived based on the information of the identified peptides.

15. The method according to claim 14, wherein the lectin or the binding fragment thereof is an N-acetylglucosamine oligomer-binding lectin selected from the group consisting of *Solanum tuberosum* lectin, *Lycopersicon esculentum* lectin, *Datura stramonium* lectin, and wheat germ agglutinin, or a binding fragment thereof.

16. The method according to claim 14, wherein the lectin or the binding fragment thereof is a fucose-binding lectin selected from the group consisting of *Ulex europaeus* agglutinin-1, *Aleuria aurantia* lectin, and *Aspergillus oryzae* lectin, or a binding fragment thereof.

17. The method according to claim 14, wherein the lectin or the binding fragment thereof is a combination comprising:
1) *Solanum tuberosum* Lectin or a binding fragment thereof; and
2) a lectin selected from the group consisting of *Lycopersicon esculentum* lectin, *Datura stramonium* lectin, wheat germ agglutinin, and *Aleuria aurantia* lectin, or a binding fragment thereof.

18. The method according to claim 17, wherein the lectin or the binding fragment thereof is a combination comprising:
*1) Solanum tuberosum* Lectin or a binding fragment thereof; and
*2) Lycopersicon esculentum* lectin or a binding fragment thereof.

19. The method according to claim 14, further comprising subjecting the glycoproteins in the biological sample recovered in step 1 to reductive alkylation.

20. The method according to any one of claims 14 to 19, wherein the biological sample is selected from the group consisting of serum, plasma, whole blood, cerebrospinal fluid, ascites, synovial fluid, and lymph.

21. The method according to any one of claims 14 to 20, wherein, in step 1, the glycoproteins having binding affinity to the lectin in the biological sample are recovered by eluting proteins bound to the lectin or the binding fragment thereof with an acidic aqueous solution.

22. The preparation method according to claim 21, wherein the acidic aqueous solution contains a sugar-based nonionic surfactant.

23. The preparation method according to claim 22, wherein the sugar-based nonionic surfactant is LMNG.

24. The preparation method according to any one of claims 21 to 23, wherein the eluate is neutralized and subjected to the digestion with the protease in step 2.
